(19) Europäisches Patentamt
European Patent Office
Office européen des brevets

(11) EP 2 512 428 B1

(12) **EUROPEAN PATENT SPECIFICATION**

(45) Date of publication and mention of the grant of the patent:
**05.04.2017 Bulletin 2017/14**

(21) Application number: **10793236.0**

(22) Date of filing: **15.12.2010**

(51) Int Cl.:
***A61K 8/49*** *(2006.01)* ***A61Q 1/00*** *(2006.01)*
***A61Q 1/04*** *(2006.01)* ***A61Q 5/00*** *(2006.01)*
***A61Q 19/00*** *(2006.01)*

(86) International application number:
**PCT/EP2010/069841**

(87) International publication number:
**WO 2011/073295 (23.06.2011 Gazette 2011/25)**

# (54) COSMETIC COMPOSITION COMPRISING A SUPRAMOLECULAR COMPOUND CAPABLE OF ESTABLISHING HYDROGEN BONDS, AND A PARTICULAR OIL

KOSMETISCHE ZUSAMMENSETZUNG MIT EINER SUPRAMOLEKULAREN VERBINDUNG MIT FÄHIGKEIT ZUR HERSTELLUNG VON WASSERSTOFFBINDUNGEN SOWIE EINEM SPEZIELLEN ÖL

COMPOSITION COSMÉTIQUE COMPRENANT UN COMPOSÉ SUPRAMOLÉCULAIRE CAPABLE D'ÉTABLIR DES LIAISONS HYDROGÈNE AINSI QU'UNE HUILE PARTICULIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2009 FR 0959207**
**23.12.2009 US 289409 P**

(43) Date of publication of application:
**24.10.2012 Bulletin 2012/43**

(73) Proprietor: **L'Oréal**
**75008 Paris (FR)**

(72) Inventors:
- **BARBA, Claudia**
  **F-75010 Paris (FR)**
- **CAVAZZUTI, Roberto**
  **F-75015 Paris (FR)**
- **GEFFROY, Nathalie**
  **F-91370 Verrières-le-Buisson (FR)**

(74) Representative: **Goudet, Sylvain et al**
**L'Oréal**
**Service DIPI**
**9 Rue Pierre Dreyfus**
**92110 Clichy (FR)**

(56) References cited:
**EP-A1- 2 140 858** **WO-A1-02/098377**
**WO-A1-2006/118460**

- **FOLMER B J B ET AL: "SUPRAMOLECULAR POLYMER MATERIALS: CHAIN EXTENSION OF TELECHELIC POLYMERS USING A REACTIVE HYDROGEN-BONDING SYNTHON", ADVANCED MATERIALS, WILEY VCH VERLAG, DE LNKD-DOI:10.1002/1521-4095(200006)12:12<874::AID-ADMA874>3.3.CO;2-3, vol. 12, no. 12, 16 June 2000 (2000-06-16) , pages 874-878, XP000959548, ISSN: 0935-9648 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a cosmetic composition, especially for caring for and/or making up keratin materials, in particular the skin or the lips, comprising novel compounds A (referred to in the context of the present patent application as supramolecular compounds) capable of establishing hydrogen bonds with partner junction groups, combined with an oil, chosen from apolar hydrocarbon-based oils and silicone oils, and with a dyestuff. Many cosmetic compositions exist for which gloss properties of the deposited film, after application to keratin materials, are desired. Mention may be made, for example, of lipsticks or nail varnishes. In order to obtain such a result, it is possible to combine particular starting materials, especially lanolins, with "glossy" oils such as polybutenes, or esters of fatty acid or of fatty alcohol with a high carbon number; or alternatively certain plant oils; or alternatively esters resulting from the partial or total esterification of a hydroxylated aliphatic compound with an aromatic acid, as described in patent application EP 1 097 699.

However, these oils combined with compounds obtained by modification of oils containing an OH or NH2 function may give rise to a problem of tackiness. This tacky nature causes these formulations to leave marks on supports such as glasses and cups.

Formulators are thus in search of starting materials and/or systems for obtaining compositions whose deposit is characterized by gloss and a tack-free (or at least sparingly tack-free) effect.

The aim of the present invention is to propose cosmetic compositions for obtaining such a uniform deposit on keratin materials, the said deposit combining gloss, gloss remanence over time (in particular 1 hour after application), while being non-tacky (or sparingly tacky) and particularly comfortable to wear.

One subject of the present invention is thus a cosmetic composition for making up and/or caring for keratin materials (especially the skin or the lips), comprising, in a cosmetically acceptable medium:

(i) a compound A (referred to, in the context of this patent application, as a supramolecular compound) that can be obtained by reaction between:

- at least one oil bearing at least one nucleophilic reactive function chosen from OH and $NH_2$, and
- at least one junction group capable of establishing hydrogen bonds with one or more partner junction groups, each pairing of a junction group involving at least three hydrogen bonds, the said junction group bearing at least one isocyanate or imidazole reactive function capable of reacting with the reactive function borne by the oil, the said junction group also comprising at least one unit of formula (I) or (II):

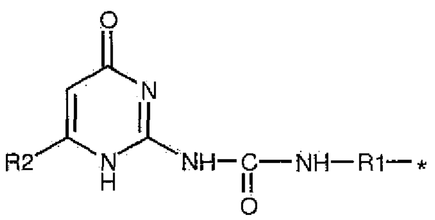

(I)

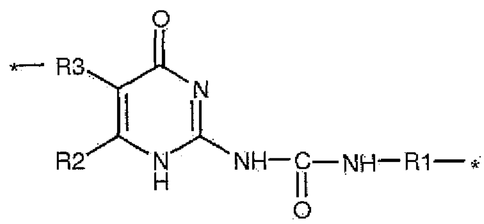

(II)

in which:

- R1 and R3, which may be identical or different, represent a divalent carbon-based radical chosen from (i) a linear or branched $C_1$-$C_{32}$ alkyl group, (ii) a $C_4$-$C_{16}$ cycloalkyl group and (iii) a $C_4$-$C_{16}$ aryl group; optionally comprising 1 to 8 heteroatoms chosen from O, N, S, F, Si and P; and/or optionally substituted with an ester or amide function or with a $C_1$-$C_{12}$ alkyl radical; or a mixture of these groups;
- R2 represents a hydrogen atom or a linear, branched or cyclic, saturated or unsaturated, optionally aromatic, $C_1$-$C_{32}$ carbon-based and especially hydrocarbon-based radical, which may comprise one or more heteroatoms chosen from O, N, S, F, Si and P;

(ii) at least one oil, chosen from non-volatile apolar hydrocarbon-based oils having a vapour pressure of less than 0.13Pa (at ambient temperature (25°C)), and silicone oils;

(iii) at least one dyestuff.

**Supramolecular compounds:**

The compounds A (also known as supramolecular compounds) functionalized according to the present invention are in the form of a solid; this makes it possible especially to form a non-tacky material, which does not transfer onto the fingers once applied to keratin materials; this is not the case for the functionalized compounds of the prior art, especially as described in US 5 707 612, which are in the form of a more or less viscous liquid, and which form a tacky material that transfers onto the fingers after application to keratin materials.

Moreover, it has been found that crosslinking by means of four hydrogen bonds, via ureidopyrimidone groups, can increase the strength of this crosslinking, and thus improve the remanence of the desired cosmetic effect, most particularly the remanence of the deposit or of the gloss.

Furthermore, the compounds, or functionalized oils, according to the invention are easy to convey in the usual cosmetic media, especially the usual cosmetic oily media.

They are advantageously compatible with the oils usually present in cosmetic compositions, and also have good properties of dispersing pigments or fillers.

They are easy to convey in cosmetic oily or solvent media, especially oils, fatty alcohols and/or fatty esters, which facilitates their use in the cosmetic field, especially in lipsticks. They show acceptable solubility in varied cosmetic oily media, such as plant oils, alkanes, esters, whether they are short esters such as butyl or ethyl acetate, or fatty esters, and fatty alcohols, and most particularly in media comprising isododecane, parleam, isononyl isononanoate, octyldodecanol and/or a C12-C15 alkyl benzoate.

The cosmetic compositions according to the invention moreover show good applicability and good coverage; good adherence to the support, whether it is to the nails, the eyelashes, the skin or the lips; adequate flexibility and strength of the film, and also an excellent gloss durability. The comfort and glidance properties are also very satisfactory.

In general, in the context of the present patent application, the compounds A may be referred to without preference as "supramolecular compounds" for convenience and for greater clarity.

The compounds A (or supramolecular compounds) of the compositions according to the invention may be obtained by reaction between:

- at least one oil bearing at least one nucleophilic reactive function chosen from OH and $NH_2$, and
- at least one junction group capable of establishing hydrogen bonds with one or more partner junction groups, each pairing of a junction group involving at least three hydrogen bonds, the said junction group bearing at least one isocyanate or imidazole reactive function capable of reacting with the reactive function borne by the oil, the said junction group also comprising at least one unit of formula (I) or (II):

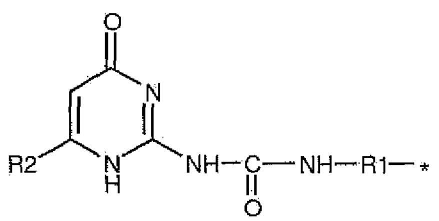

(I)

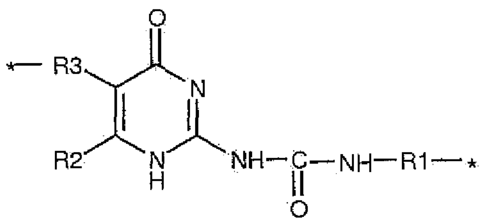

(II)

in which:

- R1 and R3, which may be identical or different, represent a divalent carbon-based radical chosen from (i) a linear or branched $C_1$-$C_{32}$ alkyl group, (ii) a $C_4$-$C_{16}$ cycloalkyl group and (iii) a $C_4$-$C_{16}$ aryl group; optionally comprising 1 to 8 heteroatoms chosen from O, N, S, F, Si and P; and/or optionally substituted with an ester or amide function or with a $C_1$-$C_{12}$ alkyl radical; or a mixture of these groups;
- R2 represents a hydrogen atom or a linear, branched or cyclic, saturated or unsaturated, optionally aromatic, $C_1$-$C_{32}$ carbon-based and especially hydrocarbon-based radical, which may comprise one or more heteroatoms chosen from O, N, S, F, Si and P.

In conclusion, the supramolecular compounds of the compositions according to the invention thus comprise at least one part (HB) originating from the oil and at least one part (G) originating from the junction group, the said part (G) comprising at least one unit of formula (I) or (II).

In particular, the said parts (HB) and (G) are connected via a covalent bond and may especially be connected

via a covalent bond formed during the reaction between the OH and/or $NH_2$ reactive functions borne by the oil and the isocyanate reactive functions borne by the junction group; or alternatively between the $NH_2$ reactive functions borne by the oil and the isocyanate or imidazole functions borne by the junction group.

The preferential production of the compounds according to the invention may thus especially be represented schematically by the chemical reaction between the following species:

$$(HB)\text{-}(OH)_m(NH_2)_n + (G)\text{-}(NCO)_p$$

or

$$(HB)\text{-}(OH)_m(NH_2)_n + (G)\text{-}(imidazole)_p$$

with m, n and p being non-zero integers.

The oil that may be used to prepare the compound according to the invention, which may preferably be represented schematically as $(HB)\text{-}(OH)_m(NH_2)_n$, is a fatty substance or a mixture of fatty substances, which is not crystalline at 25°C, and is liquid at room temperature and at atmospheric pressure (25°C, 1 atm.); preferably apolar or even, preferably, water-insoluble.

Preferably, the oil that may be used to prepare the supramolecular compound according to the invention is non-polymeric.

The term liquid" means that the viscosity of the compound is less than or equal to 2500 centipoises, at 110°C and 1 atm., measured with a Brookfield DV-I or Brookfield Cap 1000+ rheometer, a person skilled in the art selecting the machine that is suited to the viscosity measurement.

The term "apolar" means a compound whose HLB value (hydrophilic/lipophilic balance) is low; especially less than or equal to 8, preferably less than or equal to 4 and better still less than or equal to 2; preferentially, the HLB value should be low enough to make it possible to obtain a supramolecular material that is not hygroscopic, or not too hygroscopic.

The term "insoluble" means that the oil fraction that can dissolve in water, at 25°C and 1 atm., is less than 5% by weight (i.e. 5 g of oil in 100 ml of water); preferably less than 3%.

The term "fatty substance" means especially, but not exclusively, a hydrocarbon-based compound comprising one or more saturated or unsaturated, linear, cyclic or branched alkyl chains, containing at least 6 carbon atoms and possibly comprising polar groups such as an acid, hydroxyl or polyol, amine, amide, phosphoric acid, phosphate, ester, ether, urea, carbamate, thiol, thioether or thioester group, this chain possibly containing up to 100 carbon atoms.

Preferably, the oil that may be used to prepare the compound according to the invention is a glossy oil, i.e. an oil with a refractive index of greater than or equal to 1.46 at 25°C and in particular between 1.46 and 1.55 (the refractive index being defined relative to the sodium D line, at 25°C).

Preferably, the oil that may be used to prepare the compound according to the invention is a nonvolatile oil. The term "non-volatile oil" means an oil that is capable of remaining on keratin materials at room temperature and atmospheric pressure for at least several hours, and that especially has a vapour pressure of less than $10^{-3}$ mmHg (0.13 Pa).

Preferably, the oil has a molar mass (Mw) of between 150 and 6000, especially between 170 and 4000, or even between 180 and 2000, more preferentially between 200 and 1500 and better still between 220 and 800 g/mol.

The oil that may be used in the context of the present invention bears at least one reactive function capable of reacting with the reactive function borne on the junction group, and is especially capable of reacting chemically with the isocyanate or imidazole groups borne by the junction group; preferably, this function is an OH or $NH_2$ function. Preferably, the oil comprises only OH functions, in particular 1 to 3 OH functions, preferentially primary or secondary OH functions, and better still only primary functions.

The oil according to the present invention is preferably a carbon-based and especially a hydrocarbon-based oil, which, besides the reactive function capable of reacting with the junction group, may comprise oxygen, nitrogen, sulfur and/or phosphorus atoms. The oil is very preferentially chosen from cosmetically acceptable oils.

The oil that may be used in the context of the present invention may be chosen from:

(i) saturated or unsaturated, linear, branched or cyclic fatty alcohols containing 6 to 50 carbon atoms, comprising one or more OH; optionally comprising one or more $NH_2$.
Mention may be made in particular of:

- saturated or unsaturated, linear or branched C6-C50, especially C6-C32 and in particular C8-C28 monoalcohols, and especially isostearyl alcohol, cetyl alcohol, oleyl alcohol, isopalmitoyl alcohol, 2-butyloctanol, 2-hexyldecanol, 2-octyldecanol, 2-octyldodecanol, 2-octyltetradecanol, 2-decyltetradecanol and 2-dodecylhexadecanol, and

especially the alcohols sold under the name Jarcol by the company Jarchem Industries, such as Jarcol I-12, Jarcol 1-16, Jarcol I-20 and Jarcol I-24;

- saturated or unsaturated, linear or branched C6-C50, especially C6-C40 and in particular C8-C38 diols, and especially branched C32-C36 diols, and in particular the commercial product Pripol 2033 from Uniqema ;
- saturated or unsaturated, linear or branched C6-C50, especially C6-C32 and in particular C8-C28 triols, and especially phytanetriol;

(ii) esters and ethers bearing at least one free OH, and especially partial polyol esters and ethers, and hydroxylated carboxylic acid esters.

The term "partial polyol ester" means esters prepared by esterification of a polyol with a substituted or unsubstituted carboxylic acid, the reaction not being total, i.e. not performed on all of the free OHs of the polyol; as a result, the ester thus still comprises at least one free OH.

Preferably, the carboxylic acid is a monoacid. A mixture of carboxylic acids, especially monocarboxylic acids, may also be used.

The term "partial polyol ether" means ethers prepared by etherific.ation of a polyol, with itself or with at least one other monohydroxylated or polyhydroxylated alcohol, preferably a monoalcohol, the etherification reaction not being total, i.e. not performed on all of the free OHs of the polyol; as a result, the ether still comprises at least one free OH.

The term hydroxylated carboxylic acid ester" means (mono and poly)esters prepared by reaction between a carboxylic acid bearing at least one free OH function, and one or more (mono or poly)alcohols, preferably a monoalcohol, the reaction possibly being total or partial (performed on all or some of the free OHs of the alcohol).

Among the polyols that may be used for preparing the above esters or ethers, mention may be made of propylene glycol, glycerol, neopentyl glycol, trimethylolpropane, trimethylolethane, polyglycerols and especially polyglycerol-2, polyglycerol-3 and polyglycerol-10; erythritol, dipentaerythritol, pentaerythritol, bis(trimethylolpropane), phytanetriol, sucrose, glucose, methylglucose, sorbitol, fructose, xylose, mannitol or glucosamine; and also diol dimers obtained especially from fatty acid dimers, especially branched aliphatic and/or alicyclic C32-C38 and especially C36 diols, such as those defined in the article Hofer et al., European Coating Journal (March 2000), pages 26-37; and mixtures thereof.

Among the monoalcohols that may be used for preparing the above esters or ethers, mention may be made of linear or branched, preferably branched, C3-C50 alcohols, and especially 2-ethylhexanol, octanol and isostearyl alcohol, and mixtures thereof.

Among the carboxylic acids that may be used for preparing the above esters or ethers, mention may be made of linear or branched, saturated or unsaturated monoacids containing 6 to 50 carbon atoms and diacids containing 3 to 12 carbon atoms, among which mention may be made of octylneodecanoic acid, hexyldecanoic acid, ethylhexanoic acid, isostearic acid, nonanoic acid, isononanoic acid, arachidic acid, stearic acid, palmitic acid, oleic acid, oxalic acid, adipic acid, succinic acid, fumaric acid, maleic acid, capric acid, hexanedioic acid and decanoic acid, and mixtures thereof.

Among the hydroxylated carboxylic acids that may be used for preparing the above esters or ethers, mention may be made of monohydroxylated or polyhydroxylated acids, preferably monohydroxylated acids, containing for example 4 to 28 carbon atoms, and especially 12-hydroxystearic acid, ricinoleic acid, malic acid, lactic acid and citric acid; and mixtures thereof.

Thus, the oil that may be used in the present invention may be chosen, alone or as a mixture, from:

- pentaerythritol partial esters, and especially pentaerythritol adipate, pentaerythritol caprate, pentaerythrityl succinate, pentaerythritol tetraisononanoate, pentaerythrityl triisononanoate, pentaerythrityl tetraisostearate, pentaerythrityl triisostearate, pentaerythrityl 2-(tetradecyl)-tetradecanoate, pentaerythritol (tetraethyl)hexanoate and pentaerythrityl (tetraoctyl)dodecanoate;
- dipentaerythritol diesters, triesters, tetraesters or pentaesters, and especially dipentaerythrityl pentaisononanoate, dipentaerythrityl pentaisostearate, dipentaerythrityl tetraisostearate and dipentaerythrityl tris(polyhydroxystearate);
- trimethylolpropane monoesters and diesters, for instance trimethylolpropane monoisostearate, trimethylolpropane diisostearate, trimethylolpropane mono-2-ethylhexanoate and trimethylolpropane bis(2-ethylhexanoate);
- bis(trimethylolpropane) monoesters, diesters and triesters, for instance bis(trimethylolpropane) diisostearate, bis(trimethylolpropane) triisostearate and bis(trimethylolpropane) triethylhexanoate;
- partial monoesters or polyesters of glycerol or of pplyglycerols, and especially:
    - glyceryl diisostearate and glyceryl diisononanoate;
    - polyglycerol-2 monoesters, diesters and triesters; for example with isostearic acid, 2-ethylhexanoic acid and/or isononanoic acid; and especially polyglyceryl-2 isostearate; polyglyceryl-2 diisostearate; polyglyceryl-2 triisostearate; polyglyceryl-2 nonaisostearate; polyglyceryl-2 nonanoate;

  - polyglycerol-3 monoesters, diesters, triesters or tetraesters; for example with either isostearic acid, 2-ethylhexanoic acid and/or isononanoic acid; and especially polyglyceryl-3 isostearate; polyglyceryl-3 diisostearate; polyglyceryl-3 triisostearate; polyglyceryl-3 nonaisostearate; polyglyceryl-3 nonanoate;
  - polyglycerol-10 partial esters and in particular polyglyceryl-10 nonaisostearate; polyglyceryl-10 nonanoate; polyglyceryl-10 isostearate; polyglyceryl-10 diisostearate; polyglyceryl-10 triisostearate;

- propylene glycol monoesters, for instance propylene glycol monoisostearate, propylene glycol neopentanoate or propylene glycol monooctanoate;
- diol dimer monoesters, for instance isostearyl dimer dilinoleate and octyldodecyl dimer dilinoleate;
- glycerol ethers, such as polyglyceryl-2 oleyl ether, polyglyceryl-3 cetyl ether, polyglyceryl-3 decyl tetradecyl ether and polyglyceryl-2 stearyl ether;
- esters between a hydroxylated monocarboxylic, dicarboxylic or tricarboxylic acid and monoalcohols, and in particular:

  - esters, especially monoesters, of 12-hydroxystearic acid; such as octyl hydroxystearate and 2-octyldodecyl hydroxystearate; mention may also be made of the corresponding oligomeric polyhydroxystearates, especially having a degree of polymerization of from 1 to 10, bearing at least one residual OH;
  - lactic acid esters, and especially C4-40 alkyl lactates, such as 2-ethylhexyl lactate, diisostearyl lactate, isostearyl lactate, isononyl lactate or 2-octyldodecyl lactate;
  - malic acid esters, and especially C4-40 alkyl malates, such as bis (2-ethylhexyl) malate, diisostearyl malate or bis(2-octyldodecyl) malate;
  - citric acid esters, and especially C4-40 alkyl citrates, such as triisostearyl citrate, triisocetyl citrate and triisoarachidyl citrate;

(iii) hydroxylated natural oils, modified natural oils and plant oils, and especially:

- triglyceryl esters bearing one or more OHs;
- hydrogenated or non-hydrogenated castor oil, and also derivatives thereof derived especially from the transesterification of castor oil; for instance the products Polycin M-365 or Polycin 2525 sold by Vertellus;
- modified epoxidized oils, the modification consisting in opening the epoxy function to obtain a diol, and especially hydroxylated modified soybean oil; hydroxylated soybean oils (directly hydroxylated or epoxidized beforehand); and especially the oils Agrol 2.0, Agrol 3.0 and Agrol 7.0 sold by Bio-Based Technologies, LLC; the oil Soyol R2-052 from the company Urethane Soy System; the Renuva oils sold by Dow Chemical; the oils BioH Polyol 210 and 500 sold by Cargill.

According to a first particularly preferred embodiment, the oil that may be used to prepare the supramolecular compound in the context of the present invention is chosen from linear, branched or cyclic, saturated or unsaturated fatty alcohols, comprising 6 to 50 carbon atoms, comprising one or more OH; optionally comprising one or more $NH_2$, such as:

- saturated or unsaturated, linear or branched C6-C50, especially C6-C32 and in particular C8-C28 monoalcohols, and especially isostearyl alcohol, cetyl alcohol, oleyl alcohol, isopalmitoyl alcohol, 2-butyloctanol, 2-hexyldecanol, 2-octyldodecanol, 2-octyldodecanol, 2-octyltetradecanol, 2-decyltetradecanol and 2-clodecylhexadecanol, and especially the alcohols sold under the name Jarcol by the company Jarchem Industries, such as Jarcol I-12, Jarcol I-16, Jarcol I-20 and Jarcol I-24;
- saturated or unsaturated, linear or branched, C6-C50, especially C5-C40 and in particular C8-C38 diols, and especially branched C32-36 diols, and in particular the commercial product Pripol 2033 from Uniqema;
- saturated or unsaturated, linear or branched C6-C50, especially C5-C32 and in particular C8-C28 triols, and especially phytanetriol.

According to this first preferred embodiment, the oil that may be used to prepare the supramolecular compound in the context of the present invention is preferably chosen from linear or branched, saturated or unsaturated C6-C50, especially C6-C32 and in particular C8-C28 monoalcohols, and especially isostearyl alcohol, cetyl alcohol, oleyl alcohol, isopalmitoyl alcohol, 2-butyloctanol, 2-hexyldecanol, 2-octyldodecanol, 2-octyldodecanol, 2-octyltetradecanol, 2-decyltetradecanol and 2-dodecylhexadecanol, and especially the alcohols sold under the name Jarcol by the company Jarchem Industries, such as Jarcol I-12, Jarcol 1-16, Jarcol I-20 and Jarcol I-24.

According to a second particularly preferred embodiment, the oil that may be used to prepare the supramolecular

compound in the context of the present invention is chosen from esters between a hydroxylated mono-, di- or tricarboxylic acid and monoalcohols, and in particular:

- esters, especially monoesters, of 12-hydroxystearic acid; such as octyl hydroxystearate and 2-octyldodecyl hydroxystearate; mention may also be made of the corresponding oligomeric polyhydroxystearates, especially with a degree of polymerization of from 1 to 10, containing at least one residual OH;
- lactic acid esters, especially C4-40 alkyl lactates, such as 2-ethylhexyl lactate, diisostearyl lactate, isostearyl lactate, isononyl lactate and 2-octyldodecyl lactate;
- malic acid esters, and especially C4-40 alkyl malates, such as 2-diethylhexyl malate, diisostearyl malate and 2-dioctyldodecyl malate;
- citric acid esters, and especially C4-40 alkyl citrates, such as triisostearyl citrate, triisocetyl citrate and triisoarachidyl citrate.

According to this second preferred embodiment, the oil that may be used in the context of the present invention is preferably chosen from esters between a hydroxylated dicarboxylic acid and monoalcohols, and in particular of malic acid, and especially C4-40 alkyl malates, such as 2-diethylhexyl malate, diisostearyl malate and 2-dioctyldodecyl malate.

In particular, when glossy oils are used, the following glossy oils, the refractive index of which at 25°C is indicated in parentheses, may be used: polyglyceryl-3 diisostearate (1.472), phytanetriol (1.467), castor oil (1.475), 2-octyldodecanol (1.46), oleyl alcohol (1.461), octyl hydroxystearate (1.46), polyglyceryl-2 isostearate (1.468), polyglyceryl-2 diisostearate (1.464), diisostearyl malate (1.462), 2-butyloctanol, 2-hexyldecanol (1.45), 2-decyltetra-decanol (1.457), and also mixtures thereof.

Preferably, the oils that may be used in the present invention are chosen from 2-octyldodecanol, diisostearyl malate, 2-butyloctanol, 2-hexyldecanol, 2-decyltetradecanol; hydrogenated or non-hydrogenated castor oil, and also derivatives thereof; hydroxylated modified soybean oil, and mixtures thereof.

<u>Junction group</u>

The junction group that may be used to form the supramolecular compound of the compositions according to the invention bears at least one reactive group, especially isocyanate or imidazole, capable of reacting with the reactive functions, especially OH and/or $NH_2$ (exclusively $NH_2$ for imidazole), of the oil, so as to form a covalent bond, especially of urethane type, between the said oil and the said junction group.

Preferably, the junction group that may be used to form the supramolecular compound of the compositions according to the invention bears at least one reactive group, especially isocyanate.

The said junction group is capable of establishing H bonds with one or more partner junction groups, of identical or different chemical nature, each junction group pairing involving at least 3 H (hydrogen) bonds, preferably at least 4 H bonds and preferentially 4 H bonds.

For the purposes of the invention, the term "junction group" means any functional group comprising groups that are H bond donors or acceptors, and that are capable of establishing at least 3 H bonds, preferably at least 4 H bonds, preferentially 4 H bonds, with an identical or different partner junction group.

For the purposes of the invention, the term "partner junction group" means any junction group that can establish H bonds with one or more junction groups, of the same or of another polymer according to the invention. The junction groups may be of identical or different chemical nature. If they are identical, they may then establish H bonds between themselves and are then referred to as self-complementary junction groups. If they are different, they are chosen such that they are complementary with respect to H interactions.

The said junction group, bearing isocyanate groups, may thus be represented schematically as (G) $(NCO)_p$, p being a non-zero integer, preferably equal to 1 or 2.

The junction group moreover comprises at least one monovalent unit of formula (I) and/or at least one divalent unit of formula (II), as defined below:

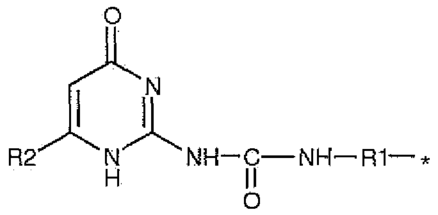

(I)

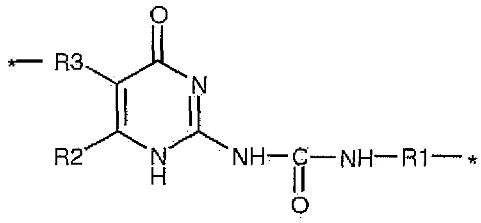

(II)

in which:

- R1 and R3, which may be identical or different, represent a divalent carbon-based radical chosen from (i) a linear or branched $C_1$-$C_{32}$ alkyl group, (ii) a $C_4$-$C_{16}$ cycloalkyl group and (iii) a $C_4$-$C_{16}$ aryl group; optionally comprising 1 to 8 heteroatoms chosen from O, N, S, F, Si and P; and/or optionally substituted with an ester or amide function or with a $C_1$-$C_{12}$ alkyl radical; or a mixture of these groups;
- R2 represents a hydrogen atom or a linear, branched or cyclic, saturated or unsaturated, optionally aromatic, C1-C32 carbon-based and especially hydrocarbon-based (alkyl) radical, which may comprise one or more heteroatoms chosen from O, N, S, F, Si and P.

Preferably, the junction group moreover comprises at least one monovalent unit of formula (I).
The radical R1 may especially be:

- a linear or branched, divalent C2-C12 alkylene group, especially a 1,2-ethylene, 1,6-hexylene, 1,4-butylene, 1,6-(2,4,4-trimethylhexylene), 1,4-(4-methylpentylene), 2,5-(5-methylhexylene), 1,6-(6-methylheptylene), 1,5-(2,2,5-trimethylhexylene) or 1,7-(3,7-dimethyloctylene) group;
- a divalent C4-C12 cycloalkylene or arylene group, chosen especially from the following radicals: -isophorone-, tolylene, 2-methyl-1,3-phenylene, 4-methyl-1,3-phenylene; 4,4'-methylenebiscyclohexylene; 4,4-bisphenylenemethylene; or of structure:

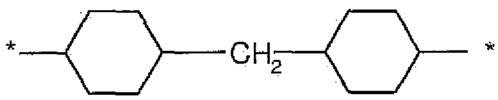

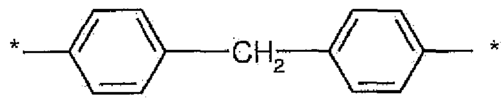

The term "-isophorone-" means the divalent radical having the structure:

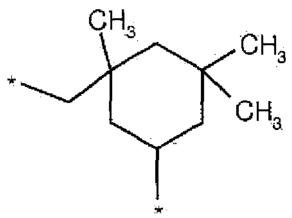

Preferentially, R1 represents -isophorone-, $-(CH_2)_6-$ or 4,4'-methylenebiscyclohexylene.
The radical R2 may especially be H or:

- a $C_1$-$C_{12}$, in particular $C_1$-$C_{16}$ or even $C_1$-$C_{10}$ alkyl group;
- a $C_4$-$C_{12}$ cycloalkyl group;
- a $C_4$-$C_{12}$ aryl group;
- a ($C_4$-$C_{12}$) aryl ($C_1$-$C_{18}$) alkyl group;
- a $C_1$-$C_4$ alkoxy group;
- an arylalkoxy group, in particular an aryl ($C_1$-$C_4$) alkoxy group;
- a $C_4$-$C_{12}$ heterocycle;

or a combination of these radicals, which may be optionally substituted with an amino, ester and/or hydroxyl function.

Preferably, R2 represents H, $CH_3$, ethyl, $C_{13}H_{27}$, $C_7H_{15}$, phenyl, isopropyl, isobutyl, n-butyl, tert-butyl, n-propyl or -CH($C_2H_5$) ($C_4H_9$).

Preferably, R3 represents a divalent radical -R'3-O-C(O)-NH-R'4- in which R'3 and R'4, which may be identical or different, represent a divalent carbon-based radical chosen from a linear or branched $C_1$-$C_{32}$ alkyl group, a $C_4$-$C_{16}$ cycloalkyl group and a $C_4$-$C_{16}$ aryl group; or a mixture thereof.

In particular, R'3 and R'4 may represent methylene, 1,2-methylene, 1,6-hexylene, 1,4-butylene, 1,6-(2,4,4-trimethylhexylene), 1,4-(4-methylpentylene), 1,5-(5-methylhexylene) ; 1,5-(6-methylheptylene); 1,5-(2,2,5-trimethylhexylene), 1,7-(3,7-dimethyloctylene) ; 4,4'-methylenebiscyclohexylene; 2-methyl-1,3-phenylene; 4-methyl-1,3-phenylene; 4,4'-bisphenylenemethylene; 1,2-tolylene, 1,4-tolylene, 2,4-tolylene, 2,6-tolylene; 1,5-naphthylene; tetramethylxylylene; isophorone.

Most particularly, R'3 may represent a C1-C4 alkylene, especially 1,2-ethylene.

Preferably, R'4 may represent the divalent radical derived from isophorone.

Most particularly, R3 may have the structure:

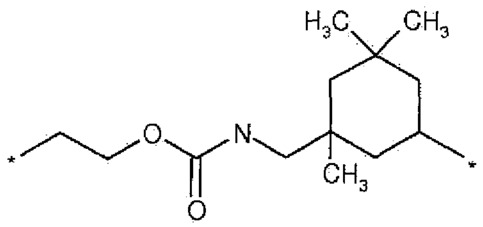

In a particularly preferred manner, the following may apply in formula (I):

- $R_1$ = -isophorone-, R2 = methyl, which gives the unit of formula:

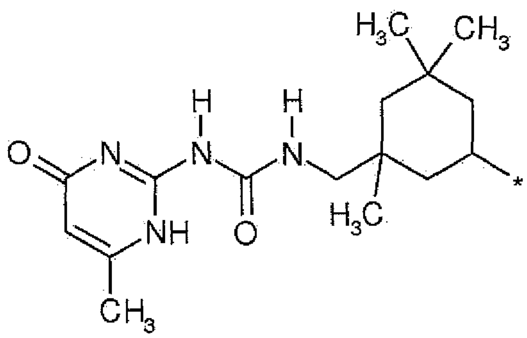

- $R_1$ = $(CH_2)_6$-, R2 = methyl, which gives the unit of formula:

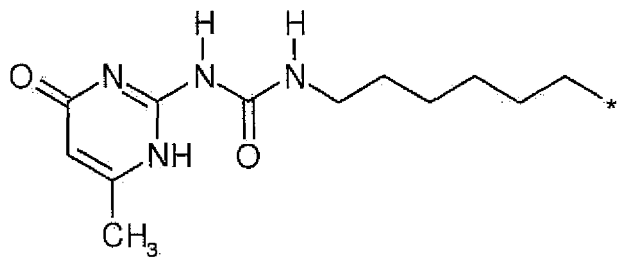

- $R_1$ = -$(CH_2)_6$-, R2 = isopropyl, which gives the unit of formula:

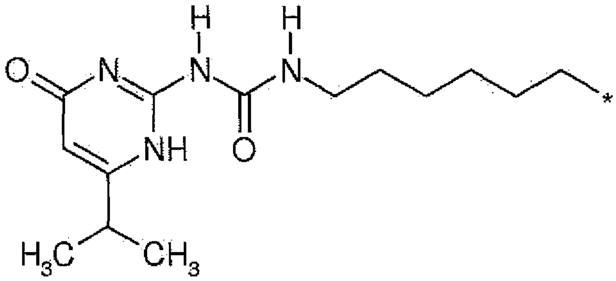

- $R_1$ = 4,4'-methylenebiscyclohexylene and R2 = methyl, which gives the unit of formula:

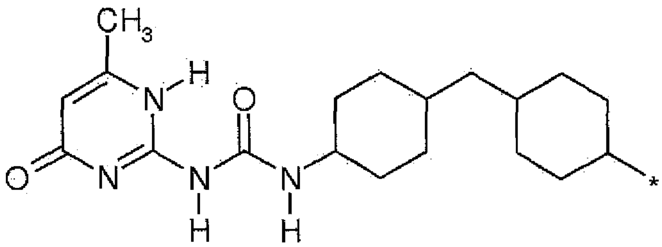

In a particularly preferred manner, in formula (II), R1 may represent the -isophorone- radical, R2 = methyl and R3 = - $(CH_2)_2$OCO-NH-isophorone-, which gives the divalent unit of formula:

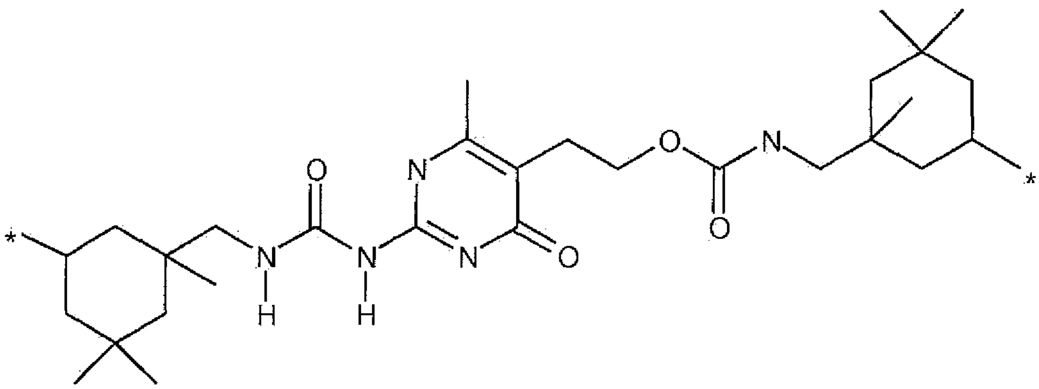

The junction groups bearing only one isocyanate function may have the formula:

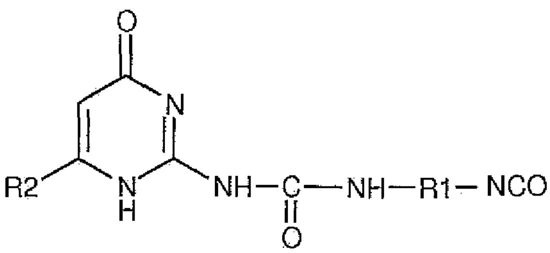

in which R1 and R2 are as defined above; and in particular:

- R1 represents -isophorone-, $-(CH_2)_6-$ , $-CH_2CH(CH_3)-CH_2-C(CH_3)_2-CH_2-CH_2$, 4,4'-methylenebiscyclohexylene or 2-methyl-1,3-phenylene; and/or
- R2 represents H, $CH_3$, ethyl, $C_{13}H_{27}$, $C_7H_{15}$, phenyl, isopropyl, isobutyl, n-butyl, tert-butyl, n-propyl or $-CH(C_2H_5)(C_4H_9)$.

Preferably, the junction groups may be chosen from the following groups:

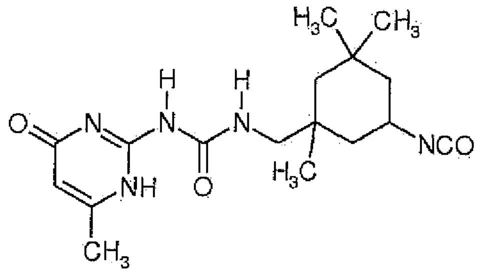

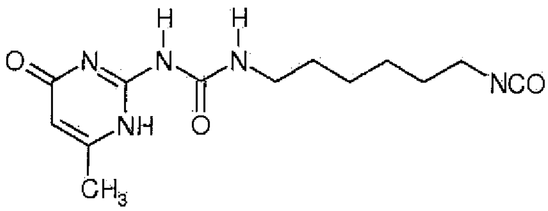

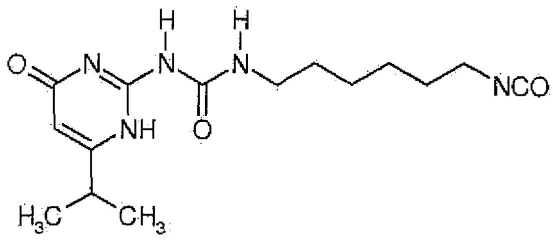

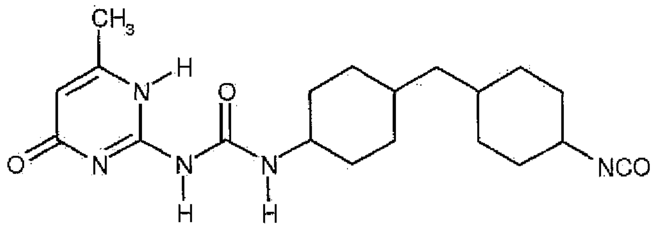

The junction groups bearing two isocyanate functions may have the formula:

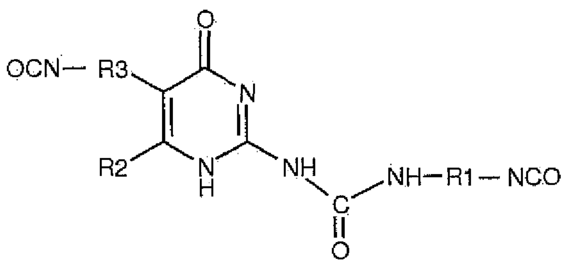

in which R1, R2 and R3 are as defined above, and in particular:

- R1 represents -isophorone-, - $(CH_2)_2$-, - $(CH_2)_5$-, -$CH_2CH$ $(CH_3)$-$CH_2$-C $(CH_3)_2$-$CH_2$-$CH_2$, 4,4'-methylenebiscyclohexylene or 2-methyl-1,3-phenylene; and/or
- R2 represents H, $CH_3$, ethyl, $C_{13}H_{27}$, $C_7H_{15}$, phenyl, isopropyl, isobutyl, n-butyl, tert-butyl, n-propyl or -$CH(C_2H_5)$ $(C_4H_9)$; and/or
- R3 represents a divalent radical -R'3-O-C(O)-NH-R'4- in which R'3 and R'4, which may be identical or different, represent a divalent carbon-based radical chosen from a linear or branched $C_1$-$C_{30}$ alkyl group, a $C_4$-$C_{12}$ cycloalkyl group and a $C_4$-$C_{12}$ aryl group; or a mixture thereof; and especially R'3 represents a C1-C4 alkylene, especially 1,2-methylene, and R'4 represents the divalent radical derived from isophorone.

A junction group that is most particularly preferred is the one having the formula:

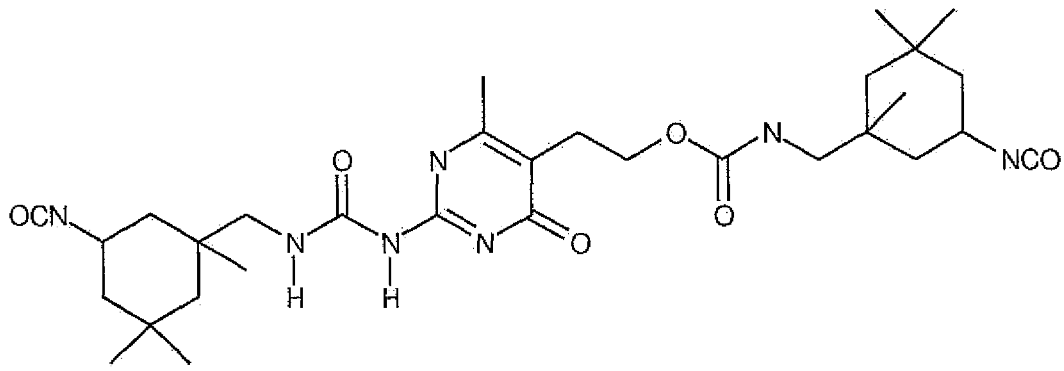

Among the junction groups bearing an imidazole group, mention may be made of the following compound:

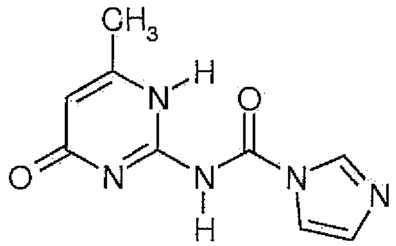

According to one particular embodiment of the invention, the junction groups may be attached to the oil by functionalization of the junction group with an isocyanate or imidazole.

According to another embodiment, it is possible to perform the reverse reaction by prefunctionalizing the oil with a diisocyanate.

As mentioned above (first mode), the compound according to the invention may thus result from the chemical reaction between an oil $(HB)-(OH)_m(NH_2)_n$ and a junction group $(G)-(NCO)_p$ or $(G)-(imidazole)_p$.

Preferably, the oil comprises only hydroxyl functions and the junction group comprises 1 or 2 isocyanate functions, which leads to the following reactions:

$$(HB)-(OH)_m + OCN-(G)-(NCO) \rightarrow (HB)-OC(O)NH-(G)-NHC(O)-(HB)$$

$$(HB)-(OH)_m + (G)-NCO \rightarrow (HB)-OC(O)NH-(G)$$

with m = integer greater than or equal to 1.

Preferably, the degree of grafting of the free OHs of the oil is between 1% and 100%, especially between 20% and 99% and better still between 50% and 95%; preferably, this degree is 100% (all the free OHs are functionalized with a junction group), especially when the oil initially comprises only one OH function.

The supramolecular compound according to the invention may be prepared via the processes usually used by those skilled in the art for forming a urethane bond, between the free OH functions of the oil and the isocyanate functions borne by the junction group. By way of illustration, a general preparation process consists in:

- ensuring that the oil to be functionalized does not comprise any residual water,
- heating the oil comprising at least one reactive function, especially OH, to a temperature that may be between 60°C and 140°C;
- adding the junction group bearing the reactive functions, especially isocyanate;
- optionally stirring the mixture, under a controlled atmosphere, at a temperature of about 100-130°C; for 1 to 24 hours;
- monitoring by infrared spectroscopy the disappearance of the characteristic band for isocyanates (between 2500 and 2800 $cm^{-1}$) so as to stop the reaction at the total disappearance of the peak, and then to allow the final product to cool to room temperature.

The reaction may be performed in the presence of a solvent, especially methyltetrahydrofuran, tetrahydrofuran, toluene or butyl acetate; the reaction may also be performed without solvent, in which case the oil may serve as solvent.

It is also possible to add a conventional catalyst for the formation of a urethane bond. An example that may be mentioned is dibutyltin dilaurate.

Finally, the supramolecular compound may be washed and dried, or even purified, according to the general knowledge of a person skilled in the art.

According to the second embodiment, the reaction may include the following steps:

(i) functionalization of the oil with a diisocyanate according to the reaction scheme:

(HB)-OH (1 eq.) + NCO-X-NCO (1 eq.) → (HB)-OC(O)-NH-X-NCO

and then

(iia) either reaction with 6-methylisocytosine:

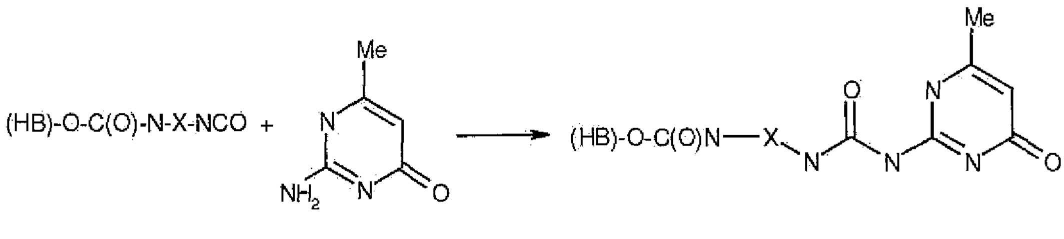

or

(iib) reaction with 5-hydroxyethyl-6-methylisocytosine:

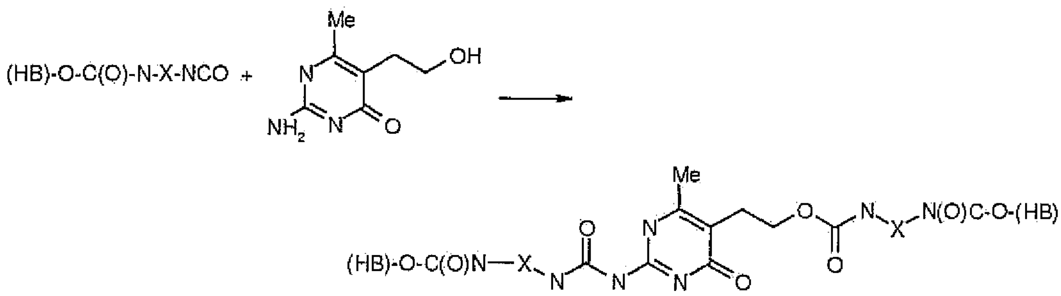

An illustration of such a reaction is given in Folmer et al., Adv. Mater., 12, 874-78 (2000).

The supramolecular compounds of the compositions according to the invention may especially correspond to the following structures:

- ureidopyrimidone-functionalized octyldodecanol of structure:

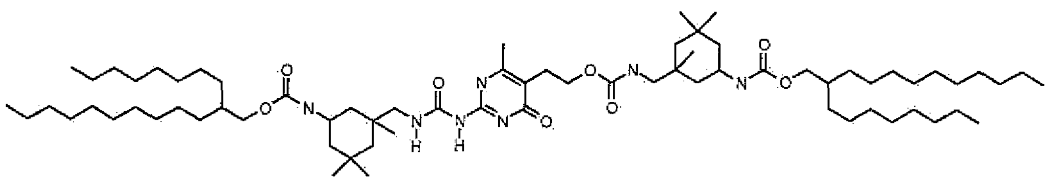

or of structure:

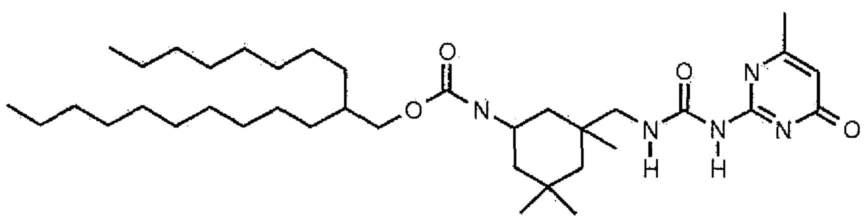

- ureidopyrimidone-functionalized diisostearyl malate of structure:

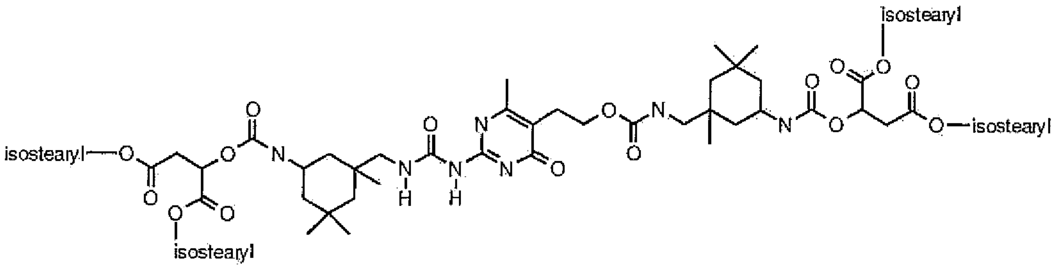

or of structure:

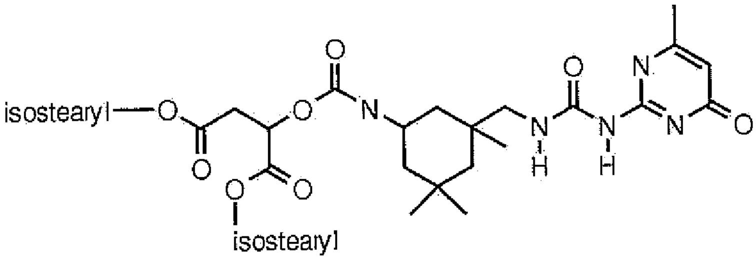

- ureidopyrimidone-functionalized castor oil of structure:

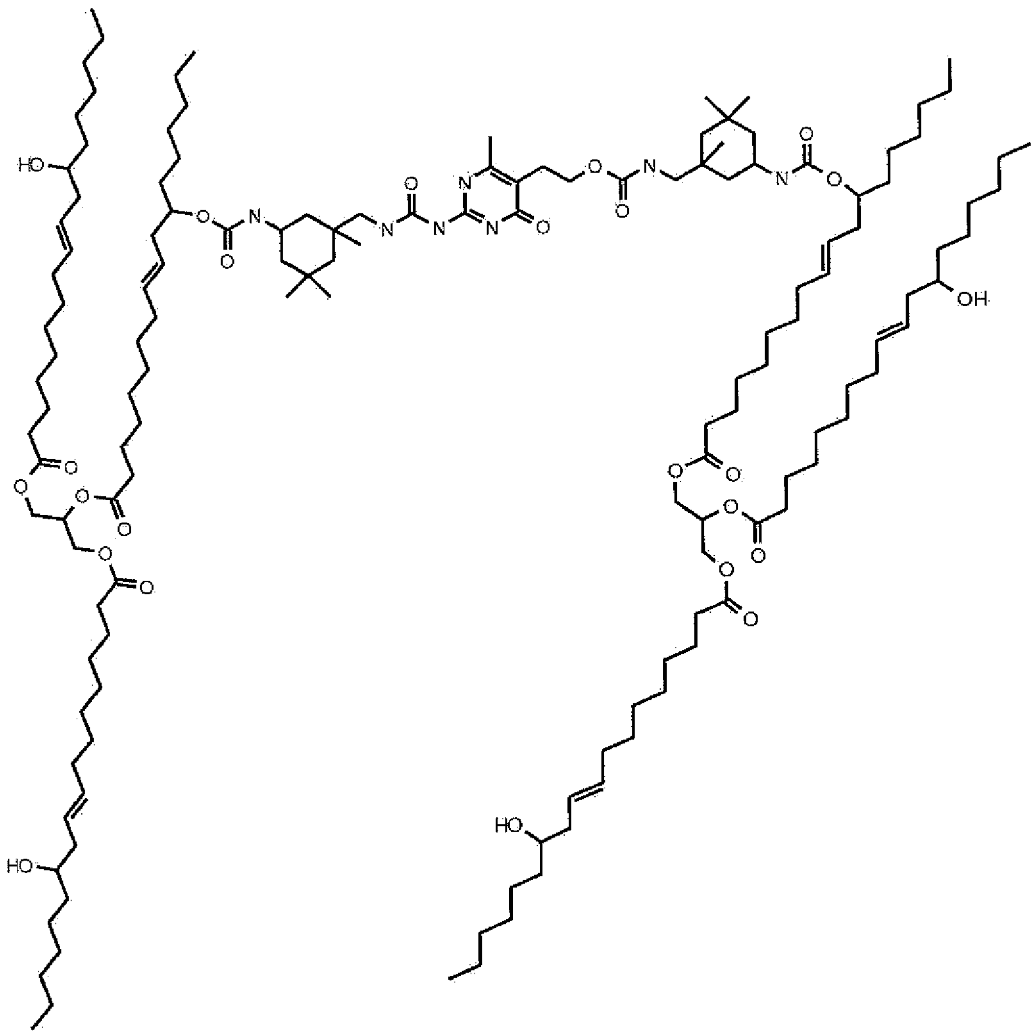

or of structure:

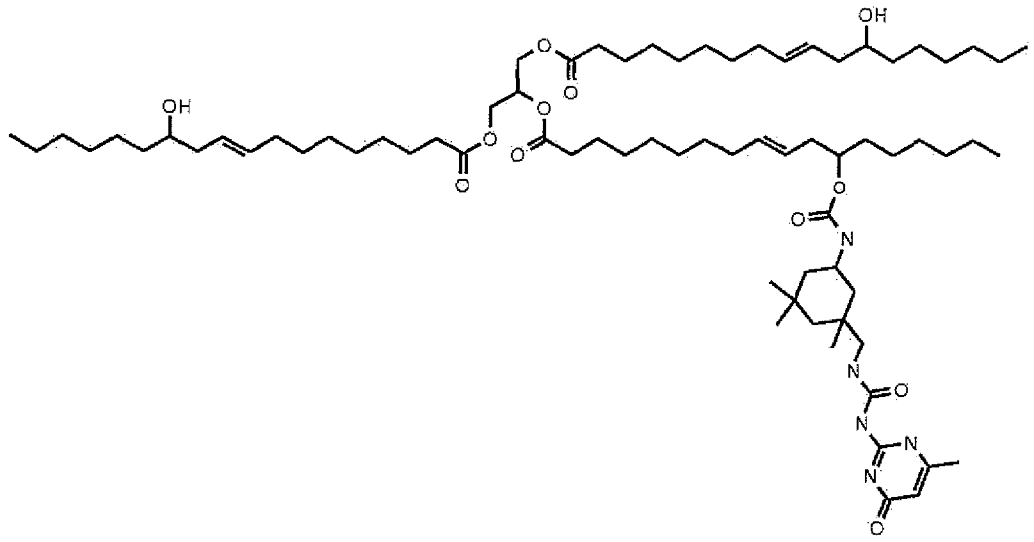

- ureidopyrimidone-functionalized 2-hexyldodecanol of structure:

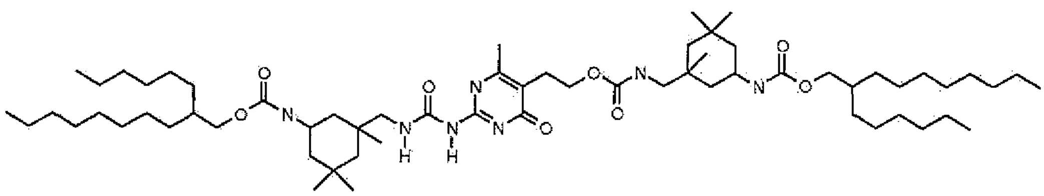

or of structure:

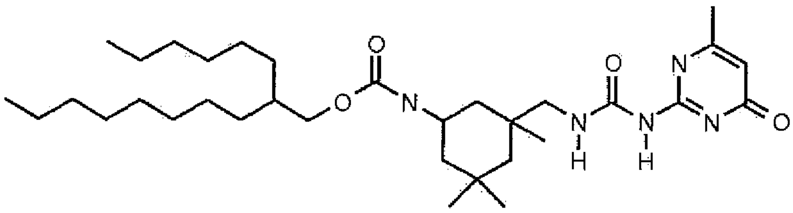

- ureidopyrimidone-functionalized 2-decyltetradecanol of structure:

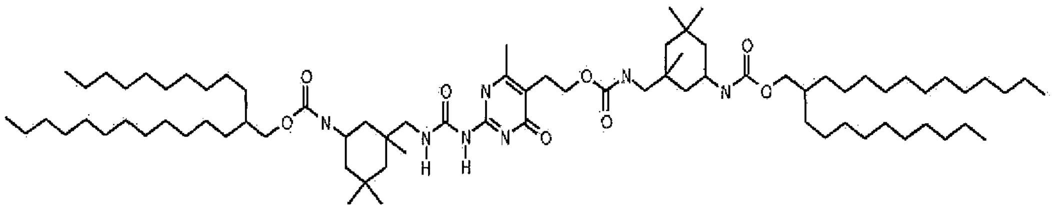

or of structure:

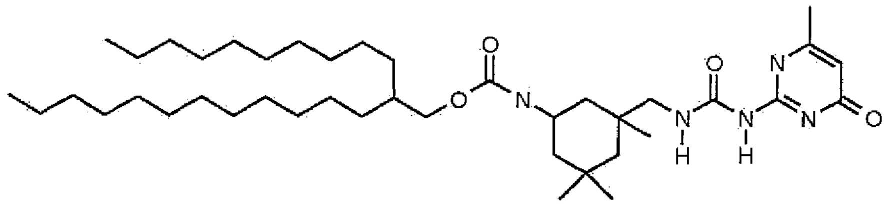

It has been found that the use of the compounds according to the invention may lead, after application of the composition to keratin materials, to the formation of a supramolecular polymer in the form of a physically crosslinked network, especially by means of hydrogen bonds, which is generally in the form of a film, and which has very good mechanical strength.

For the purposes of the invention, the term "supramolecular polymer" means a polymer chain or network formed from the assembly of non-polymeric compounds according to the invention with at least one other identical or different non-polymeric compound according to the invention, each assembly comprising at least one pair of identical or different paired junction groups.

For the purposes of the invention the term "pair of paired junction groups" means two junction groups, each of which may optionally be borne by the same compound according to the invention, the two groups being connected together via 4 H bonds.

Thus, the supramolecular polymer will have points of physical crosslinking provided by the H bonds between these pairs of junction groups. The physical crosslinking will ensure the maintenance and persistence of the cosmetic effect in a similar manner to chemical crosslinking, while at the same time allowing reversibility, i.e. the possibility of totally removing the deposit.

Preferably, the supramolecular compound according to the invention has a viscosity, measured at 125°C, of between 30 and 6000 mPa.s, especially between 150 and 4000 mPa.s, or even between 500 and 3500 mPa.s and better still between 750 and 3000 mPa.s.

The number-average molecular mass (Mn) of the supramolecular compound according to the invention is preferably between 180 and 8000, preferably from 200 to 6000, or even from 300 to 4000, better still from 400 to 3000 and preferentially from 500 to 1500.

The supramolecular compound according to the invention is advantageously soluble in the cosmetic oily media usually used, especially in plant oils, C6-C32 alkanes, C8-C32 fatty esters, C2-C7 short esters, C8-C32 fatty alcohols, and more particularly in media comprising at least isododecane, Parleam, isononyl isononanoate, octyldodecanol, a C12-C15 alkyl benzoate, butyl acetate or ethyl acetate, alone or as a mixture.

The term "soluble" means that the compound forms a clear solution in at least one solvent chosen from isododecane, Parleam, isononyl isononanoate, octyldodecanol, a C12-C15 alkyl benzoate, butyl acetate or ethyl acetate, in a proportion of at least 50% by weight, at 25°C.

The supramolecular compounds according to the invention may be used advantageously in a cosmetic composition, which moreover comprises a cosmetically acceptable medium, i.e. a medium that is compatible with keratin materials such as facial or bodily skin, the eyelashes, the eyebrows, the lips and the nails.

Preferably, the composition according to the invention has a content of supramolecular compound of between 5% and 95% by weight, preferably between 10% and 95% by weight and better still preferably between 20% and 90% by weight relative to the total weight of the composition.

As examples of supramolecular compounds that may be used in the compositions according to the invention, mention may be made of the following compounds:

**Compound 1: Ureidopyrimidone-functionalized octyldodecanol**

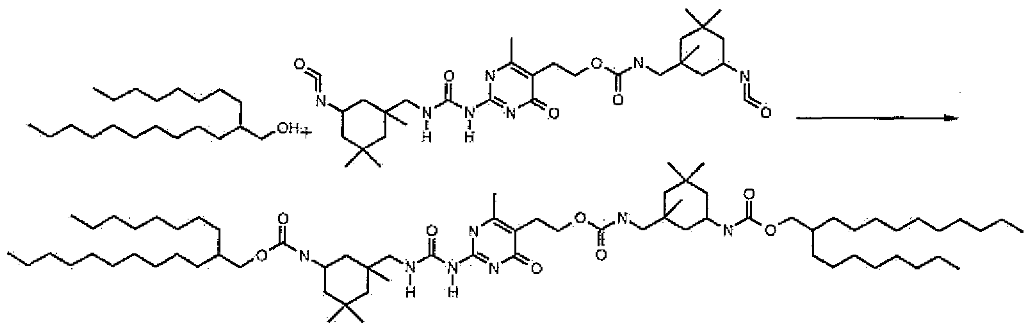

70 g of ureidopyrimidone diisocyanate are dissolved in methyltetrahydrofuran, under argon. 80.3 g of octyldodecanol in 100 ml of dichloromethane are added, under argon, followed by addition of 15 microlitres of dibutyltin dilaurate (catalyst). The reaction mixture is refluxed until the isocyanate peak (2250-2265 $cm^{-1}$) has disappeared on IR spectrometry.

The excess octyldodecanol is removed by successive washing of the reaction medium with methanol, followed by three extractions and drying over $MgSO_4$. After evaporation of the organic phase, 103 g of a pale yellow powder, characterized by 1H NMR (structure in conformity), are obtained.

This powder may be conveyed in isododecane, for example at a concentration of 10% by weight; this concentration may especially be up to 60% by weight in isododecane, which then leads to a solution that is viscous but still manipulable. It is thus found that by functionalizing with a ureidopyrimidone, the oil changes from a liquid to a solid, which can be conveyed in isododecane at concentrations above 30%.

When a solution comprising 50% by weight of compound in isododecane is applied, after evaporating off the solvent, a glossy transparent film is obtained, which shows good adhesion by fragmentation, and low resistance to friction.

**Compound 2: Diisostearyl malate functionalized with a ureidopyrimidone**

15 g (0.0234 mol) of diisostearyl malate are dried under reduced pressure at 80°C for 4 hours. 7.21 g (0.0117 mol) of ureidopyrimidone diisocyanate dissolved in 60 ml of methyltetrahydrofuran, and 12 µl of dibutyltin dilaurate catalyst are added. The mixture is heated at 95°C, under argon, for 26 hours (disappearance of the characteristic band for isocyanates on IR spectroscopy). 20 ml of methyltetrahydrofuran are added to the reaction mixture, and the resulting mixture is then filtered through Celite. After evaporating off the solvent and drying under reduced pressure, a pale yellow solid is obtained.

**Compound 3: Castor oil functionalized with a ureidopyrimidone**

15 g of castor oil (0.016 mol) are dried under reduced pressure at 80°C for 4 hours. A solution of 4.9 g of ureidopyrimidone diisocyanate (0.008 mol) in 60 ml of methyltetrahydrofuran, and 12 µl of dibutyltin dilaurate catalyst are added. The mixture is heated at 90°C for 19 hours (total disappearance of the characteristic band for isocyanates on IR spectroscopy). At the end of the reaction, the solvent is evaporated off and the resulting product is dried under reduced pressure at 35°C overnight.

A pale yellow solid gum is obtained.

**Compound 4 (comparative to Example 1): Octyldodecanol functionalized with isophorone**

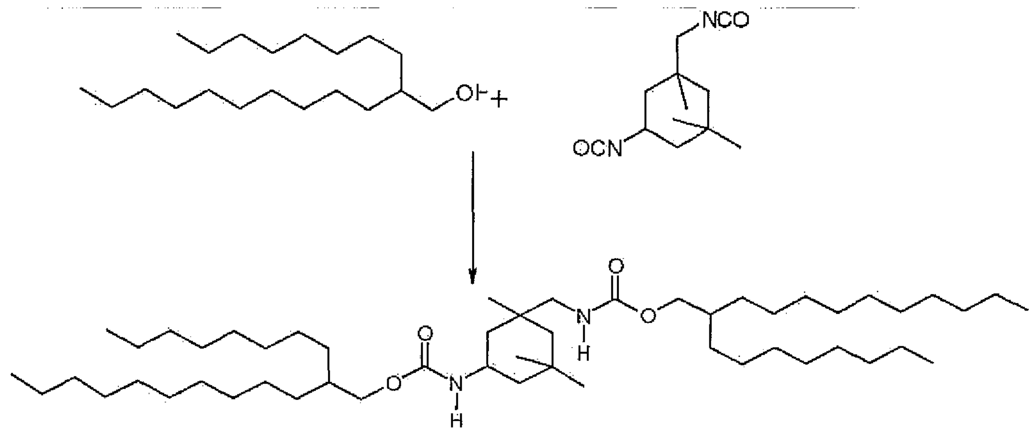

10 g of octyldodecanol are dried under reduced pressure at 80°C for 2 hours, followed by addition of 3.72 g of isophorone diisocyanate and 25 microlitres of dibutyltin dilaurate catalyst. The mixture is heated at 95°C under argon. The disappearance of the isocyanate is monitored by IR spectroscopy (disappearance of the band between 2250 and 2265 $cm^{-1}$, after heating for 12 hours).

A viscous oil that does not form a cohesive material is obtained.

**Compound 5 (comparative to Example 2): Diisostearyl malate functionalized with isophorone**

10 g (0.0159 mol) of diisostearyl malate are dried under reduced pressure at 80°C for 3 hours. 1.77 g (0.079 mol) of isophorone diisocyanate and 2.5 μl of catalyst (dibutyltin dilaurate) are added under argon, and the reaction mixture is heated at 95°C for 16 hours. During the reaction, the viscosity of the reaction medium increases. The reaction is stopped after disappearance of the characteristic peak for isocyanates on IR spectroscopy.

**Compound 6 (comparative to Example 3) : Castor oil functionalized with isophorone**

15 g (0.016 mol) of castor oil are dried under reduced pressure at 80°C for 6 hours. 1.78 g (0.008 mol) of isophorone diisocyanate and 12 μl of dibutyltin dilaurate catalyst are added, and the mixture is heated at 90°C for 16 hours. The reaction is stopped after disappearance of the characteristic peak for isocyanates on IR spectroscopy.

**Example 7**

The compounds prepared in Examples 1 to 6 are observed, visually and by feel, and the results are summarized in the following table:

| | physical appearance of the compound | Appearance of the film * Refractive index ** (refractive index unfunctionalized oil) |
|---|---|---|
| Compound 1 | Yellow solid | Glossy tacky film, which does not dewet; uniform deposit. No transfer onto the fingers. 1.488 (1.46) |
| Compound 4 (comparative) | Transparent viscous oil | Film which dewets; non-uniform deposit. Transfers onto the fingers. 1.474 (1.46) |
| Compound 2 | Yellow solid | Glossy, sparingly tacky film, which does not dewet; uniform deposit. No transfer onto the fingers. 1.478 (1.462) |
| Compound 5 (comparative) | Transparent viscous oil | Glossy tacky film which dewets; non-uniform deposit. No transfer onto the fingers. 1.4598 (1.462) |
| Compound 3 | Yellow solid (solid gum) | Glossy, slightly tacky film; behaviour of a fragile solid, which does not dewet; uniform deposit. No transfer onto the fingers. 1.4852 (1.48) |

(continued)

| | physical appearance of the compound | Appearance of the film * Refractive index ** (refractive index unfunctionalized oil) |
|---|---|---|
| Compound 6 (comparative) | Transparent viscous oil | Very tacky glossy film, which dewets; non-uniform deposit. Transfers onto the fingers. 1.4813 (1.48) |
| * The films are formed from a solution containing 40% by weight of the compound, either in isododecane for Examples 1-2 and 4-5, or in tetrahydrofuran for compounds 3 and 6. ** For the refractive index measurements, all the films are formed from a solution containing 40% by weight of the compound in tetrahydrofuran; the refractive index is measured after evaporating off the solvent. | | |

The term "film which does not dewet" means that, after deposition and evaporation of the solvent, a continuous, uniform "true" film is obtained.

The term "film which dewets" means that, after deposition and evaporation of the solvent, a non-uniform, discontinuous film "with holes" is obtained.

A tribometry test is performed on these deposits/films: the films are formed from a solution at 40% by weight in tetrahydrofuran, by deposition onto a nitrile elastomer, followed by drying for 24 hours at 25°C.

The tests are performed using a CSEM tribometer and equipped with a ball 6 mm in diameter. This ball, subjected to a 0.15 N load, rubs repeatedly on a film (10 to 20 μm thick). The rotation speed of the disk is set at 6.3 cm/s, which corresponds to a frequency of one revolution per second. The test is ended when wear is complete, or else is stopped after 1000 stress revolutions.

| | Observations |
|---|---|
| Compound 1 | The film remains unchanged (uniform) for 300 revolutions (no wear or brittleness); the material is thus cohesive; behaviour of a solid. |
| Compound 4 (comparative) | No measurement possible: the material has no cohesion, and behaves like an oil. |
| Compound 2 | The film remains unchanged (uniform) for 1000 revolutions (no wear or brittleness) ; the material is thus cohesive and does not wear out |
| Compound 5 (comparative) | The material behaves like an oil, with a buttering effect when it is subjected to the wear test. |
| Example 3 | The film is sparingly brittle but remains unchanged for 10 revolutions; after 10 revolutions, the wear is more pronounced; this reflects the behaviour of a solid. |
| Compound 6 (comparative) | No measurement possible since no film was initially formed: behaviour of an oil. |

It is thus found that there is no decrease in the refractive index after functionalization. The oil keeps its glossy nature, even when functionalized. It is also found that functionalization with ureidopyrimidones leads to films that are more or less tacky, but that do not transfer onto the fingers, unlike the comparative films.

Furthermore, and principally, in the case of the oils functionalized with isophorone (comparative), the films dewet and do not form a uniform deposit. In contrast, the films obtained with the compounds according to the invention do not dewet and are uniform and cohesive. The tribometry results confirm the cohesion properties obtained with the compounds of the invention.

Functionalization with ureidopyrimidones thus leads to materials that are cohesive enough to be able to ensure remanence of the deposit, which, incidentally, is glossy, superior to the remanence of the prior art (isophorone).

In summary: the gloss is maintained, the cohesion of the deposit is improved, and thus its remanence is improved.

**Compound 8: Diisostearyl malate functionalized with a ureidopyrimidone**

Preparation protocol

Preparation of the supramolecular oil: Diisostearyl malate functionalized with a ureidopyrimidone

150 g of diisostearyl malate were added over 1 hour 20 minutes at 50°C to a solution of 57.4 g of isophorone diisocyanate and 38.18 g of methyl isocytosine, in the presence of the catalyst dibutyltin dilaurate, with control of the

exothermicity and under an inert atmosphere. Stirring was continued for 55 minutes at 50°C after the addition, and 50 ml of propylene carbonate were then added. The temperature of the reaction medium was then raised to 140°C with a contact time of 2 hours, with stirring. The temperature of the reaction medium was then lowered to 70°C, the medium was neutralized by adding 30 ml of ethanol, and stirring was continued for 1 hour.

After adding 780 ml of ethyl acetate, the medium was filtered through Celite. After evaporating off the ethyl acetate, 400 ml of cyclohexane were added to the reaction medium, and the mixture was washed twice with an $H_2O$/EtOH mixture (2v/1v) saturated with NaCl. The organic phase was then stripped with isododecane, down to a viscous liquid, corresponding to the desired molecule in a solids content of 50%. For the purposes of the formulation, this dry extract may optionally be modified by adding isododecane to the medium.

**Compound 9: 2-Hexyldecanol functionalized with ureidopyrimidone**

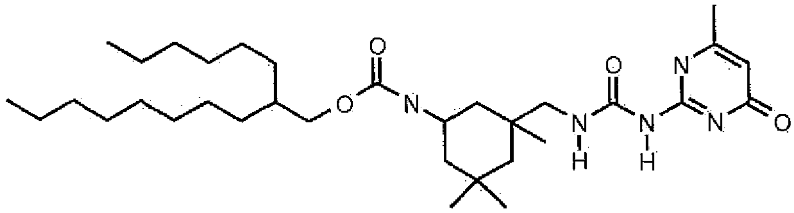

126.4 g of 2-hexyldecanol are heated at 60°C under reduced pressure for 2 hours to dry them. After 2 hours, the oil is allowed to cool to 20°C under argon, and is then added slowly, over 5 hours, to a mixture of 116 g of isophorone diisocyanate and 55 mg of DBTL catalyst at 50°C. At the end of the addition, the temperature of the reaction mixture is brought to 110°C, and 90 ml of propylene carbonate and 78.4 g of 6-methylisocytosine are then added, which produces a homogeneous white suspension. Stirring is continued at 110°C for 2 hours, and the disappearance of the isocyanate is monitored by infrared spectroscopy. The disappearance of the peak at 2250 $cm^{-1}$ is observed. In parallel, disappearance of the amine originating from the isocytosine is monitored by means of an amine assay. At the end of the reaction, 500 g of isododecane are added, at 100°C, and a slightly cloudy pale yellow solution is obtained. 300 ml of ethanol are added and stirring is continued for 2 hours. After filtering through Celite, the reaction mixture is stripped with isododecane at 80°C in order to remove the alcohol and the propylene carbonate.

Finally, the desired product conveyed in isododecane, in a solids content of 50%, is obtained. The product is especially characterized by HPLC and GPC (structure confirmed).

**Compound 10: 2-Hexyldecanol functionalized with ureidopyrimidone**

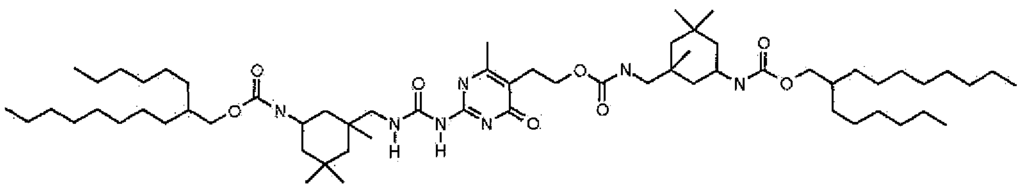

173.1 g of 2-hexyldecanol are heated at 60°C under reduced pressure for 2 hours to dry them. After 2 hours, the oil is allowed to cool to 50°C under argon, and is then added slowly, over 5 hours, to a mixture of 158.7 g of isophorone diisocyanate and 77 mg of DBTL catalyst at 50°C. At the end of the addition, the temperature of the reaction mixture is brought to 110°C, and 150 ml of propylene carbonate and 60.3 g of 5-hydroxyethyl-6-methylisocytosine are added, which produces a uniform white suspension. Stirring is continued at 110°C for 5 hours, and the disappearance of the isocyanate is monitored by infrared spectroscopy. The disappearance of the peak at 2250 $cm^{-1}$ is observed. At the end of the reaction, the temperature of the reaction medium is reduced to 100°C, and 780 g of isododecane are added; a pale yellow cloudy mixture is obtained. 100 ml of ethanol are added and stirring is continued for 2 hours. After filtering through Celite, the reaction mixture is stripped with isododecane at 80°C in order to remove the alcohol and the propylene carbonate.

Finally, the desired product conveyed in isododecane, in a solids content of 50%, is obtained. The product is especially characterized by HPLC and GPC (structure confirmed).

**Compound 11: 2-Decyltetradecanol functionalized with ureidopyrimidone**

126 g of 2-decyltetradecanol are heated at 100°C under reduced pressure for 4 hours to dry them. After 2 hours, the oil is added, over 4 hours, at 50°C and under argon, to a mixture of 94.7 g of isophorone diisocyanate and of DBTL catalyst (qs). Monitoring by assay of the isocyanate allows the reaction progress to be followed; at half-equivalence, 126 g of propylene carbonate and 53.3 g of 6-methylisocytosine are added. Stirring and heating are continued at 100°C for 16 hours, and disappearance of the isocyanate is monitored by infrared spectroscopy. The disappearance of the peak at 2250 $cm^{-1}$ is observed. In parallel, disappearance of the amine originating from the isocytosine is monitored by means of an amine assay. At the end of the reaction, the temperature is cooled to 50°C, 100 ml of ethanol are added and stirring is continued for 5 hours. After filtering through Celite and stripping with isododecane, the desired product conveyed in isododecane, at a solids content of 50%, is obtained. The product is especially characterized by GPC and HPLC coupled to mass spectroscopy.

**Compound 12: Ureidopyrimidone-functionalized Jarcol 24 (J24)**

200 g of Jarcol 1-24 are added at 50°C to IPDI (1.1 eq. IPDI) in the presence of the catalyst, with control of the exothermicity and under an inert atmosphere. Stirring is continued after the addition, for 30 minutes at 50°C. 1.3 equivalents of methylisocytosine (MIC) are then added to the mixture, followed by addition of 100 ml of propylene carbonate. The temperature of the reaction medium is then raised to 140°C, with a contact time of 1 hour at 140°C. The disappearance of the isocyanate functions is monitored by infrared spectroscopy, and the temperature of the medium is then lowered to 70°C, followed by addition of 30 ml of ethanol and stirring for 1 hour. After addition of ethyl acetate, the medium is filtered through filter paper. After evaporating off the ethyl acetate, cyclohexane is added, followed by 5 washes with a mixture of water saturated with NaCl/ethanol (2v/1v). The organic phase is then dried over $Na_2SO_4$, filtered and stripped with isododecane. A solution with a 50% solids content of oil functionalized with a ureidopyrimidone is then obtained.

**Compound 13: Ureidopyrimidone-functionalized Jarcol 20 (J20)**

180 g of Jarcol I-20 are added at 50°C to IPDI (1.1 eq. IPDI) in the presence of the catalyst, with control of the exothermicity and under an inert atmosphere.

Stirring is continued for 30 minutes at 50°C. 1.3 equivalents of MIC are added to the reaction medium, followed by addition of 100 ml of propylene carbonate.

The temperature of the reaction medium is then raised to 140°C, with a contact time of 1 hour at 140°C. The reaction is monitored by infrared spectroscopy, with monitoring of the disappearance of the characteristic peak of the isocyanate function. The temperature is then lowered to 70°C, followed by addition of 30 ml of ethanol and stirring for 1 hour. After addition of ethyl acetate, the medium is filtered through filter paper. After evaporating off the ethyl acetate, cyclohexane is added, followed by 5 washes with a mixture of water saturated with NaCl/ethanol (2v/1v). The organic phase is then dried over $Na_2SO_4$, filtered and stripped with isododecane. A solution with a 50% solids content of oil functionalized with a ureidopyrimidone is then obtained.

Preferably, the composition according to the invention has a content of supramolecular compound of between 5% and 95% by weight, preferably between 10% and 95% by weight and better still preferably between 20% and 90% by weight relative to the total weight of the composition.

**Oil chosen from apolar hydrocarbon -based oils and silicone oils**

The composition according to the invention comprises at least one oil, chosen from apolar hydrocarbon-based oils and silicone oils.

The term "oil" means a water-immiscible non-aqueous compound that is liquid at room temperature (25°C) and at atmospheric pressure (760 mmHg).

According to a first embodiment, the oil present in the composition and chosen from silicone oils is volatile. According to a second embodiment, the oil present in the composition and chosen from apolar hydrocarbon-based oils and silicone oils is non-volatile.

The term "volatile oil" means an oil (or non-aqueous medium) that can evaporate on contact with the skin in less than one hour, at room temperature and atmospheric pressure. The volatile oil is a volatile cosmetic oil, which is liquid at room temperature, especially having a non-zero vapour pressure, at room temperature and atmospheric pressure, in particular having a vapour pressure ranging from 0.13 Pa to 40 000 Pa ($10^{-3}$ to 300 mmHg), preferably ranging from 1.3 Pa to 13 000 Pa (0.01 to 100 mmHg) and preferentially ranging from 1.3 Pa to 1300 Pa (0.1 to 10 mmHg).

The term "non-volatile oil" means an oil that remains on keratin materials at room temperature and atmospheric pressure for at least several hours and that especially has a vapour pressure of less than $10^{-3}$ mmHg (0.13 Pa). A non-volatile

oil may also be defined as having an evaporation rate such that, under the conditions defined previously, the amount evaporated after 30 minutes is less than 0.07 mg/cm$^2$.

**Silicone oil**

According to a first embodiment, the oil present in the composition according to the invention is a silicone oil.
The term "silicone oil" means an oil containing at least one silicon atom, especially containing Si-O groups.
In particular, the silicone oil may be chosen from phenyl silicone oils.
Such an oil is also referred to as a "phenyl silicone". The term "phenyl silicone" means an organopolysiloxane substituted with at least one phenyl group.
The phenyl silicone oil is preferably non-volatile.
The term "non-volatile oil" means an oil that remains on keratin materials at room temperature and atmospheric pressure for at least several hours and that especially has a vapour pressure of less than $10^{-3}$ mmHg (0.13 Pa). A non-volatile oil may also be defined as having an evaporation rate such that, under the conditions defined previously, the amount evaporated after 30 minutes is less than 0.07 mg/cm$^2$. These oils may be of plant, mineral or synthetic origin.
Preferably, the phenyl silicone oil is present in the composition in a total content ranging from 0.5% to 70% by weight, preferably ranging from 3% to 50% by weight and better still ranging from 5% to 40% by weight relative to the total weight of the composition. Preferably, the weight-average molecular weight of the phenyl silicone oil is between 500 and 10 000 g/mol.
The silicone oil may be chosen from phenyl trimethicones, phenyl dimethicones, phenyltrimethylsiloxydiphenylsiloxanes, diphenyl dimethicones, diphenyl methyldiphenyl trisiloxanes and 2-phenylethyl trimethylsiloxysilicates.
The silicone oil may correspond to the formula:

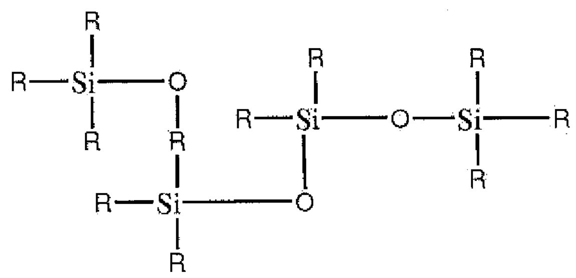

in which the groups R represent, independently of each other, a methyl or a phenyl. Preferably, in this formula, the silicone oil comprises at least three phenyl groups, for example at least four, at least five or at least six.
According to another embodiment, the silicone oil corresponds to the formula:

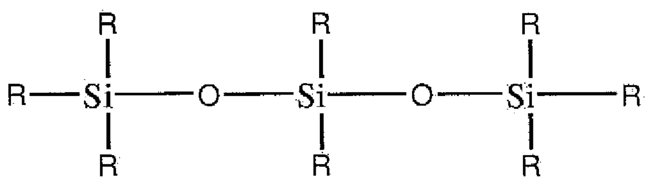

(V

in which the groups R represent, independently of each other, a methyl or a phenyl. Preferably, in this formula, the said organopolysiloxane comprises at least three phenyl groups, for example at least four or at least five.
Mixtures of the phenyl organopolysiloxanes described previously may be used.
Examples that may be mentioned include mixtures of triphenyl, tetraphenyl or pentaphenyl organopolysiloxanes.
According to another embodiment, the silicone oil corresponds to the formula:

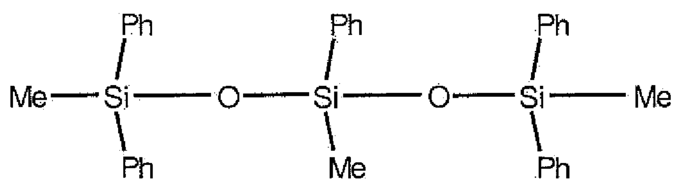

in which Me represents methyl, Ph represents phenyl. Such a phenyl silicone is especially manufactured by Dow Corning

under the reference PH-1555 HRI from Dow Corning (chemical name: 1,3,5-trimethyl 1,1,3,5,5-pentaphenyl trisiloxane; INCI name: trimethyl pentaphenyl trisiloxane). The reference Dow Corning 554 Cosmetic Fluid may also be used.

According to another embodiment, the silicone oil corresponds to the formula:

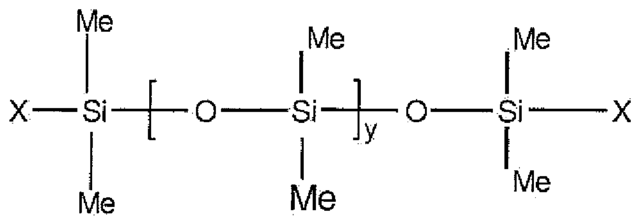

in which Me represents methyl, y is between 1 and 1000 and X represents $-CH_2-CH(CH_3)(Ph)$ .

According to another embodiment, the silicone oil corresponds to the formula:

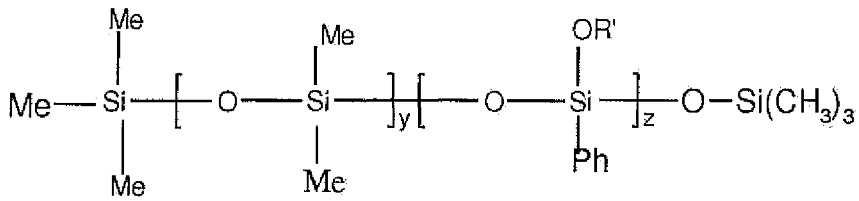

in which -OR' represents $-O-SiMe_3$, y is between 1 and 1000 and z is between 1 and 1000.

The phenyl silicone oil may be chosen from the phenyl silicones of formula (VI) below:

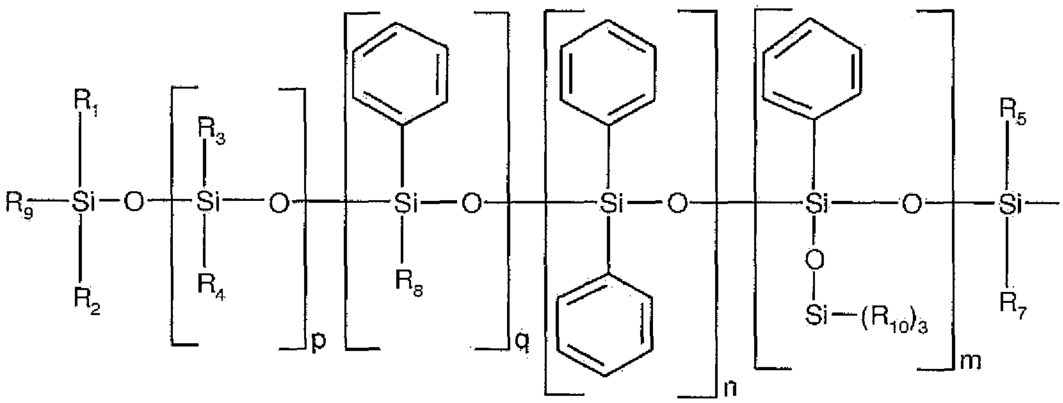

(VI)

in which

- R1 to R10 independently of each other, are saturated or unsaturated, linear, cyclic or branched C1-C30 hydrocarbon-based radicals,
- m, n, p and q are, independently of each other, integers between 0 and 900, with the proviso that the sum m + n + q is other than 0.

Preferably, the sum m + n + q is between 1 and 100. Preferably, the sum m + n + p + q is between 1 and 900, and better still between 1 and 800. Preferably, q is equal to 0.

The phenyl silicone oil may be chosen from the phenyl silicones of formula (VII) below:

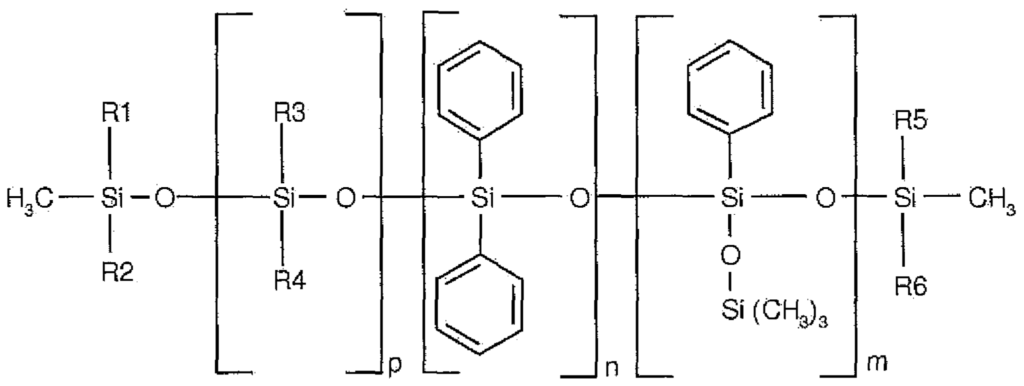 (VII)

in which

- R1 to R6, independently of each other, are saturated or unsaturated, linear, cyclic or branched C1-C30 hydrocarbon-based radicals,
- m, n and p are, independently of each other, integers between 0 and 100, with the proviso that the sum n + m is between 1 and 100.

Preferably, R1 to R6, independently of each other, represent a saturated, linear or branched C1-C30 and especially C1-C12 hydrocarbon-based radical and in particular a methyl, ethyl, propyl or butyl radical.
R1 to R6 may especially be identical, and in addition may be a methyl radical.
Preferably, the value m = 1 or 2 or 3, and/or n = 0 and/or p = 0 or 1 may apply, in formula (VII).
A phenyl silicone oil of formula (VI) with a viscosity at 25°C of between 5 and 1500 $mm^2/s$ (i.e. 5 to 1500 cSt), and preferably with a viscosity of between 5 and 1000 $mm^2/s$ (i.e. 5 to 1000 cSt) may be used.
As phenyl silicone oil of formula (VII), it is especially possible to use phenyl trimethicones such as DC556 from Dow Corning (22.5 cSt), the oil Silbione 70653V30 from Rhône Poulenc (28 cSt) or diphenyl dimethicones such as Belsil oils, especially Belsil PDM1000 (1000 cSt), Belsil PDM 200 (200 cSt) and Belsil PDM 20 (20 cSt) from Wacker. The values in parentheses represent the viscosities at 25°C.
The non-volatile silicone oil may be chosen from the silicones of formula:

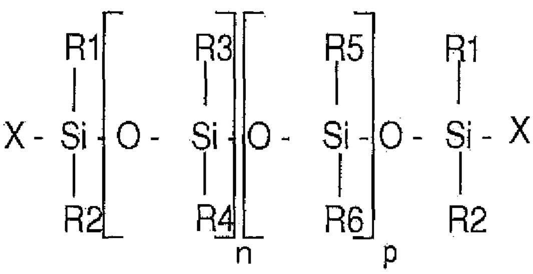

in which:

$R_1$, $R_2$, $R_5$ and $R_6$ are, together or separately, an alkyl radical containing 1 to 6 carbon atoms,
$R_3$ and $R_4$ are, together or separately, an alkyl radical containing from 1 to 6 carbon atoms or an aryl radical,
X is an alkyl radical containing from 1 to 6 carbon atoms, a hydroxyl radical or a vinyl radical,
n and p being chosen so as to give the oil a weight-average molecular mass of less than 200 000 g/mol, preferably less than 150 000 g/mol and more preferably less than 100 000 g/mol.

According to one embodiment, the non-volatile silicone oils that may be used in the composition according to the invention may be phenyl silicone oils, for instance the trimethylsiloxyphenyl dimethicone sold under the reference Belsil PDM 1000 by the company Wacker (MW = 9000 g/mol) or 1,3,5-trimethyl-1,1,3,5,5-pentaphenyltrisiloxane, sold by the company Dow Corning under the reference PH-1555 HRI from Dow Corning (TNCI name: trimethyl pentaphenyl trisiloxane), phenyl trimethicones (such as the phenyl trimethicone sold under the trade name DC 556 by Dow Corning), phenyl dimethicones, phenyltrimethylsiloxydiphenylsiloxanes, diphenyl dimethicones and diphenylmethyldiphenyltrisiloxanes.

Preferably, the phenyl silicone oil is non-volatile. Preferably, the phenyl silicone oil has a viscosity at 25°C of greater than 50 cSt and preferably greater than 100 cSt.

**Protocol for measuring the viscosity:**

The viscosity is measured at 25°C ± 0.5°C using a Haake RS75 controlled-stress rheometer from the company Thermo Rheo equipped with a spindle of cone/plate geometry with a diameter of between 2 cm and 6 cm and an angle of between 1° and 2°, the choice of the spindle depending on the viscosity to be measured (the more fluid the oil, the greater the diameter of the chosen cone and the smaller the angle). The measurement is performed by applying on the oil sample a logarithmic shear stress gradient ranging from 0.2 Pa to 1000 Pa for a duration of 20 minutes. The rheogram representing the change in viscosity as a function of the rate of shear $\varepsilon'$ is then plotted. The rheogram shows a plateau at low shear rate values (known as a Newtonian plateau); this plateau corresponds to a stable viscosity value, which is the viscosity of the oil thus determined.
Note: in general, the term "between" includes the limit values of the range.
According to another embodiment, the silicone oil may also be chosen from non-phenyl silicone oils. Such an oil may be chosen from silicone oils with a flash point ranging from 40°C to 150°C and preferably with a flash point of greater than 55°C and less than or equal to 105°C, preferentially ranging from 65°C to 95°C. The flash point is measured in particular according to standard ISO 3679.
The non-phenyl silicone oil may be volatile or non-volatile.
A volatile silicone oil may be chosen from linear or cyclic silicone oils such as linear or cyclic polydimethylsiloxanes (PDMSs) containing from 3 to 7 silicon atoms.
Linear PDMSs (also known as dimethicones) are linear dimethylpolysiloxane compounds with trimethylsiloxy end groups. Cyclic PDMSs are generally known as cyclomethicones. This is a generic name targeting cyclic dimethylpolysiloxane compounds.
Examples of such oils that may be mentioned include octyl trimethicone, hexyl trimethicone, decamethylcyclopentasiloxane (cyclopentasiloxane or D5), octamethylcyclotetrasiloxane (cyclotetradimethylsiloxane or D4), dodecamethylcyclohexasiloxane (D6), decamethyltetrasiloxane (L4), KF 96 A from Shin-Etsu, the polydimethylsiloxanes such as those sold under the reference DC 200 (1.5 cSt), DC 200 (5 cSt) and DC 200 (3 cSt) by Dow Corning.
Examples of silicone oils that may also be mentioned include polydimethylsiloxanes comprising alkyl or alkoxy groups, which are pendent and/or at the end of a silicone chain, these groups each containing from 2 to 24 carbon atoms :

**Hydrocarbon-based apolar oils**

According to a second embodiment, the oil present in the composition according to the invention is a non-volatile apolar hydrocarbon-based oil.
For the purposes of the present invention, the term "apolar oil" means an oil whose solubility parameter at 25°C, $\delta_a$, is equal to 0 $(J/cm^3)^{1/2}$.
The definition and calculation of the solubility parameters in the Hansen three-dimensional solubility space are described in the article by C.M. Hansen: "The three dimensional solubility parameters", J. Paint Technol. 39, 105 (1967).
According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{½}$.

The parameters $\delta_p$, $\delta_h$, $\delta_D$ and $\delta_a$ are expressed in $(J/cm^3)^{½}$.
The term "apolar hydrocarbon-based oil" means an oil formed essentially from, or even constituted by, carbon and hydrogen atoms, and free of heteroatoms such as N, O, Si and P.
Examples of non-volatile apolar hydrocarbon-based oils that may be mentioned include hydrocarbon-based oils, for instance squalene, linear or branched hydrocarbons such as liquid paraffin, liquid petroleum jelly and naphthalene oil, hydrogenated or partially hydrogenated polyisobutene, isoeicosane, squalane, decene/butene copolymers, polybutene/polyisobutene copolymers, especially Indopol L-14, and polydecenes such as Puresyn 10, and mixtures thereof.
In particular, mention may be made of non-volatile hydrocarbon-based apolar oils of high molecular mass, for example between 650 and 10 000 g/mol, for instance:

- polybutylenes such as Indopol H-100 (molar mass or MW = 965 g/mol), Indopol H-300 (MM = 1340 g/mol) and (Indopol H-1500 (MW = 2160 g/mol) sold or manufactured by the company Amoco,
- hydrogenated polyisobutylenes such as Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol),
- polydecenes and hydrogenated polydecenes such as Puresyn 150 (MM = 9200 g/mol) sold by the company Mobil Chemicals, and
- mixtures thereof.

The composition may also comprise a volatile apolar hydrocarbon-based oil, especially having a flash point ranging from 40 °C to 102°C, preferably ranging from 40°C to 55°C and preferentially ranging from 40°C to 50°C.

Apolar hydrocarbon-based volatile oils that may be mentioned include hydrocarbon-based volatile oils containing from 7 to 16 carbon atoms, and preferably from 8 to 16 carbon atoms, and mixtures thereof, especially branched $C_8$-$C_{16}$ alkanes such as $C_8$-$C_{16}$ isoalkanes (also known as isoparaffins), isododecane, isodecane, isohexadecane and, for example, the oils sold under the trade names Isopar or Permethyl, and mixtures thereof.

According to one embodiment, the volatile or non-volatile apolar oils that are suitable for use in the invention may be chosen from polybutene, polyisobutene, hydrogenated polyisobutene, isododecane and isohexadecane, and mixtures thereof.

Apolar hydrocarbon-based volatile oils that may be mentioned include hydrocarbon-based volatile oils containing from 7 to 15 carbon atoms and mixtures thereof, and especially linear $C_7$-$C_{15}$ alkanes. Preferably, the "volatile linear alkanes" that are suitable for use in the invention comprise from 8 to 14 carbon atoms.

Preferably, the "volatile linear alkanes" that are suitable for use in the invention comprise from 9 to 14 carbon atoms.

Preferably, the "volatile linear alkanes" that are suitable for use in the invention comprise from 10 to 14 carbon atoms.

Preferably, the "volatile linear alkanes" that are suitable for use in the invention comprise from 11 to 14 carbon atoms.

The "volatile linear alkanes" that may be used in the compositions according to the invention may in particular have a non-zero vapour pressure (also known as the saturating vapour pressure), at room temperature, in particular a vapour pressure ranging from 0.3 Pa to 6000 Pa.

Preferably, the "volatile linear alkanes" that are suitable for use in the invention have a vapour pressure ranging from 0.3 to 2000 Pa, at room temperature (25°C).

Preferably, the "volatile linear alkanes" that are suitable for use in the invention have a vapour pressure ranging from 0.3 to 1000 Pa, at room temperature (25°C).

More preferably, the "volatile linear alkanes" that are suitable for use in the invention have a vapour pressure ranging from 0.4 to 600 Pa, at room temperature (25°C).

Preferably, the "volatile linear alkanes" that are suitable for use in the invention have a vapour pressure ranging from 1 to 200 Pa, at room temperature (25°C).

Even more preferably, the "volatile linear alkanes" that are suitable for use in the invention have a vapour pressure ranging from 3 to 60 Pa, at room temperature (25°C).

According to one embodiment, a volatile linear alkane that is suitable for use in the invention may have a flash point that is in the range from 30 to 120°C and more particularly from 40 to 100°C. The flash point is in particular measured according to standard ISO 3679. A volatile linear alkane that is suitable for use in the invention may advantageously be of plant origin. Preferably, the volatile linear alkane or the mixture of volatile linear alkanes present in the composition according to the invention comprises at least one $^{14}C$ (carbon-14) carbon isotope. In particular, the $^{14}C$ isotope may be present in a $^{14}C/^{12}C$ ratio of greater than or equal to $1\times10^{-16}$, preferably greater than or equal to $1x10^{-15}$, more preferably greater than or equal to $7.5x10^{-14}$ and better still greater than or equal to $1.5x10^{-13}$. Preferably, the ratio $^{14}C/^{12}C$ ranges from $6\times10^{-13}$ to $1.2\times10^{-12}$.

The amount of $^{14}C$ isotopes in the volatile linear alkane or the mixture of volatile linear alkanes may be determined via methods known to those skilled in the art such as the Libby compacting method, liquid scintillation spectrometry or accelerator mass spectrometry.

Such an alkane may be obtained, directly or in several steps, from a plant raw material, such as an oil, a butter, a wax, etc.

As examples of alkanes that are suitable for use in the invention, mention may be made of the alkanes described in patents WO 2007/068 371 or WO 2008/155 059 of the company Cognis (mixtures of different alkanes differing by at least one carbon). These alkanes are obtained from fatty alcohols, which are themselves obtained from coconut oil or palm oil.

As examples of linear alkanes that are suitable for use in the invention, mention may be made of n-heptane

($C_7$), n-octane ($C_8$), n-nonane ($C_9$), n-decane ($C_{10}$), n-undecane ($C_{11}$), n-dodecane ($C_{12}$), n-tridecane ($C_{13}$) and n-tetradecane ($C_{14}$), and mixtures thereof. According to one particular embodiment, the volatile linear alkane is chosen from n-nonane, n-undecane, n-dodecane, n-tridecane and n-tetradecane, and mixtures thereof. According to one preferred mode, mention may be made of mixtures of n-undecane ($C_{11}$) and of n-tridecane ($C_{13}$) obtained in Examples 1 and 2 of patent application WO 2008/155 059 of the company Cognis.

Mention may also be made of n-dodecane ($C_{12}$) and n-tetradecane ($C_{14}$) sold by Sasol under the references, respectively, Parafol 12-97 and Parafol 14-97, and also mixtures thereof.

The volatile linear alkane may also be used alone. Alternatively or preferentially, a mixture of two different volatile liquid linear alkanes, differing from each other by a carbon number n of at least 1, in particular differing from each other by a carbon number of 1 or 2, may be used.

According to a first embodiment, a mixture of at least two different volatile linear alkanes comprising from 10 to 14 carbon atoms and differing from each other by a carbon number of at least 1 is used. Examples that may especially be mentioned include mixtures of $C_{10}/C_{11}$, $C_{11}/C_{12}$ or $C_{12}/C_{13}$ volatile linear alkanes.

According to another embodiment, a mixture of at least two different volatile linear alkanes comprising from 10 to 14 carbon atoms and differing from each other by a carbon number of at least 2 is used. Examples that may especially be mentioned include mixtures of $C_{10}/C_{12}$ or $C_{12}/C_{14}$ volatile linear alkanes, for an even carbon number n, and the $C_{11}/C_{13}$ mixture for an odd carbon number n.

According to one preferred mode, a mixture of at least two different volatile linear alkanes comprising from 10 to 14 carbon atoms and differing from each other by a carbon number of at least 2, and in particular a mixture of $C_{11}/C_{13}$ volatile linear alkanes or a mixture of $C_{12}/C_{14}$ volatile linear alkanes, is used.

Other mixtures combining more than two volatile linear alkanes according to the invention, for instance a mixture of at least three different volatile linear alkanes comprising from 7 to 14 carbon atoms and differing from each other by a carbon number of at least 1, also form part of the invention, but mixtures of two volatile linear alkanes according to the invention are preferred (binary mixtures), the said two volatile linear alkanes preferably representing more than 95% and better still more than 99% by weight of the total content of volatile linear alkanes in a mixture. According to one particular mode of the invention, in a mixture of volatile linear alkanes, the volatile linear alkane having the smaller carbon number is predominant in the mixture.

According to another mode of the invention, a mixture of volatile linear alkanes in which the volatile linear alkane having the larger carbon number is predominant in the mixture is used.

As examples of mixtures that are suitable for use in the invention, mention may be made especially of the following mixtures:

- from 50% to 90% by weight, preferably from 55% to 80% by weight and more preferentially from 60% to 75% by weight of $C_n$ liquid volatile linear alkane with n ranging from 7 to 14,
- from 10% to 50% by weight, preferably from 20% to 45% by weight and preferably from 24% to 40% by weight of $C_{n+x}$ liquid volatile linear alkane with x greater than or equal to 1, preferably x = 1 or x = 2, with n+x between 8 and 14, relative to the total weight of alkanes in the said mixture.

In particular, the said mixture of alkanes according to the invention contains:

- less than 2% by weight and preferably less than 1% by weight of branched hydrocarbons,
- and/or less than 2% by weight and preferably less than 1% by weight of aromatic hydrocarbons,
- and/or less than 2% by weight, preferably less than 1% by weight and preferentially less than 0.1% by weight of unsaturated hydrocarbons in the mixture.

More particularly, a volatile linear alkane that is suitable for use in the invention may be used in the form of an n-undecane/n-tridecane mixture.

In particular, a mixture of volatile linear alkanes will be used comprising:

- from 55% to 80% by weight and preferably from 60% to 75% by weight of $C_{11}$ liquid volatile linear alkane (n-undecane),
- from 20% to 45% by weight and preferably from 24% to 40% by weight of $C_{13}$ liquid volatile linear alkane (n-tridecane),

relative to the total weight of alkanes in the said mixture.

According to one particular embodiment, the mixture of alkanes is an n-undecane/n-tridecane mixture. In particular, such a mixture may be obtained according to Example 1 or Example 2 of WO 2008/155 059.

According to another particular embodiment, the n-dodecane sold under the reference Parafol 12-97 by Sasol is used.

According to another particular embodiment, the n-tetradecane sold under the reference Parafol 14-97 by Sasol

is used.

According to yet another embodiment, a mixture of n-dodecane and n-tetradecane is used.

The composition of the invention may comprise from 1% to 70% by weight of volatile linear alkane(s), in particular from 0.5% to 60% by weight of volatile linear alkane(s) and more particularly from 5% to 40% by weight of volatile linear alkane(s) relative to the total weight of the composition.

According to one particular embodiment, the composition of the invention may comprise at least 50% by weight of volatile linear alkane(s) relative to the total content of volatile oil(s) in the composition. In particular, a composition of the invention may comprise at least 60%, more particularly at least 70%, and more particularly at least 80%, at least 90% or 100% of volatile linear alkane(s) relative to the total content of volatile oil(s) in the composition.

According to one embodiment, the composition may comprise a mixture of oils chosen from apolar hydrocarbon-based oils and silicone oils (i.e. more than one oil).

Preferably, in a composition according to the invention, the total content of oil chosen from apolar hydrocarbon-based oils and silicone oils ranges from 0.5% to 70% by weight relative to the total weight of the composition, preferably ranging from 3% to 50% by weight and better still ranging from 5% to 40% by weight relative to the total weight of the composition.

## Additional oils

A composition according to the invention may comprise, besides the oil(s) chosen from apolar hydrocarbon-based oils and silicone oils, at least one additional oil other than apolar hydrocarbon-based oils or silicone oils.

In particular, the said additional oil may be chosen from apolar hydrocarbon-based oils, or alternatively from fluoro oils.

The additional oil may be volatile or non-volatile.

For the purposes of the present invention, the term "polar oil" means an oil whose solubility parameter at 25°C, $\delta_a$, is other than 0 $(J/cm^3)^{1/2}$.

These additional oils may be of plant, mineral or synthetic origin.

The term "polar hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

The term "fluoro oil" means an oil containing at least one fluorine atom.

According to a first embodiment, the additional polar oil may be a non-volatile oil. In particular, the non-volatile polar oil may be chosen from the list of oils below, and mixtures thereof:

- hydrocarbon-based plant oils such as liquid triglycerides of fatty acids containing from 4 to 10 carbon atoms, for instance heptanoic or octanoic acid triglycerides or jojoba oil;
- hydrocarbon-based esters of formula RCOOR' in which RCOO represents a carboxylic acid residue containing from 2 to 30 carbon atoms, and R' represents a hydrocarbon-based chain containing from 1 to 30 carbon atoms, such as isononyl isononanoate, oleyl eructate or 2-octyldodecyl neopentanoate;
- polyesters obtained by condensation of an unsaturated fatty acid dimer and/or trimer and of diol, such as those described in patent application FR 0 853 634, in particular such as dilinoleic acid and 1,4-butanediol. Mention may be made especially in this respect of the polymer sold by Biosynthis under the name Viscoplast 14436H (INCI name: dilinoleic acid/butanediol copolymer) or copolymers of polyols and of diacid dimers, and esters thereof, such as Hailuscent ISDA,
- fatty alcohols containing from 12 to 26 carbon atoms, for instance octyldodecanol, 2-butyloctanol, 2-hexyldecanol, 2-undecylpentadecanol and oleyl alcohol;
- $C_{12}$-$C_{22}$ higher fatty acids, such as oleic acid, linoleic acid and linolenic acid, and mixtures thereof,
- fluoro oils that are optionally partially hydrocarbon-based and/or silicone-based,
- oils of plant origin, such as sesame oil (820.6 g/mol),
- fatty acids containing from 12 to 26 carbon atoms, for instance oleic acid,
- dialkyl carbonates, the 2 alkyl chains possibly being identical or different, such as dicaprylyl carbonate sold under the name Cetiol CC® by Cognis, and
- non-volatile oils of high molecular mass, for example between 650 and 10 000 g/mol, for instance:

  i) vinylpyrrolidone copolymers such as the vinylpyrrolidone/1-hexadecene copolymer, Antaron V-216 sold or manufactured by the company ISP (MW = 7300 g/mol),
  ii) esters such as:

    a)linear fatty acid esters with a total carbon number ranging from 35 to 70, for instance pentaerythrityl

tetrapelargonate (MW = 697.05 g/mol),
b)hydroxylated esters such as polyglycerol-2 triisostearate (MW = 96.5.58 g/mol),
c)aromatic esters such as tridecyl trimellitate (MW = 757.19 g/mol),
d)esters of $C_{24}$-$C_{28}$ fatty alcohols or branched fatty acids such as those described in patent application EP-A-0 955 039, and especially triisoarachidyl citrate (MW = 1033.76 g/mol), pentaerythrityl tetraisononanoate (MW = 697.05 g/mol), glyceryl triisostearate (MW = 891.51 g/mol), glyceryl tris(2-decyl) tetradecanoate (MW = 1143.98 g/mol), pentaerythrityl tetraisostearate (MW = 1202.02 g/mol), polyglyceryl-2 tetraisostearate (MW = 1232.04 g/mol) or pentaerythrityl tetrakis(2-decyl) tetradecanoate (MW = 1538.66 g/mol),
e)esters and polyesters of a diol dimer and of a monocarboxylic or dicarboxylic acid, such as esters of a diol dimer and of a fatty acid and esters of a diol dimer and of a dicarboxylic acid dimer,

- and mixtures thereof.

The esters of a diol dimer and of a monocarboxylic acid may be obtained from a monocarboxylic acid containing from 4 to 34 carbon atoms and especially from 10 to 32 carbon atoms, which acids are linear or branched, and saturated or unsaturated.

As illustrative examples of monocarboxylic acids that are suitable for use in the invention, mention may be made especially of fatty acids.

The esters of diol dimer and of dicarboxylic acid may be obtained from a dicarboxylic acid dimer derived in particular from the dimerization of an unsaturated fatty acid especially of $C_8$ to $C_{34}$, especially $C_{12}$ to $C_{22}$, in particular $C_{16}$ to $C_{20}$ and more particularly $C_{18}$. According to one particular variant, it is more particularly the dicarboxylic acid dimer from which the diol dimer to be esterified is also derived.

The esters of a diol dimer and of a carboxylic acid may be obtained from a diol dimer produced by catalytic hydrogenation of a dicarboxylic acid dimer as described previously, for example hydrogenated dilinoleic diacid. As illustrations of diol dimer esters, mention may be made especially of esters of dilinoleic diacids and of dilinoleyl diol dimmers sold by the company Nippon Fine Chemical under the trade names Lusplan DD-DA5® and DD-DA7®.

According to a second embodiment, the additional polar oil may be a volatile oil. In particular, the volatile polar oil may be chosen from the list of oils below, and mixtures thereof:

- ketones that are liquid at room temperature, such as methyl ethyl ketone or acetone;
- short-chain esters, especially containing from 3 to 8 carbon atoms in total, such as ethyl acetate, methyl acetate, propyl acetate or n-butyl acetate;

- ethers that are liquid at room temperature, such as diethyl ether, dimethyl ether or dichlorodiethyl ether;
- alcohols and especially linear or branched lower monoalcohols containing from 2 to 5 carbon atoms, such as ethanol, isopropanol or n-propanol.

It is also possible to use volatile fluoro oils, such as nonafluoromethoxybutane or perfluoromethylcyclopentane, and mixtures thereof.

According to one embodiment, the additional polar oils that are suitable for use in the invention may be chosen from octyldodecyl/PPG-3 myristyl ether dimerdilinoleate, fatty acid triglycerides, plant oils, octyldodecanol, diisostearyl malate, phenyl silicones and vinylpyrrolidone/1-hexadecene, and mixtures thereof.

In a composition according to the invention, the said additional polar oil may be present in a content ranging from 0.1% to 40% by weight, especially from 1% to 30% by weight and more particularly from, 2% to 20% by weight relative to the total weight of the composition.

According to one embodiment, the composition according to the invention is free of polar oil or comprises less than 10% or even less than 0.5% of polar oil.

**DYESTUFFS**

The composition according to the invention preferably comprises at least one dyestuff, preferably in a content of at least 0.1% by weight relative to the total weight of the composition. The dyestuff may be chosen from pulverulent dyestuffs (especially pigments and nacres), and water-soluble or liposoluble dyestuffs.

For the purposes of the invention, the term "pigments" means white or coloured, mineral or organic particles, which are insoluble in an aqueous solution, and which are intended to colour and/or opacify the resulting makeup film. The pigments also include nacres or nacreous pigments.

The pigments may be present in a proportion of from 0.1% to 15% by weight, especially from 1% to 10% by weight and in particular from 2% to 8% by weight relative to the total weight of the cosmetic composition.

As mineral pigments that may be used in the invention, mention may be made of titanium oxide, zirconium oxide or cerium oxide, and also zinc oxide, iron oxide or chromium oxide, ferric blue, manganese violet, ultramarine blue and chromium hydrate.

According to one embodiment, titanium oxides and iron oxides are more particularly considered in the invention.

According to one embodiment, a pigment that is suitable for use in the invention may in particular be based on titanium dioxide and iron oxide.

It may also be a pigment with a structure that may be, for example, of sericite/brown iron oxide/titanium dioxide/silica type. Such a pigment is sold, for example, under the reference Coverleaf NS or JS by the company Chemicals and Catalysts, and has a contrast ratio in the region of 30.

A pigment that is suitable for use in the invention may comprise a structure that may be, for example, of silica microsphere type containing iron oxide. An example of a pigment having this structure is the product sold by the company Miyoshi under the reference PC Ball PC-LL-100 P, this pigment consisting of silica microspheres containing yellow iron oxide.

Among the organic pigments that may be used in the invention, mention may be made of carbon black, pigments of D&C type, lakes based on cochineal carmine or on barium, strontium, calcium or aluminium, or alternatively the diketopyrrolopyrroles (DPP) described in documents EP-A-542 669, EP-A-787 730, EP-A-787 731 and WO-A-96/08537.

The terms "nacres" and "nacreous pigments" should be understood as meaning iridescent or non-iridescent coloured particles of any form, especially produced by certain molluscs in their shell, or else synthesized, and which have a colour effect by optical interference.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles at the surface of which are superposed at least two successive layers of metal oxides and/or of organic dyestuffs.

Examples of nacres that may also be mentioned include natural mica coated with titanium oxide, with iron oxide, with natural pigment or with bismuth oxychloride.

Among the nacres available on the market, mention may be made of the mica-based nacres Timica, Flamenco and Duochrome sold by the company Engelhard, the Timiron nacres sold by the company Merck, the Prestige mica-based nacres, sold by the company Eckart, and the Sunshine synthetic mica-based nacres, sold by the company Sun Chemical.

The nacres may more particularly have a yellow, pink, red, bronze, orangery, brown, gold and/or coppery colour or tint.

As illustrations of nacres that may be used in the context of the present invention, mention may be made especially of the gold-coloured nacres sold especially by the company Engelhard under the name Brilliant gold 212G (Timica), Gold 222C (Cloisonne), Sparkle gold (Timica), Gold 4504 (Chromalite) and Monarch gold 233X (Cloisonne); the bronze nacres sold especially by the company Merck under the name Bronze fine (17384) (Colorona) and Bronze (17353) (Colorona) and by the company Engelhard under the name Super bronze (Cloisonne); the orange nacres sold especially by the company Engelhard under the name Orange 363C (Cloisonne) and Orange MCR 101 (Cosmica) and by the company Merck under the name Passion orange (Colorona) and Matte orange (17449) (Microna); the brown nacres sold especially by the company Engelhard under the name Nu-antique copper 340XB (Cloisonne) and Brown CL4509 (Chromalite); the nacres with a copper tint sold especially by the company Engelhard under the name Copper 340A (Timica); the nacres with a red tint sold especially by the company Merck under the name Sienna fine (17386) (Colorona); the nacres with a yellow tint sold especially by the company Engelhard under the name Yellow (4502) (Chromalite); the red nacres with a gold tint sold especially by the company Engelhard under the name Sunstone G012 (Gemtone); the pink nacres sold especially by the company Engelhard under the name Tan opale G005 (Gemtone); the black nacres with a gold tint sold especially by the company Engelhard under the name Nu antique bronze 240 AB (Timica), the blue nacres sold especially by the company Merck under the name Matte blue (17433) (Microna), the white nacres with a silvery tint sold especially by the company Merck under the name Xirona Silver, and the golden-green pink-orange nacres sold especially by the company Merck under the name Indian summer (Xirona), and mixtures thereof.

According to one embodiment variant, a composition of the invention may comprise as pigments a pigment chosen from titanium dioxide, pigments based on titanium dioxide and iron oxide, or pigments based on titanium dioxide, for instance sericite/brown iron oxide/titanium dioxide/silica, or natural mica coated with titanium oxide, and mixtures thereof.

A composition according to the invention may also comprise at least one dyestuff different from the pigments as defined above.

Such a dyestuff may be chosen from organic or inorganic, liposoluble or water-soluble dyestuffs, and materials with a specific optical effect, and mixtures thereof.

A cosmetic composition according to the invention may thus also comprise water-soluble or liposoluble dyes. The liposoluble dyes are, for example, Sudan red, DC Red 17, DC Green 6, β-carotene, Sudan brown, DC Yellow 11,

DC Violet 2, DC Orange 5 and quinoline yellow. The water-soluble dyes are, for example, beetroot juice or methylene blue.

A cosmetic composition according to the invention may also contain at least one material with a specific optical effect.

This effect is different than a sample conventional hue effect, i.e. a unified and stabilized effect as produced by standard dyestuffs, for instance monochromatic pigments. For the purposes of the invention, the term "stabilized" means lacking an effect of variability of the colour as a function of the angle of observation or alternatively in response to a temperature change.

For example, this material may be chosen from particles with a metallic tint, goniochromatic colouring agents, diffracting pigments, thermochromic agents, optical brighteners, and also fibres, especially interference fibres.

The particles with a metallic tint that may be used in the invention are chosen in particular from:

- particles of at least one metal and/or of at least one metal derivative,
- particles comprising a mono-material or multi-material organic or mineral substrate, at least partially coated with at least one coat with a metallic tint comprising at least one metal and/or at least one metal derivative, and
- mixtures of the said particles.

Among the metals that may be present in the said particles, mention may be made, for example, of Ag, Au, Cu, Al, Ni, Sn, Mg, Cr, Mo, Ti, Zr, Pt, Via, Rb, W, Zn, Ge, Te and Se, and mixtures or alloys thereof. Ag, Au, Cu, Al, Zn, Ni, Mo and Cr and mixtures or alloys thereof (for example bronzes and brasses) are preferred metals.

The term "metal derivatives" is intended to denote compounds derived from metals, especially oxides, fluorides, chlorides and sulfides.

As illustrations of these particles, Mention may be made of aluminium particles, such as those sold under the names Starbrite 1200 EAC® by the company Siberline, and Metalure® by the company Eckart.

Mention may also be made of copper metal powders or alloy mixtures such as the reference 2844 sold by the company Radium Bronze, metallic pigments such as aluminium or bronze, such as those sold under the name Rotosafe 700 from the company Eckart, the silica-coated aluminium particles sold under the name Visibnaire Bright Silver from the company Eckart and metal alloy particles, for instance the silica-coated bronze (alloy of copper and zinc) powders sold under the name Visionaire Bright Natural Gold from the company Eckart.

They may also be particles comprising a glass substrate, such as those sold by the company Nippon Sheet Glass under the name Microglass Metashine.

The goniochromatic colouring agent may be chosen, for example, from multilayer interference structures and liquid-crystal colouring agents.

Examples, of symmetrical multilayer interference structures that may be used in the compositions prepared in accordance with the invention are, for example, the following structures: $Al/SiO_2/Al/SiO_2/Al$, pigments having this structure being sold by the company Dupont de Nemours; $Cr/MgF_2/Al/MgF_2/Cr$, pigments having this structure being sold under the name Chromaflair by the company Flex; $MoS_2/SiO_2/Al/SiO_2/MoS_2$; $Fe_2O_3/SiO_2/Al/SiO_2/Fe_2O_3$; and $Fe_2O_3/SiO_2/Fe_2O_3/SiO_2/Fe_2O_3$, pigments having these structures being sold under the name Sicopearl by the company BASF; $MoS_2/SiO_2$/micaoxide/$SiO_2/MoS_2$; $Fe_2O_3/SiO_2$/mica-oxide/$SiO_2/Fe_2O_3$; $TiO_2/SiO_2/TiO_2$ and $TiO_2/Al_2O_3/TiO_2$: $SnO/TiO_2/SiO_2/TiO_2/SnO$; $Fe_2O_3/SiO_2/Fe_2O_3$; SnO/mica/$TiO_2SiO_2/TiO_2$/mica/SnO, pigments having these structures being sold under the name Xirona by the company Merck (Darmstadt). By way of example, these pigments may be the pigments of silica/titanium oxide/tin oxide structure sold under the name Xirona Magic by the company Merck, the pigments of silica/brown iron oxide structure sold under the name Xirona Indian Summer by the company Merck and the pigments of silica/titanium oxide/mica/tin oxide structure sold under the name Xirona Caribbean Blue by the company Merck. Mention may also be made of the Infinite Colors pigments from the company Shiseido. Depending on the thickness and the nature of the various layers, different effects are obtained. Thus, with the $Fe_2O_3/SiO_2/Al/SiO_2/Fe_2O_3$ structure, the colour changes from green-golden to redgray for $SiO_2$ layers of 320 to 350 rim; from red to golden for $SiO_2$ layers of 380 to 400 nm; from violet to green for $SiO_2$ layers of 410 to 420 nm; from copper to red for $SiO_2$ layers of 430 to 440 nm.

Examples of pigments with a polymeric multilayer structure that may be mentioned include those sold by the company 3M under the name Color Glitter.

Examples of liquid-crystal goniochromatic particles that may be used include those sold by the company Chenix and also the product sold under the name Helicone® HC by the company Wacker.

The dyestuffs, in particular the pigments treated with a hydrophobic agent, may be present in the composition in a content ranging from 0.1% % to 50% by weight, preferably ranging from 0.5% to 30% by weight and preferentially ranging from 1% to 20% by weight relative to the total weight of the composition.

**Solid fatty substances**

Besides the oils described previously, the fatty phase may also comprise at least one fatty substance that is

not liquid at room temperature (25°C) and at atmospheric pressure, known as a solid fatty substance, chosen from waxes and pasty fatty substances.

Preferably, the composition according to the invention comprises at least one solid fatty substance chosen from waxes and pasty fatty substances, and a mixture thereof.

## Pasty fatty substances

For the purposes of the present invention, the term "pasty fatty substance" (also known as a paste) means a lipophilic fatty compound with a reversible solid/liquid change of state, displaying anisotropic crystal organization in the solid state, and comprising a liquid fraction and a solid fraction at a temperature of 23°C.

In other words, the starting melting point of the pasty compound can be less than 23°C. The liquid fraction of the pasty compound measured at 23°C can represent 9% to 97% by weight of the compound. This liquid fraction at 23°C preferably represents between 15% and 85% and more preferably between 40% and 85% by weight.

For the purposes of the invention, the melting point corresponds to the temperature of the most endothermic peak observed on thermal analysis (DSC) as described in standard ISO 11357-3; 1999. The melting point of a paste or wax can be measured by means of a differential scanning calorimeter (DSC), for example the calorimeter sold under the name MDSC 2920 by the company TA Instruments.

The measuring protocol is as follows:

> A sample of 5 mg of paste or wax (depending on the case) placed in a crucible is subjected to a first temperature rise passing from -20°C to 100°C, at the heating rate of 10°C/minute, then is cooled from 100°C to -20 °C. at a cooling rate of 10°C/minute and finally subjected to a second temperature rise passing from -20°C to 1.0.0°C at a heating rate of 5°C/minute. During the second temperature rise, the variation in the difference between the power absorbed by the empty crucible and the crucible containing the sample of wax as a function of the temperature is measured. The melting point of the compound is the temperature value corresponding to the top of the peak of the curve representing the variation in the difference in power absorbed as a function of the temperature.

The liquid fraction by weight of the pasty compound at 23°C is equal to the ratio of the enthalpy of fusion consumed at 23°C to the enthalpy of fusion of the pasty compound.

The enthalpy of fusion of the pasty compound is the enthalpy consumed by the compound in order to pass from the solid state to the liquid state. The pasty compound is said to be in the solid state when all of its mass is in crystalline solid form. The pasty compound is said to be in the liquid state when all of its mass is in liquid form.

The enthalpy of fusion of the pasty compound is equal to the area under the curve of the thermogram obtained by means of a differential scanning calorimeter (DSC) such as the calorimeter sold under the name MDSC 2920 by the company TA Instruments, with a temperature rise of 5 or 10°C per minute, in accordance with standard ISO 11357-3; 1999. The enthalpy of fusion of the pasty compound is the amount of energy required to make the compound pass from the solid state to the liquid state. It is expressed in J/g.

The enthalpy of fusion consumed at 23°C is the amount of energy absorbed by the sample in order to pass from the solid state to the state it displays at 23°C formed from a liquid fraction and a solid fraction.

The liquid fraction of the pasty compound measured at 32°C preferably represents from 30% to 100% by weight of the compound, preferably from 50% to 100%, more preferably from 60% to 100% by weight of the compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the enthalpy of fusion consumed at 32°C to the enthalpy of fusion of the pasty compound. The enthalpy of fusion consumed at 32°C is calculated in the same way as the enthalpy of fusion consumed at 23°C.

The pasty compound is preferably chosen from synthetic compounds and compounds of plant origin. A pasty compound may be obtained by synthesis starting from starting materials of plant origin.

The pasty compound is advantageously chosen from:

- lanolin and derivatives thereof,
- polyol ethers chosen from pentaerythrityl ethers of polyalkylene glycols, fatty alkyl ethers of sugars, and mixtures thereof, pentaerythrityl ether of polyethylene glycol comprising 5 oxyethylene units (5 OE) (CTFA name: PEG-5 pentaerythrityl ether), pentaexythrityl ether of polypropylene glycol comprising 5 oxypropylene units (5 OP) (CTFA name: PPG-5 pentaerythrityl ether), and mixtures thereof, and more especially the mixture PEG-5 pentaerythxityl ether, PPG-5 pentaerythrityl ether and soybean oil, sold under the name Lanolide by the company Vevy, in which mixture the constituents are in a 46/46/8 weight ratio: 46% of PEG-5 pentaerythrityl ether, 46% of PPG-5 pentaerythrityl ether and 8% of soybean oil,

- polymeric or non-polymeric silicone compounds,
- polymeric or non-polymeric fluoro compounds,
- vinyl polymers, especially:
  - olefin homopolymers and copolymers,
  - hydrogenated diene homopolymers and copolymers,
  - linear or branched homopolymer or copolymer oligomers of alkyl (meth)acrylates preferably containing a $C_8$-$C_{30}$ alkyl group,
  - homopolymer and copolymer oligomers of vinyl esters containing $C_8$-$C_{30}$ alkyl groups,
  - vinylpyrrolidone/eicosene copolymers (INCI name VP/eicosene copolymer), for example the product sold by the company ISP under the trade name Ganex V220F®,
  - homopolymer and copolymer oligomers of vinyl ethers containing $C_8$-$C_{30}$ alkyl groups,
- liposoluble polyethers resulting from polyetherification between one or more $C_2$-$C_{100}$ and preferably $C_2$-$C_{50}$ diols,
- esters,
- and/or mixtures thereof.

The pasty compound is preferably a polymer, especially a hydrocarbon-based polymer.

Among the liposoluble polyethers, copolymers of ethylene oxide and/or of propylene oxide with C6-C30 long-chain alkylene oxides, more preferably such that the weight ratio of the ethylene oxide and/or propylene oxide to the alkylene oxides in the copolymer is from 5/95 to 70/30, are particularly preferred. In this family, mention will be made especially of copolymers such that the long-chain alkylene oxides are arranged in blocks with an average molecular weight from 1000 to 10 000, for example a polyoxyethylene/polydodecyl glycol block copolymer, such as the ethers of dodecanediol (22 mol) and of polyethylene glycol (45 OE) sold under the brand name Elfacos ST9 by Akzo Nobel.

Among the esters, the following are especially preferred:

- esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, especially the product sold under the brand name Softisan 649 by the company Sasol,
- arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched $C_4$-$C_{50}$ dicarboxylic acid or polycarboxylic acid and a $C_2$-$C_{50}$ diol or polyol,
- aliphatic esters of an ester resulting from the esterification of an aliphatic hydroxycarboxylic acid ester with an aliphatic carboxylic acid. Preferably, the aliphatic carboxylic acid comprises from 4 to 30 and preferably from 8 to 30 carbon atoms. It is preferably chosen from hexanoic acid, heptanoic acid, octanoic acid, 2-ethylhexanoic acid, nonanoic acid, decanoic acid, undecanoic acid, dodecanoic acid, tridecanoic acid, tetradecanoic acid, pentadecanoic acid, hexadecanoic acid, hexyldecanoic acid, heptadecanoic acid, octadecanoic acid, isostearic acid, nonadecanoic acid, eicosanoic acid, isoarachidic acid, octyldodecanoic acid, heneicosanoic acid and docosanoic acid, and mixtures thereof. The aliphatic carboxylic acid is preferably branched. The aliphatic hydroxycarboxylic acid ester is advantageously derived from a hydroxylated aliphatic carboxylic acid containing from 2 to 40 carbon atoms, preferably from 10 to 34 carbon atoms and better still from 12 to 28 carbon atoms, and from 1 to 20 hydroxyl groups, preferably from 1 to 10 hydroxyl groups and better still from 1 to 6 hydroxyl groups. The aliphatic hydroxycarboxylic acid ester is chosen from:

  a) partial or total esters of saturated linear monohydroxylated aliphatic monocarboxylic acids;
  b) partial or total esters of unsaturated monohydroxylated aliphatic monocarboxylic acids;
  c) partial or total esters of saturated mono-hydroxylated aliphatic polycarboxylic acids;
  d) partial or total esters of saturated polyhydroxylated aliphatic polycarboxylic acids;
  e) partial or total esters of $C_2$ to $C_{16}$ aliphatic polyols that have reacted with a monohydroxylated or polyhydroxylated aliphatic monocarboxylic or polycarboxylic acid,

  and mixtures thereof;
- esters of a diol dimer and of a diacid dimer, where appropriate esterified on their free alcohol or acid function (s) with acid or alcohol radicals, especially dimer dilinoleate esters; such esters may be chosen especially from the

esters having the following INCI nomenclature: bis-behenyl/isostearyl/phytosteryl dimerdilinoleyl dimerdilinoleate (Plandool G), phytosteryl/isosteryl/stearyl/behenyl dimerdilinoleate (Plandool H or Plandool S), and mixtures thereof;
- hydrogenated rosinate esters, such as dilinoleyl dimers of hydrogenated rosinate (Lusplan DD-DHR or DD-DHR from Nippon Fine Chemical);
- and mixtures thereof.

Advantageously, the pasty compound(s) preferably represent 0.1% to 80%, better still 0.5% to 60%, better still 1% to 30% and even better still 1% to 20% by weight relative to the total weight of the composition.

**Waxes:**

According to one preferred embodiment, the composition according to the invention comprises at least one wax.

The wax under consideration in the context of the present invention is generally a lipophilic compound that is solid at room temperature (25°C), with a solid/liquid reversible change of state, having a melting point of greater than or equal to 30°C, which may be up to 200°C and in particular up to 120°C.

In particular, the waxes that are suitable for the invention may have a melting point of greater than or equal to 45°C and in particular greater than or equal to 55°C.

The waxes that may be used in the compositions according to the invention are chosen from waxes that are solid at room temperature, of animal, plant, mineral or synthetic origin, and mixtures thereof.

As illustrations of waxes that are suitable for use in the invention, mention may be made especially of hydrocarbon-based waxes, for instance beeswax, lanolin wax and Chinese insect waxes, rice bran wax, carnauba wax, candelilla wax, ouricury wax, alfalfa wax, berry wax, shellac wax, Japan wax and sumach wax; montan wax, orange wax and lemon wax, microcrystalline waxes, paraffins and ozokerite; polyethylene waxes, the waxes obtained by Fisher-Tropsch synthesis, and waxy copolymers, and also esters thereof.

Mention may also be made of the waxes obtained by catalytic hydrogenation of animal or plant oils with linear or branched $C_8$-$C_{32}$ fatty chains, Among these, mention may be made especially of isomerized jojoba oil such as the trans-isomerized partially hydrogenated jojoba oil manufactured or sold by the company Desert Whale under the trade reference Iso-Jojoba-50®, hydrogenated sunflower oil, hydrogenated castor oil, hydrogenated coconut oil, hydrogenated lanolin oil, and the bis (1,1,1-trimethylolpropane) tetrastearate sold under the name Hest 2T-4S® by the company Heterene.

Mention may also be made of silicone waxes ($C_{30-45}$ Alkyl Dimethicone) and fluoro waxes.

It is also possible to use the waxes obtained by hydrogenation of castor oil esterified with cetyl alcohol, sold under the names Phytowax Ricin 16L64® and 22L73® by the company Sophim. Such waxes are described in patent application FR-A-2 792 190.

It is possible to use a $C_{20}$-$C_{40}$ alkyl (hydroxystearyloxy)stearate (the alkyl group comprising from 20 to 40 carbon atoms) as wax, alone or as a mixture. Such a wax is sold especially under the names Kester Wax K 82 P®, Hydroxypolyester K 82 P® and Kester Wax K 80 P® by the company Kosher Keunen.

As microwaxes that may be used in the compositions according to the invention, mention may be made especially of carnauba microwaxes such as the product sold under the name MicroCare 350® by the company Micro Powders, synthetic microwaxes such as the product sold under the name MicroEase 114S® by the company Micro Powders, microwaxes formed from a mixture of carnauba wax and polyethylene wax, such as those sold under the names MicroCare 300® and 310® by the company Micro Powders, microwaxes formed from a mixture of carnauba wax and synthetic wax, such as the product sold under the name MicroCare 325® by the company Micro Powders, polyethylene microwaxes such as those sold under the names Micropoly 200®, 220®, 220L® and 250S® by the company Micro Powders, and polytetrafluoroethylene microwaxes such as those sold under the names Microslip 519® and 519 L® by the company Micro Powders.

The composition according to the invention may comprise a wax content ranging from 0.1% to 30% by weight, in particular from 0.5% to 20% and more particularly from 1% to 15% by weight relative to the total weight of the composition.

**Additional film-forming polymer**

Besides the copolymer described previously, the composition may comprise an additional polymer such as a film-forming polymer.

According to the present invention, the term "film-forming polymer" means a polymer that is capable of forming, by itself or in the presence of an auxiliary film-forming agent, a continuous deposit on a support, especially on keratin materials.

Among the film-forming polymers that may be used in the composition of the present invention, mention may

be made of synthetic polymers, of radical type or of polycondensate type, polymers of natural origin, and mixtures thereof. Film-forming polymers that may be mentioned in particular include acrylic polymers, polyurethanes, polyesters, polyamides, polyureas and cellulose-based polymers, for instance nitrocellulose.

The polymer may be combined with one or more auxiliary film-forming agents. Such a film-forming agent may be chosen from any compound known to those skilled in the art as being capable of fulfilling the desired function, and may be chosen especially from plasticizers and coalescers.

**Lipophilic gelling agents**

According to one embodiment, the composition according to the invention may comprise at least one gelling agent. The gelling agents that may be used in the compositions according to the invention may be organic or mineral, polymeric or molecular lipophilic gelling agents.

Mineral lipophilic gelling agents that may be mentioned include optionally modified clays such as hectorites modified with a $C_{10}$ to $C_{22}$ ammonium chloride, for instance hectorite modified with distearyldimethylammonium chloride, for instance the product sold under the name Bentone 38V® by the company Elementis.

Mention may also be made of optionally hydrophobic-surface-treated fumed silica with a particle size of less than 1 μm. Specifically, it is possible to chemically modify the surface of silica, via a chemical reaction that generates a reduction in the number of silanol groups present at the surface of the silica. It is especially possible to replace silanol groups with hydrophobic groups: a hydrophobic silica is then obtained. The hydrophobic groups may be:

- trimethylsiloxyl groups, which are obtained especially by treating fumed silica in the presence of hexamethyldisilazane. Silicas thus treated are known as Silica silylate according to the CTFA (8th edition, 2000). They are sold, for example, under the references Aerosil R812® by the company Degussa and Cab-O-Sil TS-530® by the company Cabot,
- dimethylsilyloxyl or polydimethylsiloxane groups, which are especially obtained by treating fumed silica in the presence of polydimethylsiloxane or dimethyldichlorosilane. Silicas thus treated are known as Silica dimethyl silylate according to the CTFA (8th edition, 2000). They are sold, for example, under the references Aerosil R972® and Aerosil R974® by the company Degussa, and Cab-O-Sil TS-610® and Cab-O-Sil TS-720® by the company Cabot.

Among the lipophilic gelling agents that may be used in the compositions according to the invention, mention may also be made of fatty acid esters of dextrin, such as dextrin palmitates, especially those sold under the names Rheopearl TL® or Rheopearl KL® by the company Chiba Flour.

It is also possible to use silicone polyamides of the polyorganosiloxane type such as those described in documents US-A-5 874 069, US-A-5 919 441, US-A-6 051 216 and US-A-5 981 680.

These silicone polymers may belong to the following two families:

- polyorganosiloxanes comprising at least two groups capable of establishing hydrogen interactions, these two groups being located in the polymer chain, and/or
- polyorganosiloxanes comprising at least two groups capable of establishing hydrogen interactions, these two groups being located on grafts or branches.

**Fillers:**

The composition according to the invention may comprise at least one filler.

For the purposes of the present invention, the term "filler" denotes solid particles of any form, which are in an insoluble form and dispersed in the medium of the composition, even at temperatures that may be up to the melting point of all the fatty substances of the composition.

Generally, the fillers used according to the invention are colourless or white, namely non-pigmentary, i.e. they are not used to give a particular colour or shade to the composition according to the invention, even though their use may inherently lead to such a result. These fillers serve especially to modify the rheology or texture of the composition.

In this respect, they are different from nacres, organic pigmentary materials, for instance carbon black, pigments of D&C type, and lakes based on cochineal carmine or on barium, strontium, calcium or aluminium, and inorganic pigmentary materials, for instance titanium dioxide, zirconium oxide or cerium oxide, and also iron oxides (black, yellow or red), chromium oxide, manganese violet, ultramarine blue, chromium hydrate and ferric blue, which are, themselves, used to give a shade and coloration to the compositions incorporating them.

For the purposes of the invention, such compounds are not covered by the definition of fillers, which thus covers non-pigmentary fillers, which may be organic or inorganic.

The non-pigmentary fillers used in the compositions according to the present invention may be of lamellar,

globular or spherical form, of fibre type, or of any intermediate form between these defined forms.

The size of the particles, i.e. their granulometry, is chosen so as to ensure the good dispersion of the fillers in the composition according to the invention. The granulometry of the particles may be distributed within the range from 5 μm to 10 nm and in particular from 10 μm to 10 nm.

The fillers according to the invention may or may not be surface-coated, in particular surface-treated with silicones, amino acids, fluoro derivatives or any other substance that promotes the dispersion and compatibility of the filler in the composition.

**Mineral fillers**

For the purposes of the present invention, the terms "mineral" and "inorganic" are used interchangeably.

Among the non-pigmentary mineral fillers that may be used in the compositions according to the invention, mention may be made of talc, mica, silica, perlite, which is especially commercially available from the company World Minerals Europe under the trade names Perlite P1430, Perlite P2550 or Perlite P204, kaolin, precipitated calcium carbonate, magnesium carbonate, magnesium hydrogen carbonate, hydroxyapatite, boron nitride, hollow silica microspheres (Silica Beads® from Maprecos), and glass or ceramic microcapsules, and mixtures thereof.

According to one embodiment, the cosmetic composition according to the invention comprises at least one non-pigmentary mineral filler chosen from the group comprising kaolin, talc, silica, perlite and clay, and mixtures thereof.

**Organic fillers**

Among the organic fillers that may be mentioned are polyamide powder (Orgasol® Nylon® from Atochem), poly-β-alanine powder and polyethylene powder, lauroyllysine, starch, tetrafluoroethylene polymer powders (Teflon®), hollow polymer microspheres such as those of polyvinylidene chloride/acrylonitrile, for instance Expancel® (Nobel Industrie) or of acrylic acid copolymer (such as Polytrap (Dow Corning)), acrylate copolymers, PMMA, 12-hydroxystearic acid oligomer stearate and silicone resin microbeads (for example Tospearls® from Toshiba), magnesium carbonate, magnesium hydrogen carbonate, and metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, and mixtures thereof.

For the purposes of the present invention, the organic fillers are different from the pigments.

They may also be particles comprising a copolymer, the said copolymer comprising trimethylol hexyl lactone. In particular, it may be a hexamethylene diisocyanate/trimethylol hexyl lactone copolymer. Such particles are especially commercially available, for example under the name Plastic Powder D-400® or Plastic Powder D-800® from the company Toshiki.

According to one embodiment, a composition of the invention may comprise at least one filler chosen from talc, silica, starch, clay, kaolin and perlite, and mixtures thereof.

One or more dispersants may be used, where appropriate, to protect the dispersed fillers or particles against aggregation or flocculation. They may be added independently of the solid fillers or particles or in the form of a colloidal dispersion of particles.

The concentration of dispersant is chosen so as to obtain satisfactory dispersion of the solid particles (without flocculation).

This dispersant may be a surfactant, an oligomer, a polymer or a mixture of several thereof, bearing one or more functionalities with strong affinity for the surface of the particles to be dispersed. In particular, poly(12-hydroxystearic acid) esters are used, such as poly(12-hydroxystearic acid) stearate with a molecular weight of about 750 g/mol, such as the product sold under the name Solsperse 21 000® by the company Avecia, esters of poly(12-hydroxystearic acid) with polyols such as glycerol or diglycerol, such as polyglyceryl-2 dipolyhydroxystearate (CTFA name) sold under the reference Dehymuls PGPH® by the company Henkel (or diglyceryl poly(12-hydroxystearate)), or alternatively poly(12-hydroxystearic acid), such as the product sold under the reference Arlacel P100 by the company Uniqema, and mixtures thereof.

As other dispersants that may be used in the composition of the invention, mention may be made of quaternary ammonium derivatives of polycandensated fatty acids, for instance Solsperse 17 000® sold by the company Avecia, and mixtures of polydimethylsiloxane/oxypropylene such as those sold by the company Dow Corning under the preferences DC2-5185 and DC2-5225 C.

**Additional common cosmetic ingredients**

The composition according to the invention may also comprise any common cosmetic ingredient, which may be chosen especially from film-forming polymers, antioxidants, fragrances, preserving agents, neutralizers, surfactants,

sunscreens, vitamins, moisturizers, self-tanning compounds, antiwrinkle active agents, emollients, hydrophilic or lipophilic active agents, free-radical scavengers, deodorants, sequestrants, film-forming agents and semicrystalline polymers, and mixtures thereof.

Needless to say, a person skilled in the art will take care to select the optional additional ingredients and/or the amount thereof such that the advantageous properties of the composition according to the invention are not, or are not substantially, adversely affected by the envisaged addition.

The compositions according to the invention may be in any common acceptable form for a cosmetic composition. They may thus be in the form of a suspension or a dispersion, especially of oil in water by means of vesicles; an optionally thickened or even gelled organic or oily solution; an oil-in-water, water-in-oil or multiple emulsion; a gel or a mousse; an oily or emulsified gel; a dispersion of vesicles, especially lipid vesicles; a two-phase or multiphase lotion; a spray; a lotion, a cream, a pomade, a soft paste, an ointment, a solid cast or moulded especially as a stick or in a dish, or a compacted solid.

A person skilled in the art may select the appropriate galenical form, and also the method for preparing it, on the basis of his general knowledge, taking into account firstly the nature of the constituents used, especially their solubility in the support, and secondly the intended use of the composition.

Preferably, the composition according to the invention comprises less than 3% and better still less than 1% by weight of water relative to the total weight of the composition. Even more preferably, the composition is totally anhydrous. The term "anhydrous" especially means that water is preferably not deliberately added to the composition, but may be present in trace amount in the various compounds used in the composition.

According to one preferred embodiment, the composition according to the invention is a lipstick.

According to one preferred embodiment, the composition according to the invention is in liquid form at 25°C, such as a lip gloss.

According to another embodiment, the composition is in solid form at 25°C. Preferably, according to this embodiment, the composition comprises at least one solid fatty substance, preferably chosen from waxes. In the case of a lipstick, it may be a stick of lipstick or a lipstick cast in a dish, for example.

The term "solid" refers to a composition whose hardness, measured according to the following protocol, is greater than or equal to 30 $Nm^{-1}$ at a temperature of 20°C and at atmospheric pressure (760 mmHg).

**Protocol for measuring the hardness:**

The hardness of the composition is measured according to the following protocol:

The stick of lipstick is stored at 20°C for 24 hours before measuring the hardness.

The hardness may be measured at 20°C via the "cheese wire" method, which consists in transversely cutting a wand of product, which is preferably a circular cylinder, by means of a rigid tungsten wire 250 $\mu$m in diameter, by moving the wire relative to the stick at a speed of 100 mm/minute.

The hardness of the samples of compositions of the invention, expressed in $Nm^{-1}$, is measured using a DFGHS2 tensile testing machine from the company Indelco-Chatillon.

The measurement is repeated three times and then averaged. The average of the three values read using the tensile testing machine mentioned above, noted Y, is given in grams. This average is converted into newtons and then divided by L which represents the longest distance through which the wire passes. In the case of a cylindrical wand, L is equal to the diameter (in metres).

The hardness is converted into $Nm^{-1}$ by the equation below:

$$(Y \times 10^{-3} \times 9.8)/L$$

For a measurement at a different temperature, the stick is stored for 24 hours at this new temperature before the measurement.

The compositions in accordance with the invention may be used for caring for or making up keratin materials such as the skin, the eyelashes, the eyebrows, the nails or the lips, and more particularly for making up the lips, the eyelashes and/or the face.

They may thus be in the form of a care and/or makeup product for bodily or facial skin, the lips, the eyelashes, the eyebrows or the nails; an antisun or self-tanning product; they may advantageously be in the form of a makeup composition, especially a mascara, an eyeliner, a lipstick, a lip gloss, a face powder, an eyeshadow, a foundation, a nail varnish or a nailcare product.

A subject of the invention is also a cosmetic process for treating keratin materials, especially bodily or facial skin, the lips, the nails and/or the eyelashes, comprising the application to the said materials of a cosmetic composition as defined previously.

This process according to the invention especially allows the said keratin materials, in particular the lips and/or the nails, to be cared for or made up, by applying a composition, especially a lipstick, a lip gloss, a nailcare product or a nail varnish according to the invention.

The invention is illustrated in greater detail in the following preparation examples.

**Examples 1 to 4: Liquid lipsticks**

The following liquid lipstick compositions were prepared:

| Compound | Composition 1 according to the invention (weight%) | Composition 2 according to the invention (weight %) | Composition 3 according to the invention (weight%) | Composition 4 according to the invention (weight %) |
|---|---|---|---|---|
| Jarcol 24 (55.6% in isododecane) (supramolecular compound 12 prepared previously) | 90 | 90 | 90 | 90 |
| Diphenyl dimethicone (400 cSt) (KF 54 from Shin-Etsu) | 9 | - | - | - |
| Trimethylsiloxyphenyl dimethicone (Wacker-Belsil PDM 1000 from Wacker) | - | 9 | - | - |
| Phenyl trimethicone (Dow Corning 556 Cosmetic Grade Fluid from Dow Corning) (viscosity 15 cSt) | - | - | 9 | |
| Phenyl trimethicone (KF 56A from Shin-Etsu) (viscosity 20 cSt) | - | - | - | 9 |
| Red 7 (Unipure Red LC 3079 OR from LCW (Sensient) | 1 | 1 | 1 | 1 |
| Total: | 100 | 100 | 100 | 100 |

**Preparation protocol:**

In a first stage, the pigments are ground in a three-roll mill in part of the oily phase.

The rest of the liposoluble ingredients are then mixed together at a temperature of about 50°C (above the melting point of the solid fatty substances (paste or wax) present in the composition) with stirring using a Rayneri blender.

The ground material is then added to the mixture and is stirred to homogenize thoroughly. The fluid is then allowed to cool to room temperature and is conditioned in a heating bag with an applicator.

After application to the lips, for compositions 1 to 4 according to the invention, a non-tacky deposit is formed for compositions 1 and 2 and a sparingly tacky deposit is formed for compositions 3 and 4, and for each of the compositions 1 to 4, the deposit is very glossy immediately after application and 1 hour after application.

**Examples 5 to 8: Liquid lipsticks**

The following liquid lipstick compositions were prepared:

| Compound | Composition 5 according to the invention (weight%) | Composition 6 according to the invention (weight%) | Composition 7 according to the invention (weight%) | Composition 8 according to the invention (weight%) |
|---|---|---|---|---|
| Supramolecular compound prepared from Jarcol 24 (55.6% in isododecane) (supramolecular compound 12 prepared previously) | 90 | 90 | 90 | 90 |
| Dimethicone (Dow Corning 200 Fluid 350 cSt from Dow Corning) | 9 | - | - | - |
| Dimethicone (Dow Corning 200 Fluid 5 cSt from Dow Corning) | - | 9 | - | - |
| Hydrogenated polyisobutene (Parleam from NOF Corporation) | - | - | 9 | |
| Polybutene (Indopol H 1500 from Ineos) | - | - | - | 9 |
| Red 7 (Unipure Red LC 3079 OR from LCW (Sensient) | 1 | 1 | 1 | 1 |
| Total: | 100 | 100 | 100 | 100 |

**Preparation protocol:**

In a first stage, the pigments are ground in a three-roll mill in part of the oily phase.

The rest of the liposoluble ingredients are then mixed together at a temperature of about 50°C (above the melting point of the solid fatty substances (paste or wax) present in the composition) with stirring using a Rayneri blender.

The ground material is then added to the mixture and is stirred to homogenize thoroughly. The fluid is then allowed to cool to room temperature and is conditioned in a heating bag with an applicator.

After application to the lips, compositions 5 and 6 according to the invention form a non-tacky deposit, and compositions 7 and 8 form a sparingly tacky deposit, and for each of the compositions 5 to 8, the deposit is very glossy immediately after application and 1 hour after application.

**Comparative Examples 9 to 12: Liquid lipsticks**

The following liquid lipstick compositions were prepared:

| Compound | Comparative composition 9 (weight%) | Comparative composition 10 (weight%) | Comparative composition 11 (weight%) | Comparative composition 12 (weight%) |
|---|---|---|---|---|
| Supramolecular compound prepared from Jarcol 24 (55.6% in isododecane) (supramolecular compound 12 prepared previously) | 90 | 90 | 90 | 90 |
| Isononyl Isononanoate | 9 | - | - | - |
| Diisostearyl malate | - | 9 | - | - |
| Octyldodecanol | - | - | 9 | |
| Caprylic/caprictriglyceride(Myritol 318 from Cognis) | - | - | - | 9 |
| Red 7 (Unipure Red LC 3079 OR from LCW (Sensient) | 1 | 1 | 1 | 1 |

(continued)

| Compound | Comparative composition 9 (weight%) | Comparative composition 10 (weight%) | Comparative composition 11 (weight%) | Comparative composition 12 (weight%) |
|---|---|---|---|---|
| Total: | 100 | 100 | 100 | 100 |

**Preparation protocol:**

In a first stage, the pigments are ground in a three-roll mill in part of the oily phase.

The rest of the liposoluble ingredients are then mixed together at a temperature of about 50°C (above the melting point of the solid fatty substances (paste or wax) present in the composition) with stirring using a Rayneri blender.

The ground material is then added to the mixture and is stirred to homogenize thoroughly. The fluid is then allowed to cool to room temperature and is conditioned in a heating bag with an applicator.

After application to the lips, the comparative compositions 9 to 13 according to the invention form a glossy but tacky or even very tacky deposit, immediately after application and 1 hour after application.

Examples 1 to 12 were repeated with the supramolecular compound 9 prepared previously, using the oil 2-hexyldecanol (Jarcol I-16), replacing weight for weight in compositions 1 to 12 compound 12 with compound 9. Results similar to those obtained with compound 12 were obtained for each of the oils formulated in combination with the supramolecular compound for each of the examples, that is to say that the compositions obtained with the polar oils are very tacky, unlike the compositions obtained using the silicone oils and the apolar hydrocarbon-based oils.

## Claims

1. Cosmetic composition for making up and/or caring for keratin materials, comprising, in a cosmetically acceptable medium,

    (i) a supramolecular compound that may be obtained by reaction between:

    - at least one oil bearing at least one nucleophilic reactive function chosen from OH and $NH_2$, and
    - at least one junction group capable of establishing hydrogen bonds with one or more partner junction groups, each pairing of a junction group involving at least three hydrogen bonds, the said junction group bearing at least one isocyanate or imidazole reactive function capable of reacting with the reactive function borne by the oil, the said junction group also comprising at least one unit of formula (I) or (II):

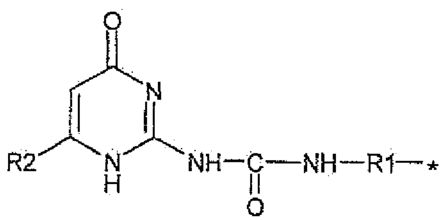

(I)

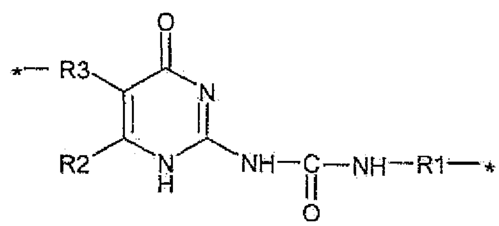

(II)

    in which:

    - R1 and R3, which may be identical or different, represent a divalent carbon-based radical chosen from (i) a linear or branched $C_1$-$C_{32}$ alkyl group, (ii) a $C_4$-$C_{16}$ cycloalkyl group and (iii) a $C_4$-$C_{16}$ aryl group; optionally comprising 1 to 8 heteroatoms chosen from O, N, S, F, Si and P; and/or optionally substituted with an ester or amide function or with a $C_1$-$C_{12}$ alkyl radical; or a mixture of these groups;
    - R2 represents a hydrogen atom or a linear, branched or cyclic, saturated or unsaturated, optionally aromatic, $C_1$-$C_{32}$ carbon-based and especially hydrocarbon-based radical, which may comprise one or more heteroatoms chosen from O, N, S, F, Si and P;

(ii) at least one oil, chosen from non volatile apolar hydrocarbon-based oils having a vapour pressure of less than 0.13 Pa (at ambient temperature (25°C)), and silicone oils,
(iii) at least one dyestuff.

**2.** Composition according to Claim 1, in which the oil bearing the reactive function is chosen, alone or as a mixture, from:

(i) linear, branched or cyclic, saturated or unsaturated fatty alcohols, comprising 6 to 50 carbon atoms, and comprising one or more OH; optionally comprising one or more $NH_2$;
(ii) esters and ethers bearing at least one free OH group, and especially partial esters and ethers of polyols, and hydroxylated carboxylic acid esters;
(iii) hydroxylated natural oils, modified natural oils and plant oils.

**3.** Composition according to either of the preceding claims, in which the oil bearing the reactive function is chosen, alone or as a mixture, from:

- saturated or unsaturated, linear or branched C6-C50, especially C6-C32 and in particular C8-C28 monoalcohols, and especially isostearyl alcohol, cetyl alcohol, oleyl alcohol, isopalmitoyl alcohol, 2-butyloctanol, 2-hexyldecanol, 2-octyldecanol, 2-octyldodecanol, 2-octyltetradecanol, 2-decyltetradecanol and 2-dodecylhexadecanol;
- saturated or unsaturated, linear or branched C6-C50, especially C6-G40 and in particular C8-C38 diols, and especially branched C32-C36 diols;
- saturated or unsaturated, linear or branched C6-C50, especially C6-C32 and in particular C8-C28 triols, and especially phytanetriol;
- pentaerythritol partial esters, and especially pentaerythritol adipate, pentaerythrityl caprate, pentaerythrityl succinate, pentaerythrityl tetraisononanoate, pentaerythrityl triisononanoate, pentaerythrityl tetraisostearate, pentaerythrityl triisostearate, pentaerythritol 2-(tetradecyl)-tetradecanoate, pentaerythrityl (tetraethyl)hexanoate and pentaerythrityl (tetraoctyl)dodecanoate;
- dipentaerythritol diesters, triesters, tetraesters or pentaesters, and especially dipentaerythrityl pentaisononanoate, dipentaerythrityl pentaisostearate, dipentaerythrityl tetraisostearate and dipentaerythrityl tris(polyhydroxystearate);
- trimethylolpropane monoesters and diesters, for instance trimethylolpropane monoisostearate, trimethylolpropane diisostearate, trimethylolpropane mono-2-ethylhexanoate and trimethylolpropane bis(2-ethylhexanoate);
- bis(trimethylolpropane) monoesters, diesters and triesters, for instance bis(trimethylolpropane) diisostearate, bis(trimethylolpropane) triisostearate and bis(trimethylolpropane) triethylhexanoate;
- partial monoesters or polyesters of glycerol or of polyglycerols, and especially:

  - glyceryl diisostearate and glyceryl diisononanoate;
  - polyglycerol-2 monoesters, diesters and triesters; for example with isostearic acid, 2-ethylhexanoic acid and/or isononanoic acid; and especially polyglyceryl-2 isostearate; polyglyceryl-2 diisostearate; polyglyceryl-2 triisostearate; polyglyceryl-2 nonaisostearate; polyglyceryl-2 nonanoate;
  - polyglycerol-3 monoesters, diesters, triesters or tetraesters; for example with either isostearic acid, 2-ethylhexanoic acid and/or isononanoic acid; and especially polyglyceryl-3 isostearate; polyglyceryl-3 diisostearate; polyglyceryl-3 triisostearate; polyglyceryl-3 nonaisostearate; polyglyceryl-3 nonanoate;
  - polyglycerol-10 partial esters and in particular polyglyceryl-10 nonaisostearate; polyglyceryl-10 nonanoate; polyglyceryl-10 isostearate; polyglyceryl-10 diisostearate; polyglyceryl-10 triisostearate;

- propylene glycol monoesters, for instance propylene glycol monoisostearate, propylene glycol neopentanoate or propylene glycol monooctanoate;
- diol dimer monoesters, for instance isostearyl dimer dilinoleate and octyldodecyl dimer dilinoleate;
- glycerol ethers, such as polyglyceryl-2 oleyl ether, polyglyceryl-3 cetyl ether, polyglyceryl-3 decyl tetradecyl ether and polyglyceryl-2 stearyl ether;
- esters between a hydroxylated monocarboxylic, dicarboxylic or tricarboxylic acid and monoalcohols, and in particular :

  - esters, especially monoesters, of 12-hydroxystearic acid; such as octyl hydroxystearate and 2-octyldodecyl hydroxystearate; mention may also be made of the corresponding oligomeric polyhydroxystearates, especially having a degree of polymerization of from 1 to 10, bearing at least one residual OH;
  - lactic acid esters, and especially C4-40 alkyl lactates, such as 2-ethylhexyl lactate, diisostearyl lactate,

isostearyl lactate, isononyl lactate or 2-octyldodecyl lactate;
- malic acid esters, and especially C4-40 alkyl malates, such as bis(2-ethylhexyl) malate, diisostearyl malate or bis(2-octyldodecyl) malate;
- citric acid esters, and especially C4-40 alkyl citrates, such as triisostearyl citrate, triisocetyl citrate and triisoarachidyl citrate;

- triglyceryl esters bearing one or more OHs;
- hydrogenated or non-hydrogenated castor oil, and also derivatives thereof derived especially from the transesterification of castor oil;
- modified epoxidized oils, the modification consisting in opening the epoxy function to obtain a diol, and especially hydroxylated modified soybean oil; hydroxylated soybean oils (directly hydroxylated or epoxidized beforehand).

**4.** Composition according to one of the preceding claims, in which the oil is a glossy oil, i.e. an oil with a refractive index of greater than or equal to 1.46 at 25°C.

**5.** Composition according to one of the preceding claims, in which the oil has a molar mass (Mw) of between 150 and 6000, especially between 170 and 4000, or even between 180 and 2000, preferentially between 200 and 1500 and better still between 220 and 800 g/mol.

**6.** Composition according to Claim 1, in which the oil bearing the reactive function is chosen from:

- linear, branched or cyclic, saturated or unsaturated fatty alcohols, comprising 6 to 50 carbon atoms, and comprising one or more OH; optionally comprising one or more $NH_2$, such as saturated or unsaturated, linear or branched C6-C50, especially C6-C32 and in particular C8-C28 monoalcohols, and especially isostearyl alcohol, cetyl alcohol, oleyl alcohol, isopalmitoyl alcohol, 2-butyloctanol, 2-hexyldecanol, 2-octyldecanol, 2-octyldodecanol, 2-octyltetradecanol, 2-decyltetradecanol and 2-dodecylhexadecanol,
- esters of a hydroxylated dicarboxylic acid with a manoalcohol, and in particular of malic acid, and especially C4-40 alkyl malates, such as 2-diethylhexyl malate, diisostearyl malate and 2-dioctyldodecyl malate.

**7.** Composition according to one of the preceding claims, in which, in the junction group, the radical R1 represents:

- a linear or branched, divalent C2-C12 alkylene group, especially a 1,2-ethylene, 1,6-hexylene, 1,4-butylene, 1,6-(2,4,4-trimethylhexylene), 1,4-(4-methylpentylene), 1,5-(5-methylhexylene), 1,6-(6-methylheptylene), 1,5-(2,2,5-trimethylhexylene) or 1,7-(3,7-dimethyloctylene) group;
- a divalent C4-C12 cycloalkylene or arylene group, chosen especially from the following radicals: -isophorone-, tolylene, 2-methyl-1,3-phenylene, 4-inethyl-1,3-phenylene; 4,4'-methylenebiscyclohexylene; 4,4-bisphenylen-emethylene; or of structure:

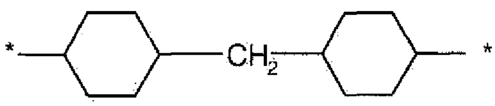

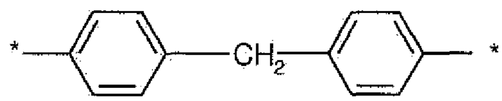

**8.** Composition according to one of the preceding claims, in which, in the junction group, the radical R2 represents H, or alternatively:

- a $C_1$-$C_{32}$, in particular $C_2$-$C_{16}$ or even $C_1$-$C_{10}$ alkyl group;
- a $C_4$-$C_{12}$ cycloalkyl group;
- a $C_4$-$C_{12}$ aryl group;
- a ($C_4$-$C_{12}$) aryl ($C_1$-$C_{18}$) alkyl group;
- a $C_1$-$C_4$ alkoxy group;
- an arylalkoxy group, in particular an aryl ($C_1$-$C_4$) alkoxy group;
- a $C_4$-$C_{12}$ heterocycle;

or a combination of these radicals, which may be optionally substituted with an amino, ester and/or hydroxyl function.

**9.** Composition according to one of the preceding claims, in which, in the junction group, the radical R3 represents a divalent radical -R'3-O-C (O)-NH-R'4- in which R'3 and R'4, which may be identical or different, represent a divalent

carbon-based radical chosen from a linear or branched $C_1$-$C_{32}$ alkyl group, a $C_4$-$C_{16}$ cycloalkyl group and a $C_4$-$C_{16}$ aryl group; or a mixture thereof.

**10.** Composition according to one of the preceding claims, in which, in the junction group,

(a) in formula (I), the following are present:

- $R_1$ = - isophorone- and R2 = methyl,
- $R_1$ = - $(CH_2)_6$- and R2 = methyl,
- $R_1$ = -$(CH_2)_6$- and R2 = isopropyl, or
- $R_1$ = 4,4'-methylenebiscyclohexylene and R2 = methyl,

or alternatively
(b) in formula (II), R1 represents the -isophorone- radical, R2 = methyl and R3 = $(CH_2)_2$OCO-NH-isophorone-.

**11.** Composition according to one of the preceding claims, in which the junction group has the formula:

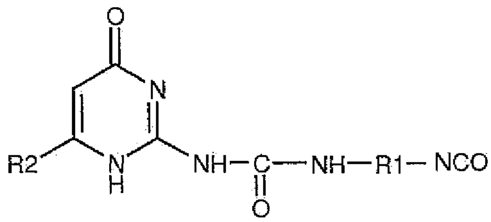

or the formula:

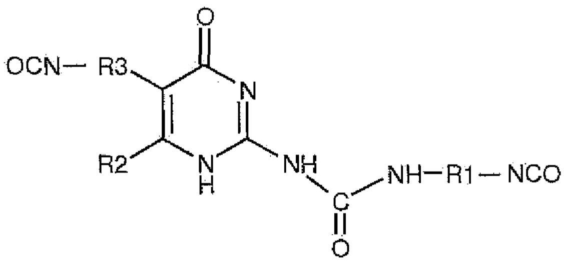

in which R1, R2 and R3 are as defined in one of the preceding claims.

**12.** Composition according to one of the preceding claims, in which the junction group is chosen from the following groups:

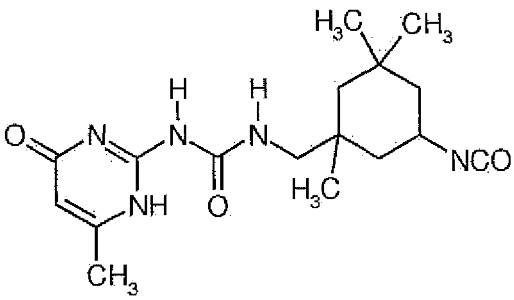

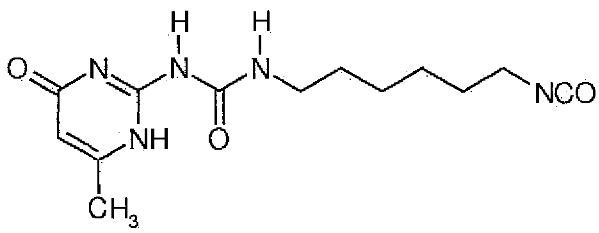

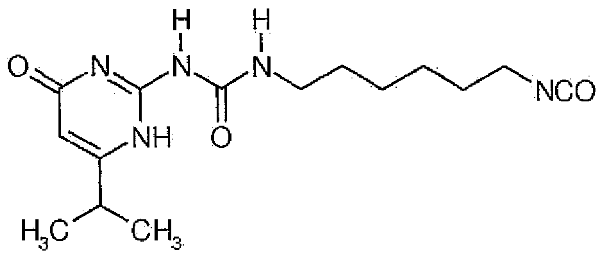

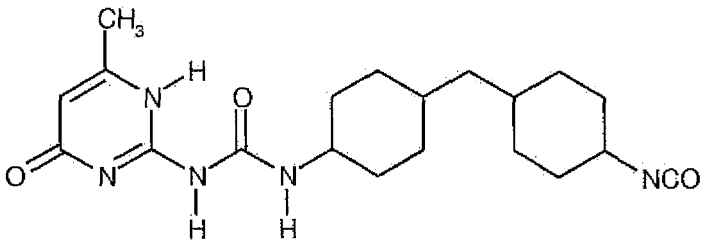

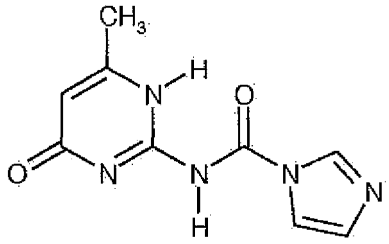

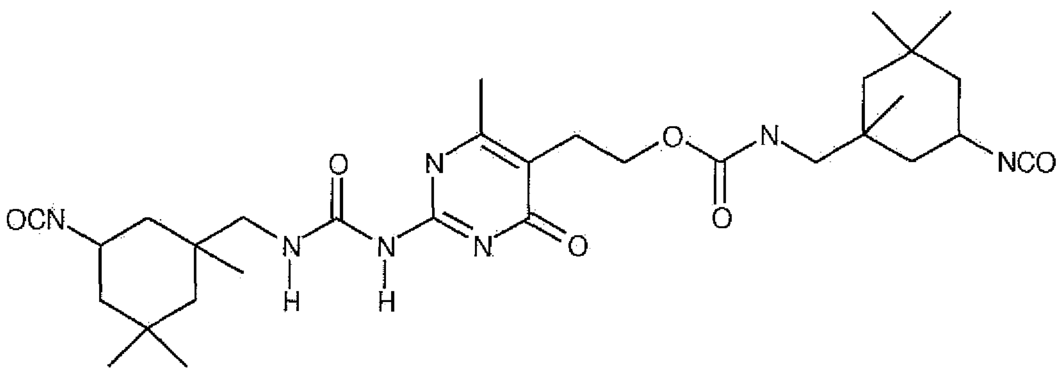

**13.** Composition according to one of the preceding claims, in which the supramolecular compounds are chosen from those corresponding to the following structures:

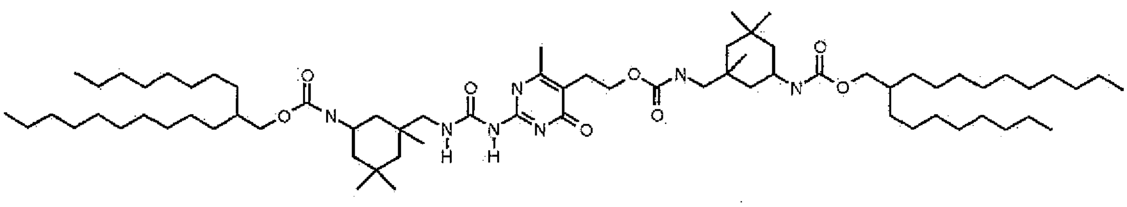

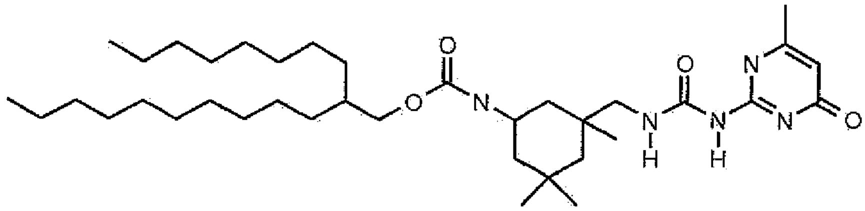

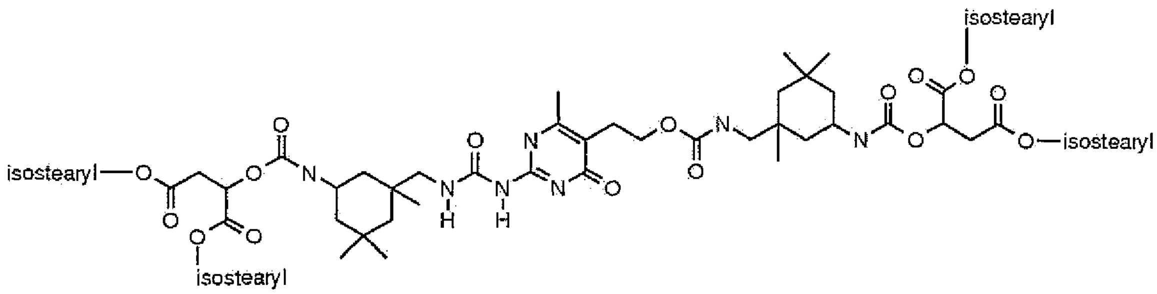

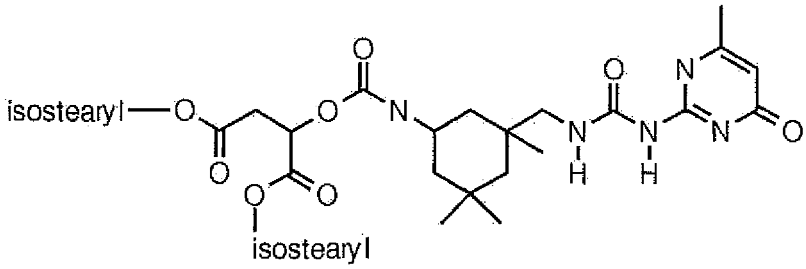

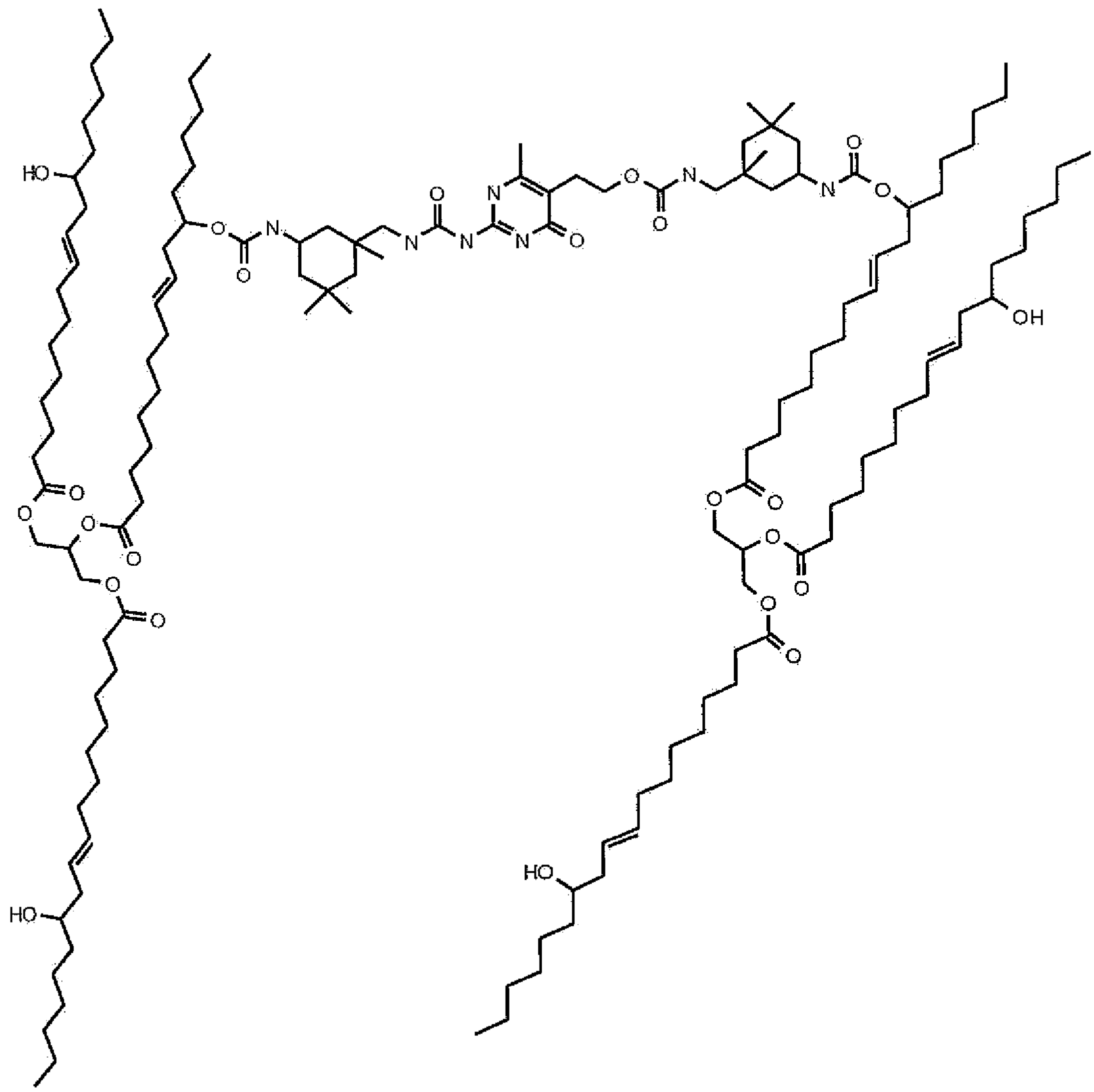

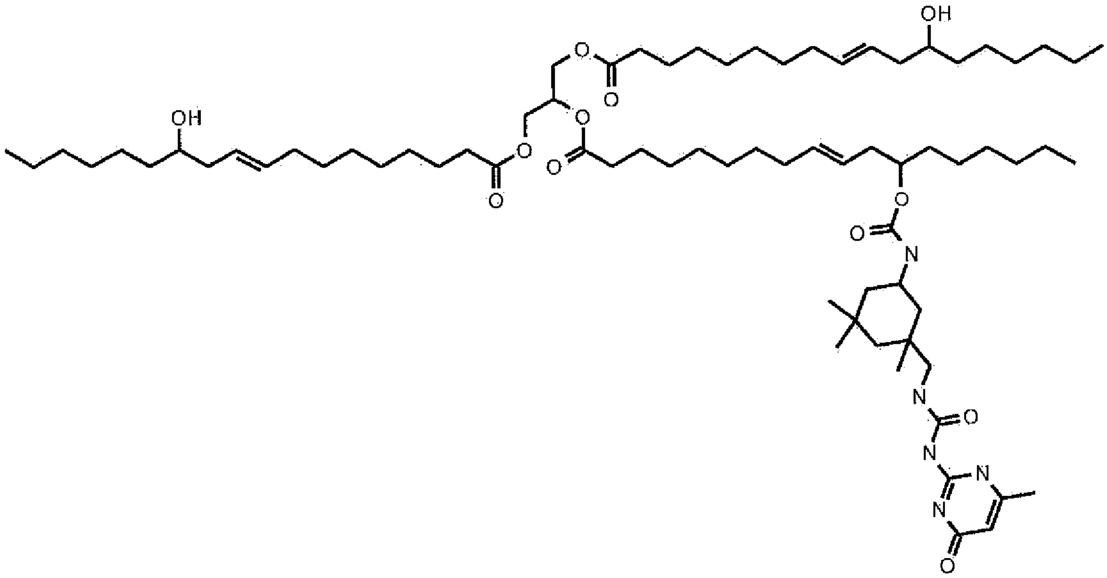

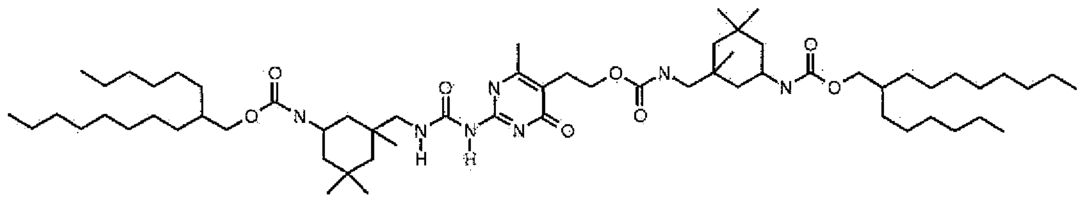

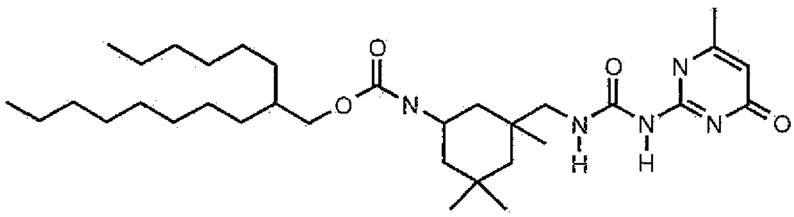

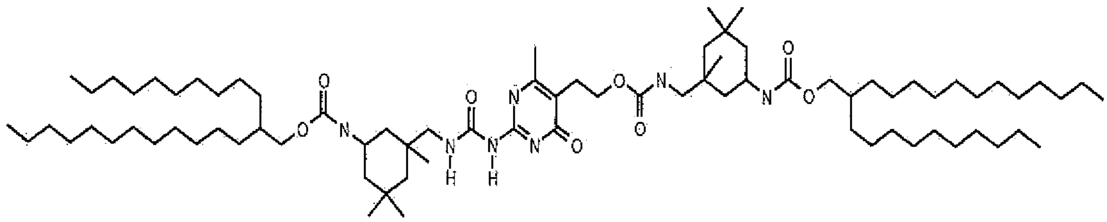

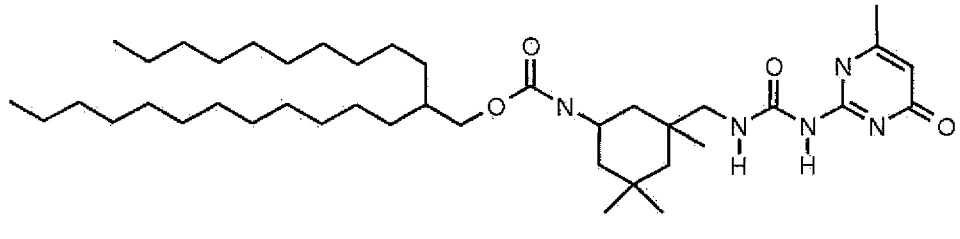

**14.** Composition according to one of the preceding claims, in which the number-average molecular mass (Mn) of the supramolecular compound is between 180 and 8000, preferably 200 to 6000, or even from 300 to 4000, better still from 400 to 3000 and preferentially from 500 to 1500.

**15.** Composition according to one of the preceding claims, in which the amount of supramolecular compound present in the composition is between 5% and 95% by weight, preferably between 10% and 95% by weight and better still preferably between 20% and 90% by weight relative to the total weight of the composition.

**16.** Composition according to one of the preceding claims, in which said oil chosen from apolar hydrocarbon-based oils and silicone oils is nonvolatile.

**17.** Composition according to any one of the preceding claims, **characterized in that** the said oil is a silicone oil chosen from:

i) phenyl silicone oils, in particular of formula (VII) or (VIII) below:

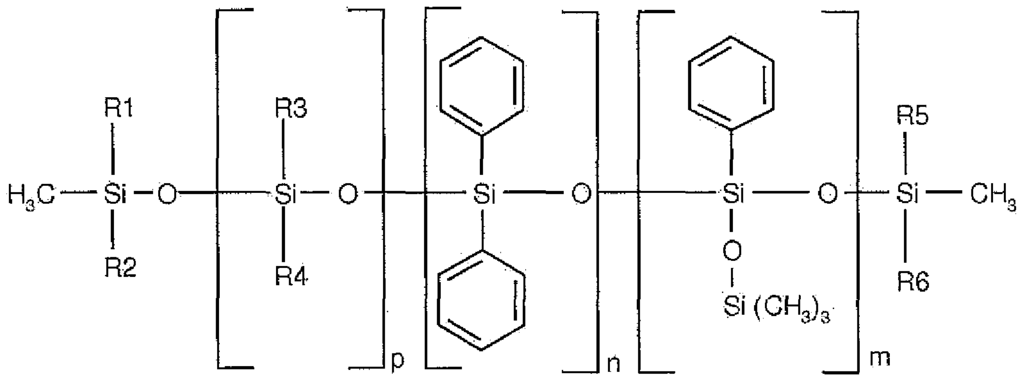

(VII)

in which:

- R1 to R6, independently of each other, are saturated or unsaturated, linear, cyclic or branched C1-C30 hydrocarbon-based radicals,
- m, n and p are, independently of each other, integers between 0 and 100, with the proviso that the sum n + m is between 1 and 100;

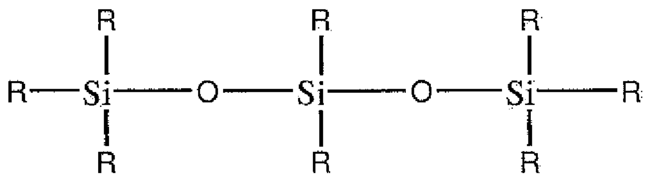

(VIII)

in which the groups R represent, independently of each other, a methyl or a phenyl;

ii) linear or cyclic polydimethylsiloxanes (PDMSs);
iii) polydimethylsiloxanes comprising alkyl or alkoxy groups, which are pendent and/or at the end of the silicone chain, these groups each containing from 2 to 24 carbon atoms.

**18.** Composition according to one of the preceding claims, **characterized in that** the said oil is an apolar hydrocarbon-based oil chosen from:

- polybutylenes,
- hydrogenated polyisobutylenes,
- polydecenes and hydrogenated polydecenes,
- hydrocarbon-based volatile oils containing from 7 to 16 carbon atoms, and mixtures thereof, and especially branched $C_8$-$C_{16}$ alkanes and linear alkanes containing from 7 to 15 carbon atoms, and mixtures thereof, and
- mixtures thereof.

**19.** Composition according to one of the preceding claims, **characterized in that** the said oil(s), chosen from apolar hydrocarbon-based oils and silicone oils, are present in a total content ranging from 0.5% to 70% by weight relative

to the total weight of the composition, preferably ranging from 3% to 50% by weight and better still ranging from 5% to 40% by weight relative to the total weight of the composition.

**20.** Composition according to one of the preceding claims, which is in the form of a composition for caring for and/or making up bodily or facial skin, the lips, the eyelashes, the eyebrows or the nails; an antisun or self-tanning product.

**21.** Cosmetic process for treating keratin materials, especially bodily or facial skin, the lips, the nails and/or the eyelashes, comprising the application to the said materials of a cosmetic composition as defined according to any one of Claims 1 to 20.

**Patentansprüche**

**1.** Kosmetische Zusammensetzung zum Zurechtmachen und/oder Pflegen von Keratinmaterialien, umfassend in einem kosmetisch annehmbaren Medium:

(i) eine supramolekulare Verbindung, die erhalten werden kann durch Reaktion zwischen:

- mindestens einem Öl, das mindestens eine nukleophile reaktive Funktion ausgewählt aus OH und $NH_2$ trägt, und
- mindestens einer Anschlussgruppe, die Wasserstoffbrückenbihdungen mit einer oder mehreren Partneranschlussgruppen herstellen kann, wobei jedes Paaren einer Anschlussgruppe mindestens drei Wasserstoffbrückenbindungen beinhaltet, wobei die Anschlussgruppe mindestens eine mit Isocyanat oder Imidazol reaktive Funktion trägt, die in der Lage ist, mit der reaktiven Funktion zu reagieren, die das Öl trägt, wobei die Anschlussgruppe auch mindestens eine Einheit der Formel (I) oder (II) umfasst:

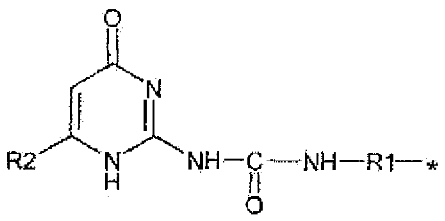

(I)

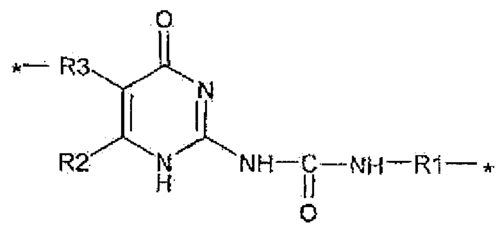

(II)

wobei:

- R1 und R3, die gleich oder unterschiedlich sein können, einen zweiwertigen, auf Kohlenstoff basierenden Rest repräsentieren, der ausgewählt ist aus (i) einer linearen oder verzweigten $C_1$-$C_{32}$-Alkylgruppe, (ii) einer $C_4$-$C_{16}$-Cycloalkylgruppe und (iii) einer $C_4$-$C_{16}$-Arylgruppe; die gegebenenfalls 1 bis 8 Heteroatome ausgewählt aus O, N, S, F, Si und P umfassen und/oder gegebenenfalls mit einer Ester- oder Amidfunktion oder mit einem $C_1$-$C_{12}$-Alkylrest substituiert sind, oder einer Mischung dieser Gruppen;
- R2 ein Wasserstoffatom oder einen linearen, verzweigten oder cyclischen, gesättigten oder ungesättigten, gegebenenfalls aromatischen, auf $C_1$-$C_{32}$-Kohlenstoff basierenden und insbesondere Kohlenwasserstoff basierenden Rest repräsentiert, der ein oder mehrere Heteroatome ausgewählt aus O, N, S, F, Si und P umfassen kann;

(ii) mindestens ein Öl ausgewählt aus nicht flüchtigen apolaren, auf Kohlenwasserstoff basierenden Ölen mit einem Dampfdruck von weniger als 0,13 Pa (bei Umgebungstemperatur (25°C)) und Silikonölen,
(iii) mindestens ein Färbemittel.

**2.** Zusammensetzung nach Anspruch 1, wobei das Öl, welches die reaktive Funktion trägt, allein oder als Mischung ausgewählt ist aus:

(i) linearen, verzweigten oder cyclischen, gesättigten oder ungesättigten Fettalkoholen, die 6 bis 50 Kohlenstoffatome umfassen und ein oder mehrere OH umfassen, gegebenenfalls ein oder mehrere $NH_2$ umfassen;
(ii) Estern und Ethern, die mindestens eine freie OH-Gruppe tragen, und insbesondere partiellen Estern und Ethern von Polyolen und hydroxylierten Carbonsäureestern;
(iii) hydroxylierten natürlichen Ölen, modifizierten natürlichen Ölen und Pflanzenölen.

**3.** Zusammensetzung nach einem der vorhergehenden Ansprüche, wobei das Öl, welches die reaktive Funktion trägt, allein oder als Mischung ausgewählt ist aus:

- gesättigten oder ungesättigten, linearen oder verzweigten $C_6$-$C_{50}$-, insbesondere $C_6$-$C_{32}$- und speziell $C_8$-$C_{28}$-Monoalkoholen und insbesondere Isostearylalkohol, Cetylalkohol, Oleylalkohol, Isopalmitoylalkohol, 2-Butyloctanol, 2-Hexyldecanol, 2-Octyldecanol, 2-Octyldodecanol, 2-Octyltetradecanol, 2-Decyltetradecanol und 2-Dodecylhexadecanol;
- gesättigten oder ungesättigten, linearen oder verzweigten $C_6$-$C_{50}$-, insbesondere $C_6$-$C_{40}$- und speziell $C_8$-$C_{38}$-Diolen und insbesondere verzweigten $C_{32}$-$C_{36}$-Diolen;
- gesättigten oder ungesättigten, linearen oder verzweigten $C_6$-$C_{50}$-, insbesondere $C_6$-$C_{32}$- und speziell $C_8$-$C_{28}$-Triolen und speziell Phytantriol;
- Pentaerythritolpartialestern und insbesondere Pentaerythrityladipat, Pentaerythritylcaprat, Pentaerythritylsuccinat, Pentaerythrityltetraisononanoat, Pentaerythrityltriisononanoat, Pentaerythrityltetraisostearat, Pentaerythrityltriisostearat, Pentaerythrityl-2-(tetradecyl)-tetradecanoat, Pentaerythrityl(tetraethyl)hexanoat und Pentaerythrityl(tetraoctyl)dodeca-noat;
- Dipentaerythritoldiestern, -triestern, -tetraestern oder -pentaestern, und insbesondere Dipentaerythritylpentaisononanoat, Dipentaerythritylpentaisostearat, Dipentaerythrityltetraisostearat und Dipentaerythrityltris (polyhydroxystearat);
- Trimethylolpropanmonoestern und -diestern, beispielsweise Trimethylolpropanmonoisostearat, Trimethylolpropandiisostearat, Trimethylolpropanmono-2-ethylhexanoat und Trimethylolpropanbis(2-ethylhexanoat);
- Bis(trimethylolpropan)monoestern, -diestern und -triestern, beispielsweise Bis(trimethylolpropan)diisostearat, Bis(trimethylolpropan)triisostearat und Bis-(trimethylolpropan)triethylhexanoat;
- partiellen Monoestern und Polyestern von Glycerol oder von Polyglycerolen und insbesondere:

  - Glyceryldiisostearat und Glyceryldiisononanoat;
  - Polyglycerol-2-monoestern, -diestern und -triestern; beispielsweise mit Isostearinsäure, 2-Ethylhexansäure und/oder Isononansäure; und insbesondere Polyglyceryl-2-isostearat; Polyglyceryl-2-diisostearat; Polyglyceryl-2-triisostearat; Polyglyceryl-2-nonaisostearat; Polyglyceryl-2-nonanoat;
  - Polyglycerol-3-monoestern, -diestern, -triestern oder -tetraestern; beispielsweise mit entweder Isostearinsäure, 2-Ethylhexansäure und/oder Isononansäure; und insbesondere Polyglyceryl-3-isostearat; Polyglyceryl-3-diisostearat; Polyglyceryl-3-triisostearat; Polyglyceryl-3-nonaisostearat; Polyglyceryl-3-nonanoat;
  - Polyglycerol-10-partialestern und insbesondere Polyglyceryl-10-nonaisosteaerat; Polyglyceryl-10-nonanoat; Polyglyceryl-10-isostearat; Polyglyceryl-10-diisostearat; Polyglyceryl-10-triisostearat;

- Propylenglykolmonoestern, beispielsweise Propylenglykolmonoisostearat, Propylenglykolneopentanoat oder Propylenglykolmonooctanoat;
- Dioldimermonoestern, beispielsweise Isostearyldimerdilinoleat und Octyldodecyldimerdilinoleat;
- Glycerolethern, wie Polyglyceryl-2-oleylether, Polyglyceryl-3-cetylether, Polyglyceryl-3-decyltetradecylether und Polyglyceryl-2-stearylether;
- Estern zwischen einer hydroxylierten Monocarbonsäure, Dicarbonsäure oder Tricarbonsäure und Monoalkoholen, und speziell:

  - Ester, insbesondere Monoester, von 12-Hydroxystearinsäure; wie Octylhydroxystearat und 2-Octyldodecylhydroxystearat; wobei auch die entsprechenden oligomeren Polyhydroxystearate erwähnt werden können, insbesondere mit einem Polymerisationsgrad von 1 bis 10, die mindestens ein restliches OH tragen;
  - Milchsäureester, und insbesondere $C_4$-$C_{40}$-Alkyl-lactate, wie 2-Ethylhexyllactat, Diisostearyllactat, Isostearyllactat, Isononyllactat oder 2-Octyldodecyllactat;
  - Äpfelsäureester, und insbesondere $C_4$-$C_{40}$-Alkylmalate, wie Bis(2-ethylhexyl)malat, Diisostearylmalat oder Bis(2-octyldodecyl)malat;
  - Citronensäureester, und insbesondere $C_4$-$C_{40}$-Alkylcitrate, wie Triisostearylcitrat, Triisocetylcitrat und Triisoarachidylcitrat;

- Triglycerylestern, die ein oder mehrere OH tragen;
- hydriertem oder nicht-hydriertem Castoröl und auch Derivaten davon, die insbesondere aus der Umesterung von Castoröl abgeleitet sind;
- modifizierten epoxydierten Öle, wobei die Modifizierung in der Öffnung der Epoxyfunktion besteht, um ein Diol zu erhalten, und insbesondere hydroxyliertes modifiziertes Sojaöl; hydroxylierte Sojaöle (direkt hydroxyliert oder zuvor epoxydiert).

**4.** Zusammensetzung nach einem der vorhergehenden Ansprüche, wobei das Öl ein glänzendes Öl ist, d. h. ein Öl mit einem Brechungsindex größer als oder gleich 1,46 bei 25 °C.

**5.** Zusammensetzung nach einem der vorhergehenden Ansprüche, wobei das Öl eine Molmasse (Mw) zwischen 150 und 6000, insbesondere zwischen 170 und 4000 oder sogar zwischen 180 und 2000, vorzugsweise zwischen 200 und 1500 und besser noch zwischen 220 und 800 g/Mol hat.

**6.** Zusammensetzung nach Anspruch 1, wobei das Öl, welches die reaktive Funktion trägt, ausgewählt ist aus:

- linearen, verzweigten oder cyclischen, gesättigten oder ungesättigten Fettalkoholen, umfassend 6 bis 50 Kohlenstoffatome und umfassend ein oder mehrere OH; gegebenenfalls umfassend ein oder mehrere $NH_2$, wie gesättigte oder ungesättigte, lineare oder verzweigte $C_6$-$C_{50}$-, insbesondere $C_6$-$C_{32}$- und speziell $C_8$-$C_{28}$-Monoalkohole und insbesondere Isostearylalkohol, Cetylalkohol, Oleylalkohol, Isopalmitoylalkohol, 2-Butyloctanol, 2-Hexyldecanol, 2-Octyldecanol, 2-Octyldodecanol, 2-Octyltetradecanol, 2-Decyltetradecanol und 2-Dodecylhexadecanol,
- Estern einer hydroxylierten Dicarbonsäure mit einem Monoalkohol und speziell von Äpfelsäure und insbesondere $C_4$-$C_{40}$-Alkylmalate, wie 2-Diethylhexylmalat, Diisostearylmalat und 2-Dioctyldodecylmalat.

**7.** Zusammensetzung nach einem der vorhergehenden Ansprüche, wobei der Rest R1 in der Anschlussgruppe folgendes repräsentiert:

- eine lineare oder verzweigte zweiwertige $C_2$-$C_{12}$-Alkylengruppe, insbesondere eine 1,2-methylen-, 1,6-Hexylen-, 1,4-Butylen-, 1, 6-(2,4,4-Trimethylhexylen)-, 1,4-(4-Methylpentylen)-, 1,5-(5-Methylhexylen)-, 1,6-(6-Methylheptylen)-, 1,5-(2,2,5-Trimethylhexylen)- oder 1,7-(3,7-Dimethyloctylen)gruppe;
- eine zweiwertige $C_4$-$C_{12}$-Cycloalkylen- oder Arylengruppe, die insbesondere ausgewählt ist aus den folgenden Resten:

    - Isophoron-, Tolylen, 2-Methyl-1,3-phenylen, 4-Methyl-1,3-phenylen; 4,4'-Methylenbiscyclohexylen; 4,4-Bisphenylenmethylen oder mit der Struktur:

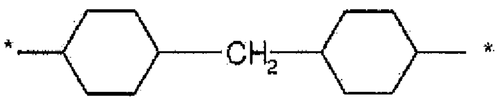 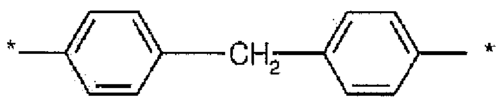

**8.** Zusammensetzung nach einem der vorhergehenden Ansprüche, wobei der Rest R2 in der Anschlussgruppe H repräsentiert, oder alternativ:

- eine $C_1$-$C_{32}$-, speziell $C_1$-$C_{16}$- oder sogar $C_1$-$C_{10}$-Alkylgruppe;
- eine $C_4$-$C_{12}$-Cycloalkylgruppe;
- eine $C_4$-$C_{12}$-Arylgruppe;
- eine ($C_4$-$C_{12}$)-Aryl-($C_1$-$C_{18}$)-alkylgruppe;
- eine $C_1$-$C_4$-Alkoxygruppe;
- eine Arylalkoxygruppe, speziell eine Aryl-($C_1$-$C_4$)-alkoxygruppe;
- einen $C_4$-$C_{12}$-Heterocyclus

oder eine Kombination dieser Reste, die gegebenenfalls mit einer Amino-, Ester- und/oder Hydroxylfrunktion substituiert sein könnten.

**9.** Zusammensetzung nach einem der vorhergehenden Ansprüche, wobei der Rest R3 in der Anschlussgruppe einen zweiwertigen Rest -R'3-O-C(O)-NH-R'4- repräsentiert, wobei R'3 und R'4, die gleich oder verschieben sein können,

einen zweiwertigen, auf Kohlenstoff basierenden Rest ausgewählt aus linearen oder verzweigten $C_1$-$C_{32}$-Alkylgruppen, einer $C_4$-$C_{16}$-Cycloalkylgruppe und einer $C_4$-$C_{16}$-Arylgruppe oder eine Mischung davon repräsentieren.

**10.** Zusammensetzung nach einem der vorhergehenden Ansprüche, wobei in der Anschlussgruppe

(a) in Formel (I) Folgende vorhanden sind:

- $R_1$ = -Isophoron- und R2 = Methyl,
- $R_1$ = - $(CH_2)_6$- und R2 = Methyl,
- $R_1$=-$(CH_2)_6$- und $R_2$= Isopropyl oder
- $R_1$ = 4,4'-Methylenbiscyclohexylen und R2 = Methyl, oder alternativ

(b) R1 in Formel (II) den Rest -Isophoron-, R2 = Methyl und R3 = -$(CH_2)_2$OCO-NH-Isophoron- repräsentiert.

**11.** Zusammensetzung nach einem der vorhergehenden Ansprüche, wobei die Anschlussgruppe die folgende Formel aufweiset:

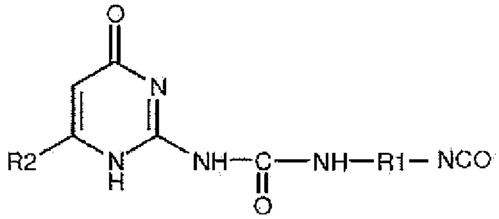

oder die Formel:

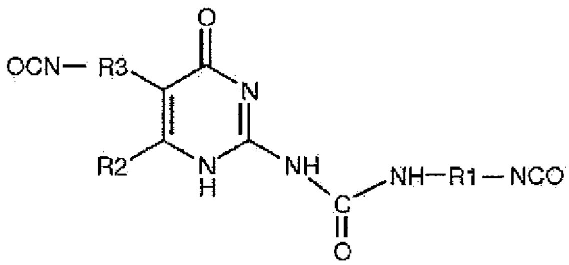

wobei R1, R2 und R3 wie in einem der vorhergehenden Ansprüche definiert sind.

**12.** Zusammensetzung nach einem der vorhergehenden Ansprüche, wobei die Änschlussgruppe ausgewählt ist aus den folgenden Gruppen:

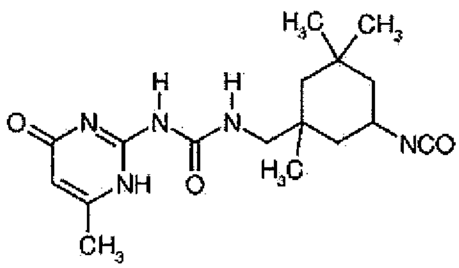

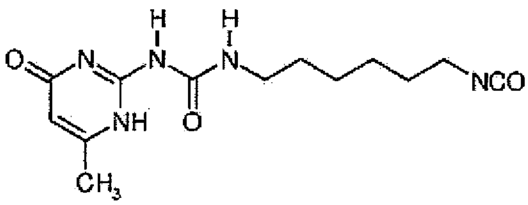

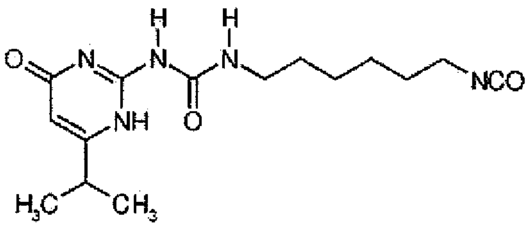

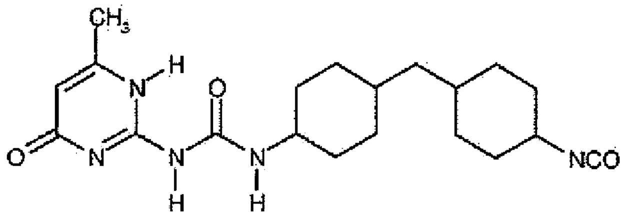

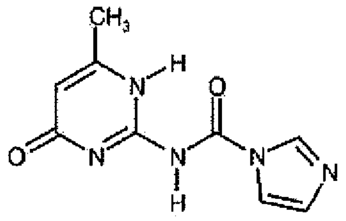

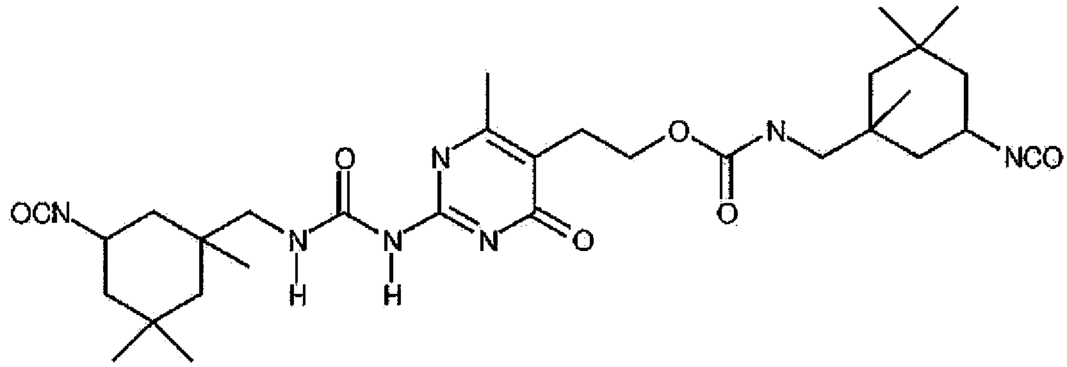

13. Zusammensetzung nach einem der vorhergehenden Ansprüche, wobei die supramolekularen Verbindungen ausgewählt aus jenen, die den folgenden Strukturen entsprechen:

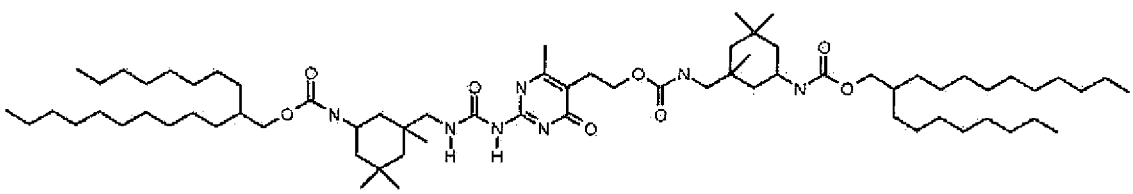

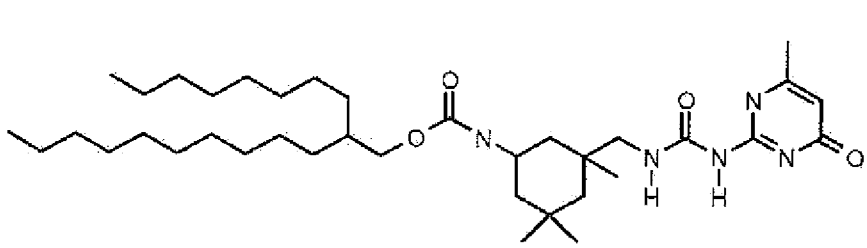

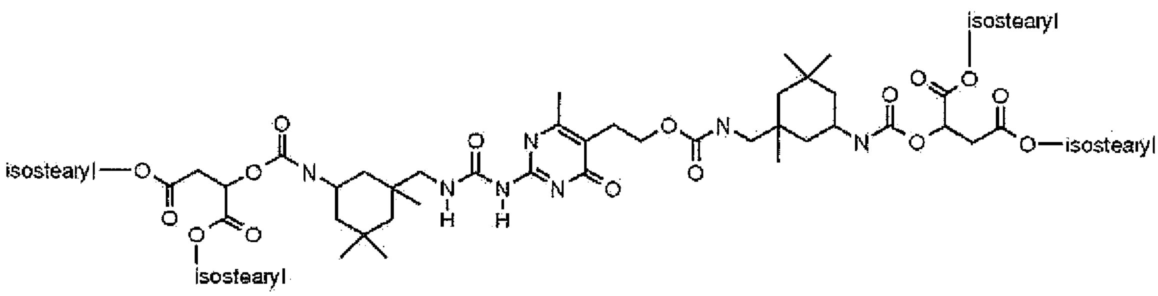

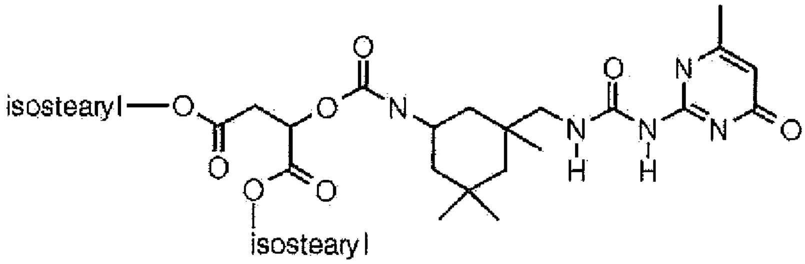

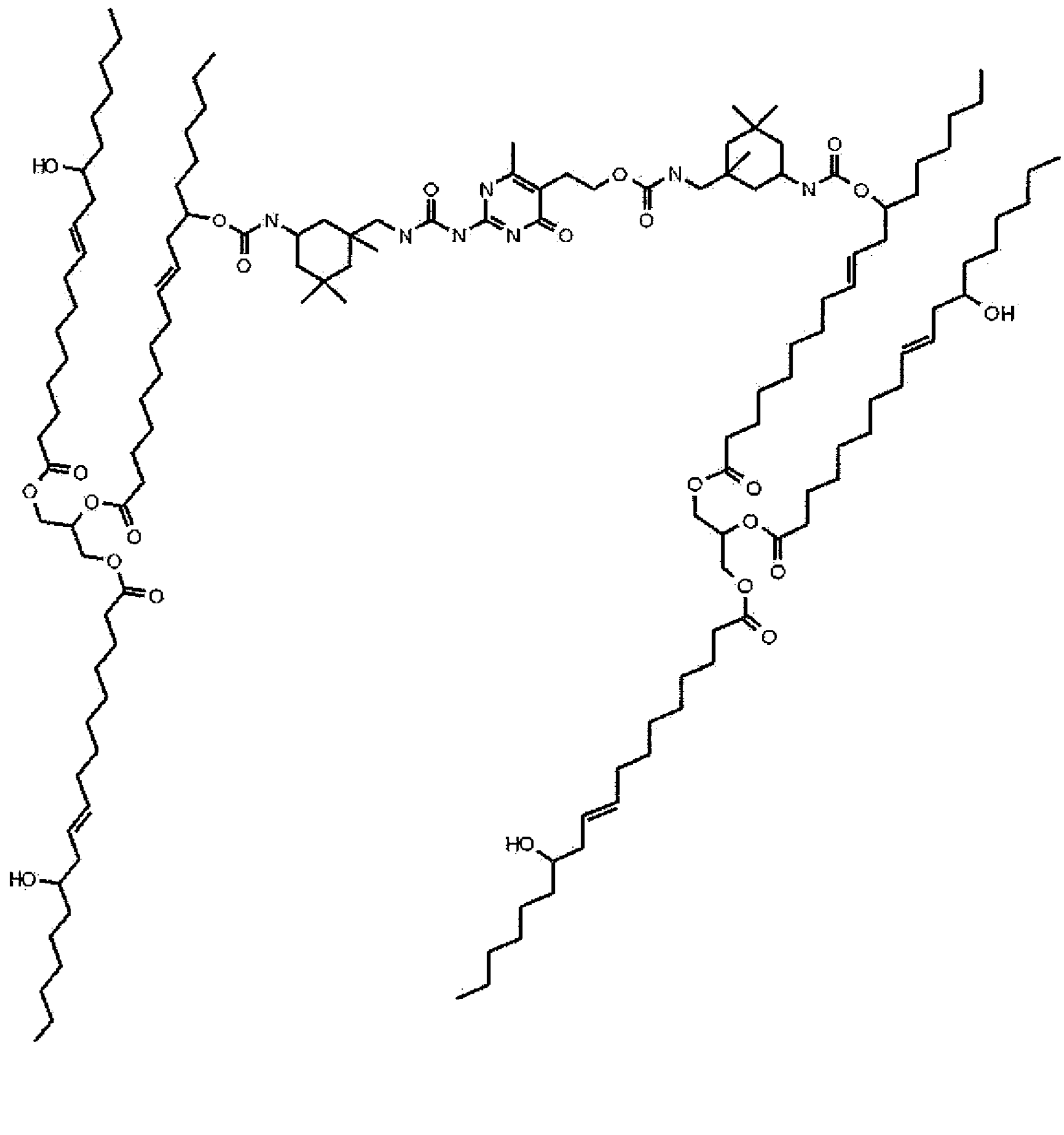

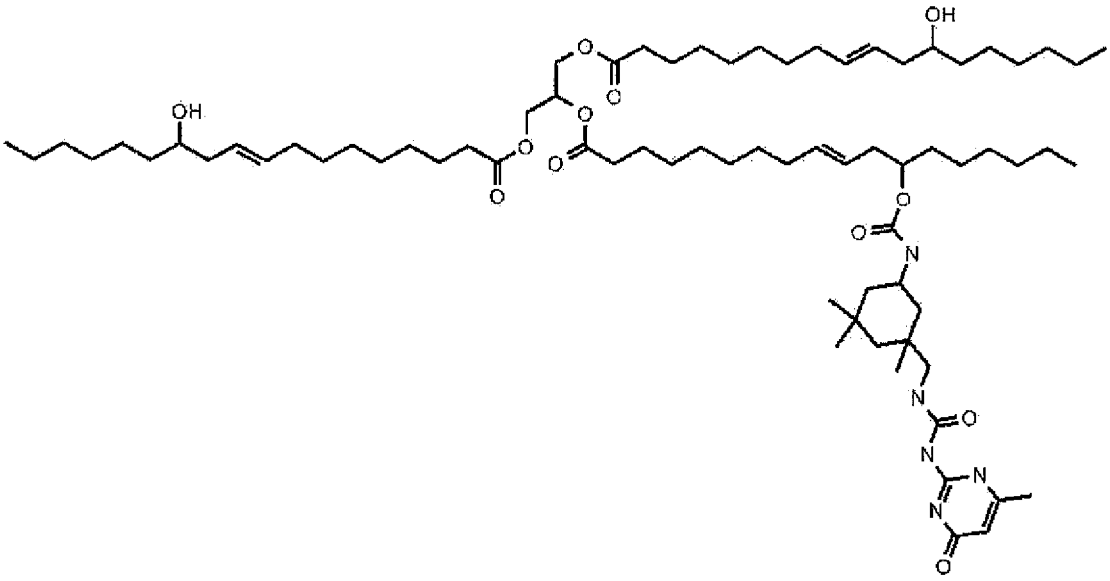

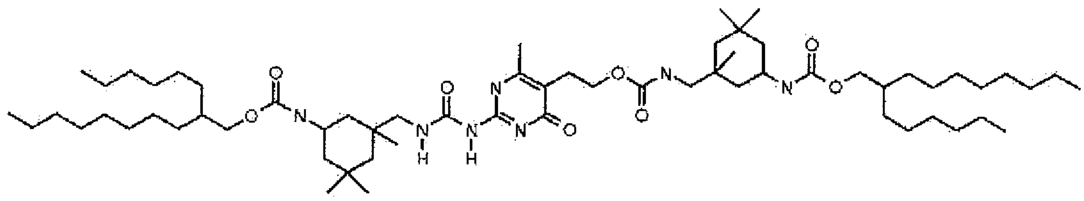

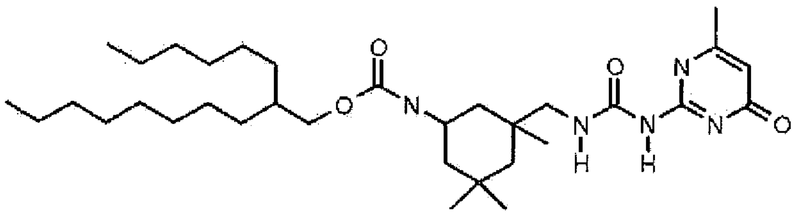

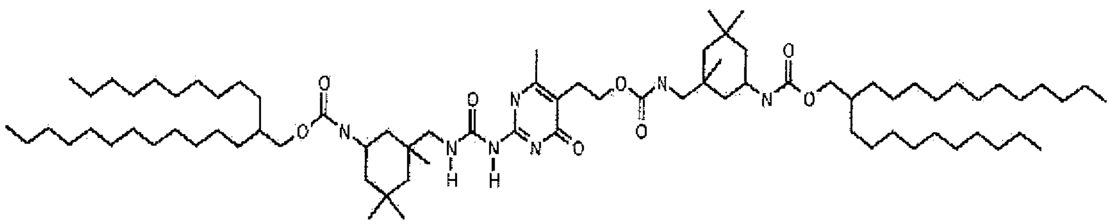

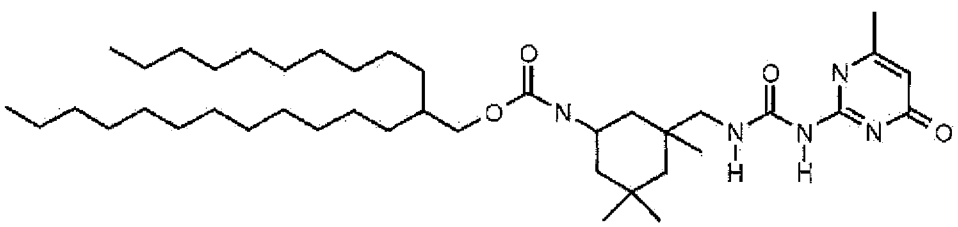

**14.** Zusammensetzung nach einem der vorhergehenden Ansprüche, wobei das durchschnittliche Molekulargewicht (Zahlenmittel) (Mn) der supramolekularen Verbindung zwischen 180 und 8000, vorzugsweise 200 bis 6000 oder sogar 300 bis 4000 liegt, noch besser 400 bis 3000 und vorzugsweise 500 bis 1500.

**15.** Zusammensetzung nach einem der vorhergehenden Ansprüche, wobei die Menge an supramolekularer Verbindung, die in der Zusammensetzung vorhanden ist, zwischen 5 Gew.% und 95 Gew.%, vorzugsweise zwischen 10 Gew.% und 95 Gew.% und noch bevorzugter zwischen 20 Gew.% und 90 Gew.% liegt, bezogen auf das Gesamtgewicht der Zusammensetzung.

**16.** Zusammensetzung nach einem der vorhergehenden Ansprüche, wobei das aus apolaren, auf Kohlenwasserstoff basierenden Ölen und Silikonölen ausgewählte Öl nichtflüchtig ist.

**17.** Zusammensetzung nach einem der vorhergehenden Ansprüche, **dadurch gekennzeichnet, dass** das Öl ein Silikonöl ausgewählt aus:

i) Fhenylsilikonölen, speziell mit der folgenden Formel (VII) oder (VIII):

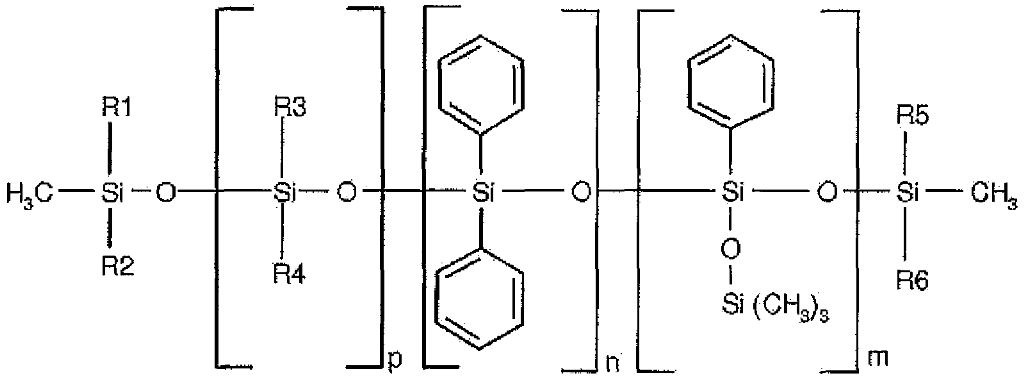

(VII)

wobei:

- R1 bis R6 unabhängig voneinander gesättigte oder ungesättigte, lineare, cyclische oder verzweigte, auf $C_1$-$C_{30}$-Kohlenwasserstoff basierende Reste sind,
- m, n und p unabhängig voneinander ganze Zahlen zwischen 0 und 100 sind, mit der Maßgabe, dass die Summe n + m zwischen 1 und 100 liegt;

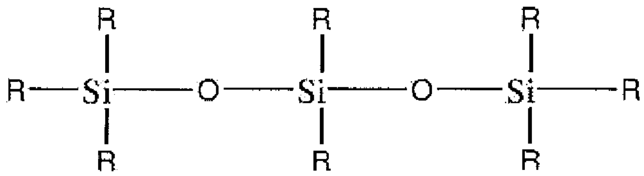

(VIII)

wobei die Gruppen R unabhängig voneinander ein Methyl oder ein Phenyl repräsentieren;

ii) linearen oder cyclischen Polydimethylsiloxanen (PDMS);
iii) Polydimethylsiloxanen ist, umfassend Alkyl- oder Alkoxygruppen, die seitenständig und/oder am Ende der Silikonkette sind, wobei diese Gruppen jeweils 2 bis 24 Kohlenstoffatome enthalten.

**18.** Zusammensetzung nach einem der vorhergehenden Ansprüche, **dadurch gekennzeichnet, dass** das Öl ein apolares, auf Kohlenwasserstoff basierendes Öl ist, das ausgewählt ist aus:

- Polybutylenen,
- hydrierten Polyisobutylenen,
- Polydecenen und hydrierten Polydecenen,
- auf Kohlenwasserstoff basierenden flüchtigen Ölen, die 7 bis 16 Kohlenstoffatome enthalten, und Mischungen davon, und insbesondere verzweigten $C_8$-$C_{16}$-Alkunen und linearen Alkanen, die 7 bis 15 Kohlenstoffatome enthalten, und Mischungen davon, und
- Mischungen davon.

**19.** Zusammensetzung nach einem der vorhergehenden Ansprüche, **dadurch gekennzeichnet, dass** das Öl/die Öle ausgewählt aus apolaren, auf Kohlenwasserstoff basierenden Ölen und Silikonölen in einem Gesamtgehalt im Bereich von 0,5 Gew.% bis 70 Gew.% vorhanden sind, bezogen auf das Gesamtgewicht der Zusammensetzung, vorzugsweise im Bereich von 3 Gew.% bis 50 Gew.% und besser noch im Bereich von 5 Gew.% bis 40 Gew.%, bezogen auf das Gesamtgewicht der Zusammensetzung.

**20.** Zusammensetzung nach einem der vorhergehenden Ansprüche, die in Form einer Zusammensetzung zur Pflege von und/oder zum Zurechtmachen von Körper- oder Gesichtshaut, den Lippen, den Wimpern, den Augenbrauen oder den Nägeln; eines Sonnenschutz- oder Selbstbräunungsprodukts vorliegt.

**21.** Kosmetikverfahren zur Behandlung von Keratinmaterialien, insbesondere Körper- oder Gesichtshaut, den Lippen, den Nägel und/oder den Wimpern, umfassend das Aufbringen einer Kosmetikzusammensetzung gemäß einem der Ansprüche 1 bis 20 auf diese Materialien.

## Revendications

**1.** Composition cosmétique de maquillage et/ou de soin des matières kératiniques comprenant, dans un milieu cosmétiquement acceptable,

(i) un composé supramoléculaire susceptible d'être obtenu par réaction entre :

- au moins une huile portant au moins une fonction réactive nucléophile choisie parmi OH et $NH_2$, et
- au moins un groupe de jonction capable d'établir des liaisons hydrogène avec un ou plusieurs groupes de jonction partenaires, chaque appariement d'un groupe de jonction faisant intervenir au moins 3 liaisons hydrogène, ledit groupe de jonction portant au moins une fonction réactive isocyanate ou imidazole susceptible de réagir avec la fonction réactive portée par l'huile, ledit groupe de jonction comprenant en outre au moins un motif de formule (I) ou (II) :

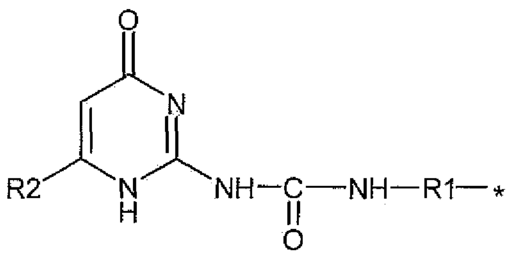

(I)

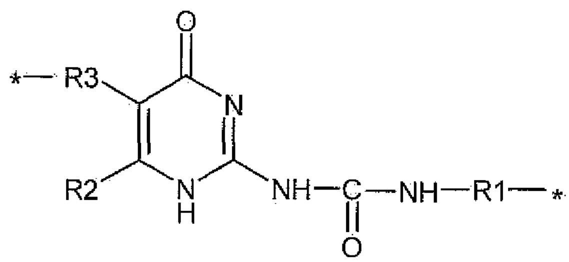

(II)

dans lesquelles :

- R1 et R3, identiques ou différents, représentent un radical carboné divalent choisi parmi (i) un groupe alkyle linéaire ou ramifié en $C_1$-$C_{32}$, (ii) un groupe cycloalkyle en $C_4$-$C_{16}$ et (iii) un groupe aryle en $C_4$-$C_{16}$; comprenant éventuellement 1 à 8 hétéroatomes choisis parmi O, N, S, F, Si et P; et/ou éventuellement substitué par une fonction ester, amide ou par un radical alkyle en $C_1$-$C_{12}$; ou un mélange de ces groupes;
- R2 représente un atome d'hydrogène ou un radical carboné, notamment hydrocarboné, linéaire, ramifié ou cyclique, saturé ou insaturé, éventuellement aromatique, en $C_1$-$C_{32}$, pouvant comprendre un ou plusieurs hétéroatomes choisis parmi O N, S, F, Si et P ;

(ii) au moins une huile, choisie parmi les huiles hydrocarbonées apolaires non volatiles présentant une pression de vapeur inférieure à 0,13 Pa (à température ambiante (25°C)) et les huiles siliconées,
(iii) au moins une matière colorante.

**2.** Composition selon la revendication 1, dans laquelle l'huile portant la fonction réactive est choisie parmi, seule ou en mélange :

(i) les alcools gras, comprenant 6 à 50 atomes de carbone, linéaires, ramifiés ou cycliques, saturés ou insaturés, comprenant 1 ou plusieurs OH; éventuellement comprenant un ou plusieurs $NH_2$;
(ii) les esters et les éthers portant au moins un groupe OH libre, et notamment les esters et éthers partiels de polyol, et les esters d'acide carboxylique hydroxylé;
(iii) les huiles naturelles, naturelles modifiées, végétales, hydroxylées.

**3.** Composition selon l'une des revendications précédentes, dans laquelle l'huile portant la fonction réactive est choisie parmi, seule ou en mélange :

- les monoalcools linéaires ou ramifiés en C6-C50, notamment en C6-C32, en particulier en C8-C28, saturés ou insaturés, et notamment l'alcool isostéarylique, l'alcool cétylique, l'alcool oléylique, l'alcool isopalmitoylique, le butyl-2 octanol, l'hexyl-2 décanol, l'octyl-2 décanol, l'octyl-2 dodécanol, l'octyl-2 tétradécanol, le décyl-2 tétradécanol, le dodécyl-2 hexadécanol;
- les diols linéaires ou ramifiés en C6-C50, notamment en C6-C40, en particulier en C8-C38, saturés ou insaturés, et notamment ramifiés en C32-C36;
- les triols linéaires ou ramifiés en C6-C50, notamment en C6-C32, en particulier en C8-C28, saturés ou insaturés, et notamment le phytantriol;
- les esters partiels de pentaérythritol, et notamment l'adipate de pentaérythrityle, le caprate de pentaérythrityle, le succinate de pentaérythrityle, le tétraisononanoate de pentaérythrityle, le triisononanoate de pentaérythrityle, le tétraisostéarate de pentaérythrityle, le triisostéarate de pentaérythrityle, le tétradécyl-2 tétradécanoate de pentaérythrityle, le tétraéthyl hexanoate de pentaérythrityle, le tétraoctyl dodécanoate de pentaérythrityle;
- les diesters, triesters, tétraesters ou pentaesters de dipentaérythritol, et notamment le dipentaérythrityle pentaisononanoate, le dipentaérythrityle pentaisostéarate, le dipentaérythrityle tétraisostéarate, le dipentaérythrityle tri(polyhydroxystéarate);
- les mono et di-esters de triméthylolpropane comme le triméthylolpropane mono-isostéarate, le triméthylolpropane di-isostéarate, le triméthylolpropane monoéthyl-2 hexanoate, le triméthylolpropane diéthyl-2 hexanoate;
- les mono-, di- et tri-esters de di-triméthylolpropane comme le di-triméthylolpropane di-isostéarate, le ditriméthylolpropane tri-isostéarate, le ditriméthylolpropane tri-éthyl hexanoate;
- les mono-esters ou poly-esters partiels de glycérol ou de polyglycérols, et notamment :

  - le di-isostéarate de glycérol, le diisononanoate de glycérol,
  - les mono-, di- et tri-esters de polyglycérol-2; par exemple avec l'acide isostéarique, l'acide éthyl-2 hexanoïque et/ou l'acide isononanoique; et notamment le polyglycéryl-2-isostéarate; le polyglycéryl-2-diisostéarate; le triisostéarate de polyglycéryl-2; le polyglycéryl-2-nonaisostéarate; le polyglycéryl-2-nonanoate;
  - les mono-, di-, tri- ou tétra-esters de polyglycérol-3; par exemple avec soit l'acide isostéarique, l'acide éthyl-2 hexanoique et/ou l'acide isononanoïque; et notamment le polyglycéryl-3-isostéarate, le polyglycéryl-3-diisostéarate; le triisostéarate de polyglycéryl-3; le polyglycéryl-3-nonaisostéarate; le polyglycéryl-3-nonanoate;
  - les esters partiels de polyglycérol-10 et en particulier le polyglycéryl-10 nonaisostéarate; le polyglycéryl-10-nonanoate; le polyglycéryl-10-isostéarate, le polyglycéryl-10-diisostéarate, le triisostéarate de polyglycéryl-10;

- les monoesters de propylène glycol comme le monoisostéarate de propylène glycol, le néopentanoate de propylène glycol, le monooctanoate de propylène glycol;
- les monoesters de dimères-diols comme l'isostéaryl dimère dilinoléate et l'octyl dodécyl dimère dilinoléate
- les éthers de glycérol, tels que le polyglycéryl-2 oléyléther, le polyglycéryl-3 cétyléther, le polyglycéryl-3 décyltétradécyléther et le polyglycéryl-2 stéaryléther;
- les esters entre acide mono-, di- ou tri-carboxylique hydroxylé et monoalcools, et en particulier:

  - les esters, notamment monoesters, d'acide 12-hydroxystéarique; tels que l'hydroxystéarate d'octyle, et l'octyl-2 dodécyl hydroxystéarate; on peut également citer les polyhydroxystéarates oligomères correspondants, notamment ayant un degré de polymérisation de 1 à 10, possédant au moins un OH résiduel;
  - les esters d'acide lactique, et notamment les lactates d'alkyles en C4-40, tels que le lactate de 2-éthylhexyle, le lactate de diisostéaryle, le lactate d'isostéaryle, le lactate d'isononyle, le lactate d'octyl-2 dodécyle;
  - les esters d'acide malique, et notamment les malates d'alkyles en C4-40, tels que le malate de diéthyl-2 hexyle, le malate de diisostéaryle, le malate de dioctyl-2 dodécyle;
  - les esters d'acide citrique, et notamment les citrates d'alkyles en C4-40, tels que le citrate de triisostéaryle,

le citrate de triisocétyle et le citrate de triisoarachidyle.

- les esters triglycériques portant un ou plusieurs OH,
- l'huile de ricin, hydrogénée ou non, ainsi que ses dérivés notamment issus de la transestérification de l'huile de ricin;
- les huiles époxydées modifiées, la modification consistant à ouvrir la fonction époxy pour obtenir un diol, et notamment l'huile de soja modifiée hydroxylée; les huiles de soja hydroxylées (directement hydroxylées ou d'abord époxydées).

**4.** Composition selon l'une des revendications précédentes, dans laquelle l'huile est une huile brillante, c'est-à-dire ayant un indice de réfraction supérieur ou égal à 1,46 à 25°C.

**5.** Composition selon l'une des revendications précédentes, dans laquelle l'huile a une masse molaire (Mw) comprise entre 150 et 6000, notamment entre 170 et 4000, voire entre 180 et 2000, préférentiellement entre 200 et 1500, et encore mieux entre 220 et 800 g/mol.

**6.** Composition selon la revendication 1, dans laquelle l'huile portant la fonction réactive est choisie parmi :

- les alcools gras, comprenant 6 à 50 atomes de carbone, linéaires, ramifiés ou cycliques, saturés ou insaturés, comprenant 1 ou plusieurs OH; éventuellement comprenant un ou plusieurs $NH_2$, tels que les monoalcools linéaires ou ramifiés en C6-C50, notamment en C6-C32, en particulier en C8-C28, saturés ou insaturés, et notamment l'alcool isostéarylique, l'alcool cétylique, l'alcool oléylique, l'alcool isopalmitoylique, le butyl-2 octanol, l'hexyl-2 décanol, l'octyl-2 décanol, l'octyl-2 dodécanol, l'octyl-2 tétradécanol, le décyl-2 tétradécanol, le dodécyl-2 hexadécanol,
- les esters d'acide dicarboxylique hydroxylé avec un monoalcool, et en particulier d'acide malique, et notamment les malates d'alkyles en C4-40, tels que le malate de diéthyl-2 hexyle, le malate de diisostéaryle, le malate de dioctyl-2 dodécyle.

**7.** Composition selon l'une des revendications précédentes, dans laquelle, dans le groupe de jonction, le radical R1 représente :

- un groupe alkylène divalent, linéaire ou ramifié, en C2-C12, notamment un groupe 1,2-éthylène, 1,6-hexylène, 1,4-butylène, 1,6-(2,4,4-triméthylhexylène), 1,4-(4-méthylpentylène) ; 1,5-(5-méthylhexylène), 1,6-(6-méthylheptylène), 1,5-(2,2,5-triméthylhexylène), 1,7-(3,7-diméthyloctylène).
- un groupe cycloalkylène ou arylène, divalent, en C4-C12, notamment choisi parmi les radicaux suivants
- isophorone-, tolylène, 2-méthyl-1,3-phénylène, 4-méthyl-1,3-phénylène; 4,4'-méthylènebiscyclohexylène; 4,4-bisphénylèneméthylène; ou de structure :

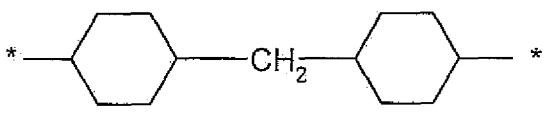
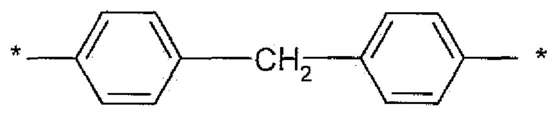

**8.** Composition selon l'une des revendications précédentes, dans laquelle, dans le groupe de jonction, le radical R2 représente H, ou bien :

- un groupe alkyle en $C_1$-$C_{32}$, en particulier en $C_1$-$C_{16}$, voire en $C_1$-$C_{10}$;
- un groupe cycloalkyle en $C_4$-$C_{12}$ ;
- un groupe aryle en $C_4$-$C_{12}$ ;
- un groupe aryl($C_4$-$C_{12}$) alkyle en $C_1$-$C_{18}$
- un groupe alcoxy en $C_1$-$C_4$ ;
- un groupe arylalcoxy, en particulier un groupe aryle ($C_1$-$C_4$) alcoxy ;
- un hétérocycle en $C_4$-$C_{12}$

ou une combinaison de ces radicaux, qui peuvent éventuellement être substitués par une fonction amino, ester et/ou hydroxyle.

**9.** Composition selon l'une dés revendications précédentes, dans laquelle, dans le groupe de jonction, le radical R3

représente un radical divalent -R'3-OC(O)-NH-R'4- dans lequel R'3 et R'4, identiques ou différents, représentent un radical carboné divalent choisi parmi un groupe alkyle linéaire ou ramifié en $C_1$-$C_{32}$ ou un groupe cycloalkyle en $C_4$-$C_{16}$ ou un groupe aryle en $C_4$-$C_{16}$; ou leur mélange.

**10.** Composition selon l'une des revendications précédentes, dans laquelle, dans le groupe de jonction,

(a) dans la formule (I), on a :

- $R_1$ = -isophorone- et R2 = méthyl,
- $R_1$ = $-(CH_2)_6-$ et R2 = méthyl,
- $R_1$ = $(CH_2)_6-$ et R2 = isopropyl, ou
- $R_1$ = 4,4'-méthylènebiscyclohexylène et R2 = méthyle, ou bien

(b) dans la formule (II), R1 représente le radical -isophorone-, R2= méthyle et R3=$-(CH_2)_2$OCO-NH-isophorone-.

**11.** Composition selon l'une des revendications précédentes, dans laquelle le groupe de jonction est de formule :

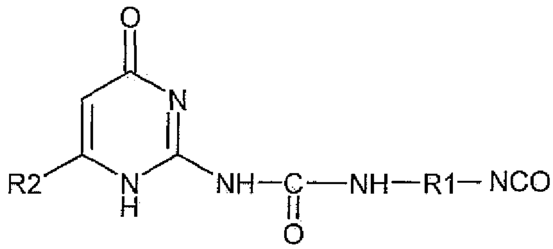

ou de formule :

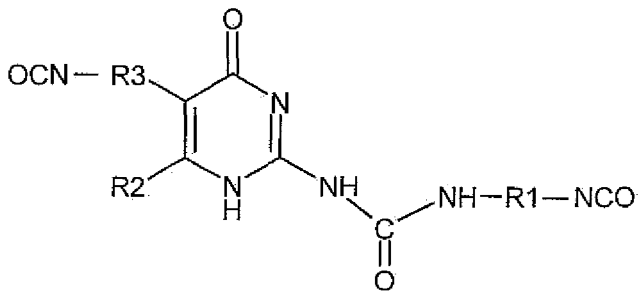

dans laquelle R1, R2 et R3 sont tels que définis dans l'une des revendications précédentes.

**12.** Composition selon l'une des revendications précédentes, dans laquelle le groupe de jonction est choisi parmi les groupes suivants :

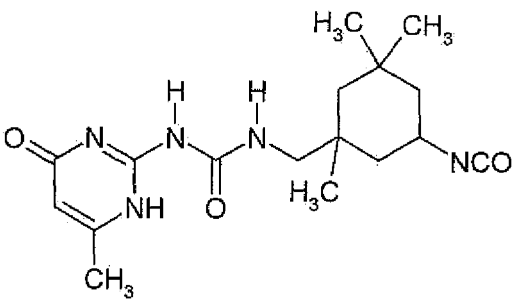

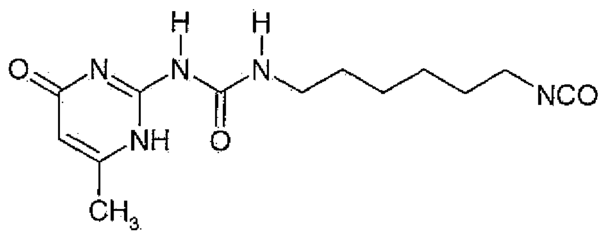

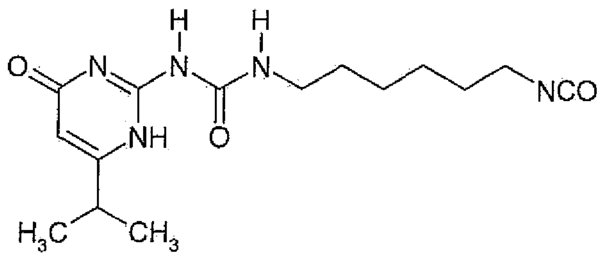

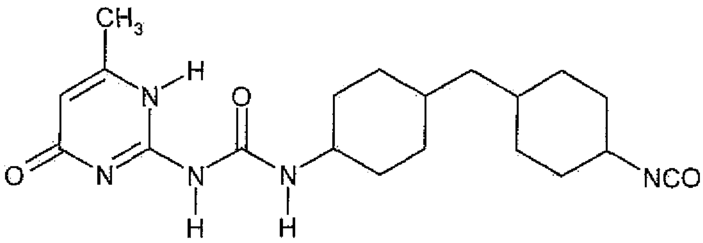

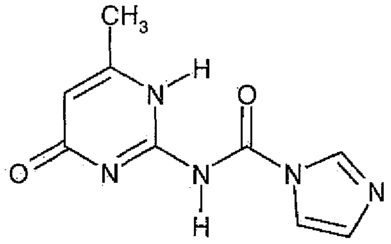

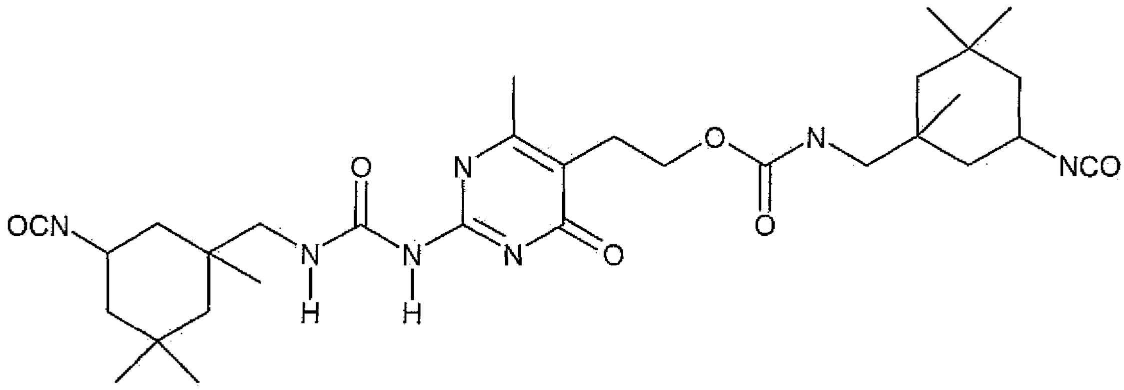

**13.** Composition selon l'une des revendications précédentes, dans laquelle les composés supramoléculaires sont choisis parmi ceux répondant aux structures suivantes :

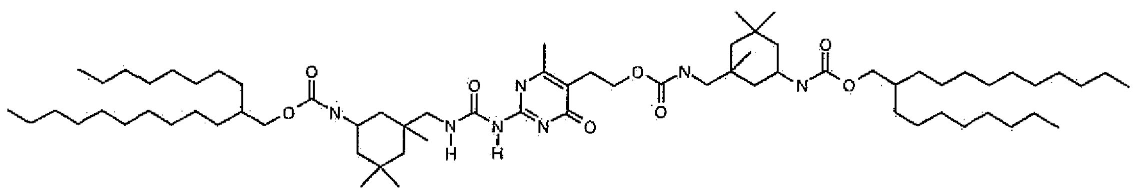

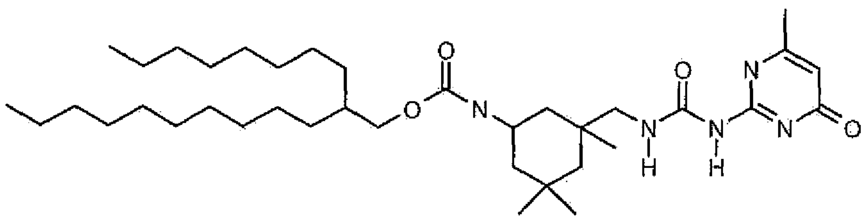

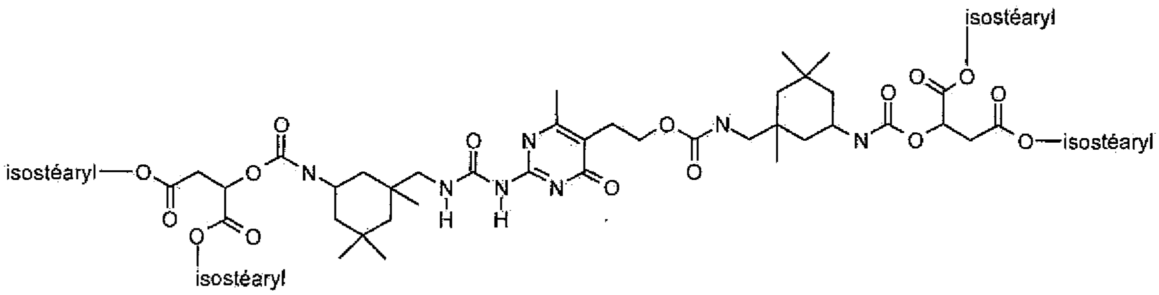

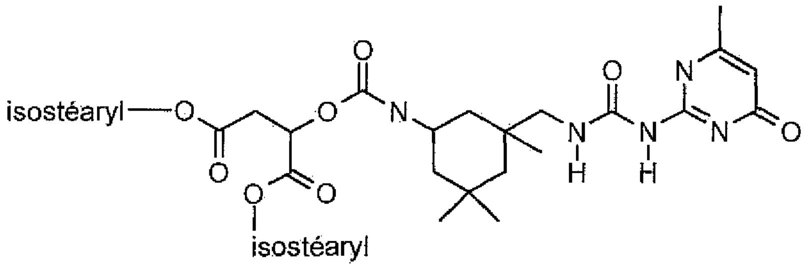

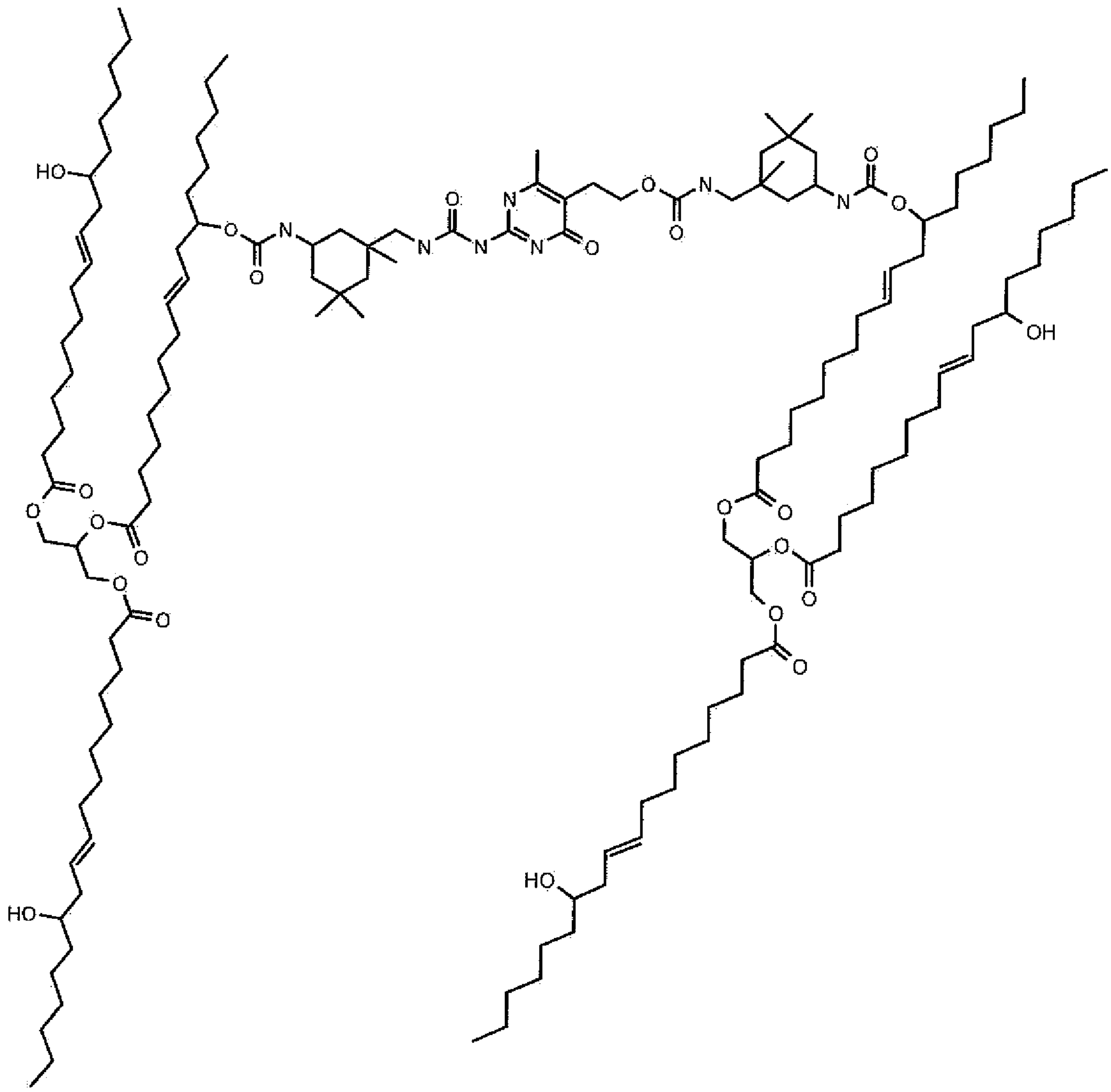

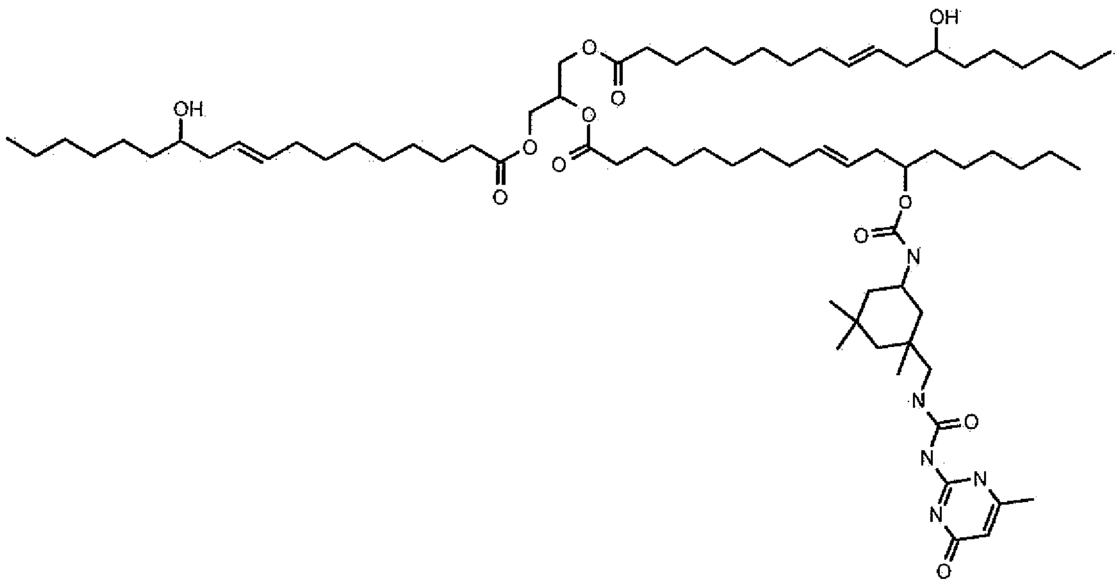

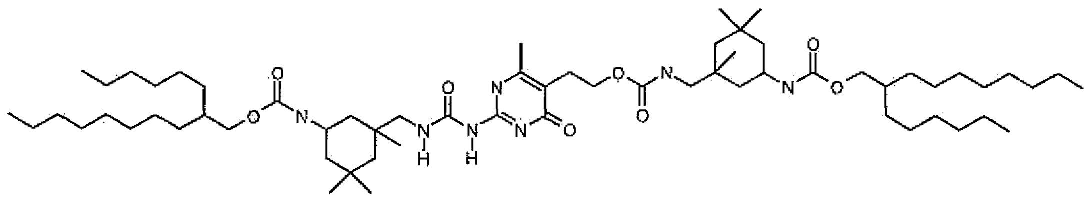

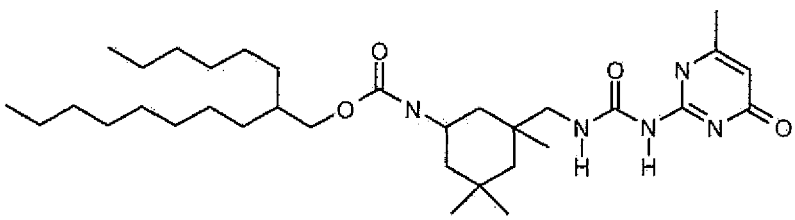

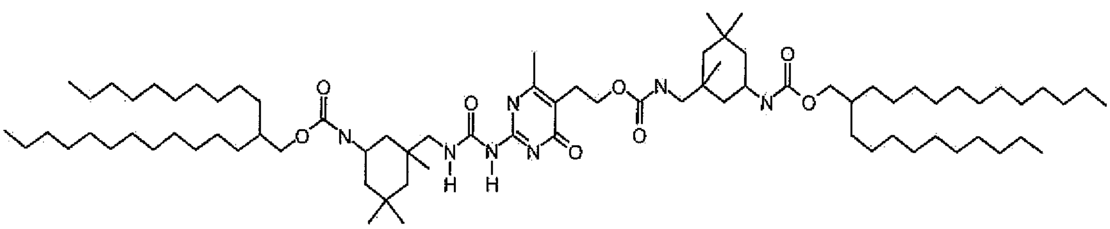

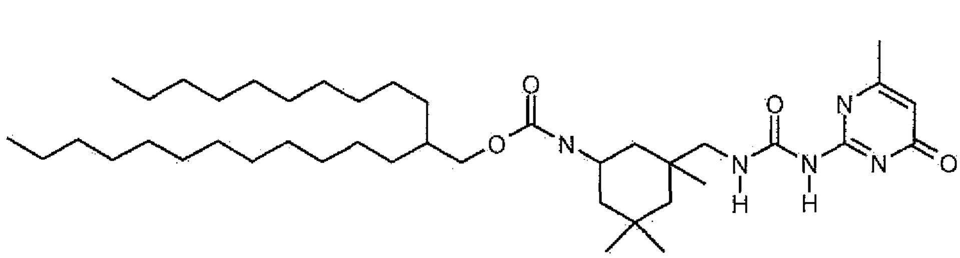

14. Composition selon l'une des revendications précédentes, dans laquelle la masse moléculaire moyenne en nombre (Mn) du composé supramoléculaire est comprise entre 180 à 8000, de préférence 200 à 6000, voire de 300 à 4000, et encore mieux de 400 à 3000, préférentiellement de 500 à 1500.

**15.** Composition selon l'une des revendications précédentes, dans laquelle la quantité de composé supramoléculaire présent dans la composition est comprise entre 5 % et 95 % en poids, de préférence entre 10 % et 95 % en poids, et mieux de préférence entre 20 % et 90 % en poids par rapport au poids total de la composition.

**16.** Composition selon l'une des revendications précédentes, dans laquelle ladite huile choisie parmi les huiles hydrocarbonées apolaires et les huiles siliconées est non volatile.

**17.** Composition selon l'une des revendications précédentes, **caractérisée en ce que** ladite huile, est une huile siliconée, choisie parmi :

i) les huiles siliconée phénylées, en particulier de formule (VII) ou (VIII) suivantes :

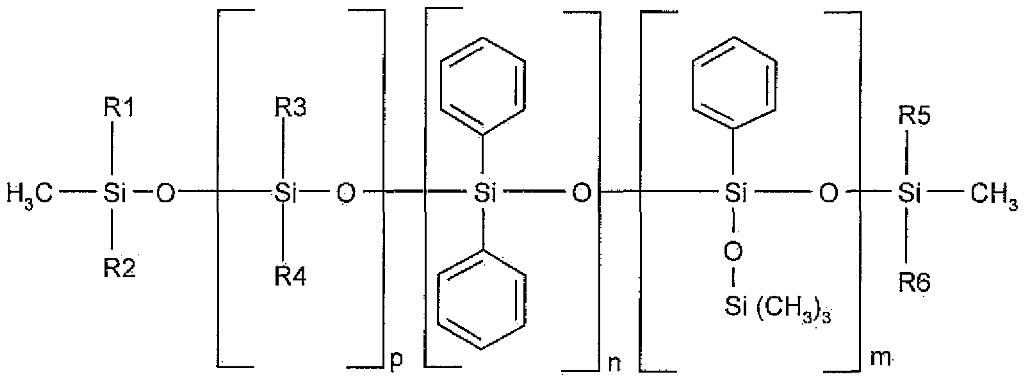

(VII)

dans laquelle :

- R1 à R6, indépendamment les uns des autres, sont des radicaux hydrocarbonés, saturés ou insaturés, linéaires cycliques ou ramifiés, en C1-C30,
- m, n et p sont, indépendamment les uns des autres, des nombres entiers compris entre 0 et 100, sous réserve que la somme 'n + m'est comprise entre 1 et 100 ;

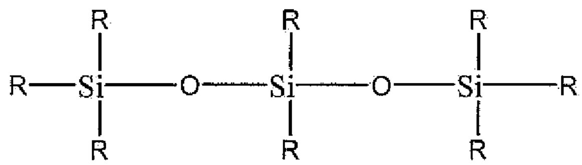

(VIII)

dans laquelle les groupements R représentent indépendamment les uns des autres un méthyle ou un phényle.

ii) les polydiméthylsiloxanes (PDMS) linéaires ou cycliques,
iii) les polydiméthylsiloxanes comportant des groupements alkyle ou alcoxy, pendant et/ou en bout de chaîne siliconée, groupements ayant chacun de 2 à 24 atomes de carbone.

**18.** Composition selon l'une des revendications précédentes, **caractérisée en ce que** ladite huile, est une huile hydrocarbonée apolaire choisie parmi :

- les polybutylènes,
- les polyisobutylènes hydrogénés,
- les polydécènes et les polydécènes hydrogénés,
- les huiles volatiles hydrocarbonées ayant de 7 à 16 atomes de carbone et leurs mélanges, et notamment les alcanes ramifiés en $C_8$-$C_{16}$ et les alcanes linéaires ayant de 7 à 15 atomes de carbone et leurs mélanges, et
- leurs mélanges.

**19.** Composition selon l'une des revendications précédentes, **caractérisée en ce que** ladite (ou lesdites) huile(s),

choisie(s) parmi les huiles hydrocarbonées apolaires et les huiles siliconées, est présente en une teneur totale allant de 0,5 % à 70 % en poids par rapport au poids total de la composition, de préférence allant de 3 % à 50 % en poids, et mieux allant de 5 % à 40 % en poids par rapport au poids total de la composition.

**20.** Composition selon l'une des revendications précédentes, se présentant sous la forme d'une composition de soin et/ou de maquillage de la peau du corps ou du visage, des lèvres, des cils, des sourcils, ou des ongles; d'un produit solaire ou autobronzant.

**21.** Procédé de traitement cosmétique des matières kératiniques, notamment de la peau du corps ou du visage, des lèvres, des ongles, et/ou des cils, comprenant l'application sur lesdites matières d'une composition cosmétique telle que définie selon l'une quelconque des revendications 1 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1097699 A
- US 5707612 A
- WO 2007068371 A
- WO 2008155059 A
- FR 0853634
- EP 0955039 A
- EP 542669 A
- EP 787730 A
- EP 787731 A
- WO 9608537 A
- FR 2792190 A
- US 5874069 A
- US 5919441 A
- US 6051216 A
- US 5981680 A


### Non-patent literature cited in the description


- **HOFER et al.** *European Coating Journal,* March 2000, 26-37
- **FOLMER et al.** *Adv. Mater.,* 2000, vol. 12, 874-78
- **C.M. HANSEN.** The three dimensional solubility parameters. *J. Paint Technol.,* 1967, vol. 39, 105
- CTFA. 2000